# EUROPEAN PATENT SPECIFICATION

(11) **EP 1 527 175 B1**
(45) Date of publication and mention of the grant of the patent: **27.05.2009**
(21) Application number: 03780549.6
(22) Date of filing: 20.06.2003
(51) Int. Cl.: C12N 15/10, C12Q 1/68

(54) **METHODS AND SYSTEMS FOR DETECTION AND ISOLATION OF A NUCLEOTIDE SEQUENCE**
METHODEN UND SYSTEME ZUR DETEKTION UND ISOLATION VON NUCLEINSÄURESEQUENZEN
PROCEDES ET SYSTEMES DE DETECTION ET D'ISOLATION D'UNE SEQUENCE NUCLEOTIDIQUE

(30) Priority: 24.06.2002 US 390928 P
(43) Date of publication of application: 04.05.2005
(73) Proprietor: Exiqon A/S, 2950 Vedbaek (DK)
(72) Inventor: KAUPPINEN, Sakari, DK-2765 Smoerum (DK); JACOBSEN, Nana, DK-2820 Gentofte (DK)
(74) Representative: Forrest, Graham Robert
(86) International application number: PCT/IB2003/006354
(87) International publication number: WO 2004/020575

(56) References cited:
- EP-A- 1 247 815
- WO-A-00/56748
- WO-A-00/56916
- WO-A-00/56920
- WO-A-99/14226

## Description

### 1. Field of the Invention

The invention provides methods for detecting and/or isolating nucleic acid molecules, using oligonucleotide capture probes comprising various amounts and designs of LNA (locked nucleic acid)/DNA molecules. Methods of the invention are especially advantageous when dealing with RNA molecules due to the rapid degradation of such molecules.

### 2. Background

Organic solvents such as phenol and chloroform are traditionally used in techniques employed to isolate nucleic acid from prokaryotic and eukaryotic cells or from complex biological samples. Nucleic acid isolations typically begin with an enzymatic digest performed with proteases followed by cell lysis using ionic detergents and then extraction with phenol or a phenol/chloroform combination. The organic and aqueous phases are separated and nucleic acids which have partitioned into the aqueous phase are recovered by precipitation with alcohol. However, phenol or a phenol/chlorofbnn mixture is corrosive to human skin and is considered as hazardous waste which must be carefully handled and properly discarded. Further, the extraction method is time consuming and labor-intensive. Marmur, J. Mol. Biol., 3:208-218 (1961), describes the standard preparative procedure for extraction and purification of intact high molecular weight DNA from prokaryotic organisms using enzymatic treatment, addition of a detergent, and the use of an organic solvent such as phenol or phenol/chloroform. Chirgwin et al., Biochemistry,18:5294-5299 (1979) described the isolation of intact RNA from tissues enriched in ribonuclease by homogenization in GuSCN and 2-mercaptoethanol followed by ethanol precipitation or by sedimentation through cesium chloride. Further developments of the methods are described by Ausubel et al. in Current Protocols in Molecular Biology, pub. John Wiley & Sons (1998).

Further, the use of chaotropic agents such as guanidinium thiocyanate (GuSCN) is widely used to lyse and release nucleic acids from cells into solution, largely due to the fact that the chaotropic salts inhibit nucleases and proteases while at the same time facilitating the lysis of the cells.

Nucleic acid hybridization is a known and documented method for identifying nucleic acids. Hybridization is based on base pairing of complementary nucleic acid strands. When single stranded nucleic acids are incubated in appropriate buffer solutions, complementary sequences pair to form stable double stranded molecules. The presence or absence of such pairing may be detected by several different methods well known in the art.

In relation to the present invention a particularly interesting technique was described by Dunn & Hassell in Cell, Vol. 12, pages 23-36 (1977). Their assay is of the sandwich-type whereby a first hybridization occurs between a "target" nucleic acid and a "capturing" nucleic acid probe which has been immobilized on a solid support. A second hybridization then follows where a "signal" nucleic acid probe, typically labelled with a fluorophore, a radioactive isotope or an antigen determinant, hybridizes to a different region of the immobilized target nucleic acid. The hybridization of the signal probe may then be detected by, for example, fluorometry.

Ranki et al. in US 4,486,539 and US 4,563,419 and EP 0,079,139 describe sandwich-type assays which first require steps to render nucleic acids single stranded and then the single stranded nucleic acids are allowed to hybridize with a nucleic acid affixed to a solid carrier and with a nucleic acid labelled with a radioisotope. Thus, the Ranki et al. assay requires the nucleic acid to be identified or targeted in the assay to be first rendered single stranded.

One approach to dissolving a biological sample in a chaotropic solution and performing molecular hybridization directly upon the dissolved sample is described by Thompson and Gillespie, Analytical Biochemistry, 163:281-291 (1987). See also WO 87/106621. Cox et al. have also described the use of GuSCN in methods for conducting nucleic acid hybridization assays and for isolating nucleic acid from cells (EP-A-0-127-327).

Bresser, Doering and Gillespie, DNA, 2:243-254 (1983), reported the use of NaI, and Manser and Gefter, Proc. Nat!. Acad. Sci. USA, 81:2470-2474 (1984) reported the use of ' NaSCN to make DNA or mRNA in biological sources available for trapping and immobilization on nitrocellulose membranes in a state which was suitable for molecular hybridization with DIVA or RNA probes.

Highly useful systems that comprise use of locked nucleic acid ("LNA") oligomers as capturing-probes and detecting oligos has been disclosed in commonly assigned U.S. Patent 6,303,315.

WO 99/14226 relates to modified oligonucleotides containing at least one LNA monomer with improved affinity and specificity towards complementary RNA and DNA oligonucleotides.

WO 00/56916 describes a method for detection of sequence variations of at least one single base difference in a sample containing nucleic acids using a LNA-containing oligonucleotide.

WO 00/56748 describes oligonucleotides containing at least one LNA monomer of the xylo-configuration for detection and isolation of nucleic acids.

### Summary Of The Invention

In one aspect, the present invention provides methods for detecting and/or isolating via hybridisation a nucleic acid molecule having a homopolymeric sequence comprising:
a) treating a sample containing nucleic acid compounds with a lysing buffer comprising a chaotropic agent; and
b) treating said sample with a Locked Nucleic Acid (LNA) oligonucleotide capture probe comprising at least twenty repeating consecutive nucleotides to thereby detect and/or isolate via hybridisation a nucleic acid molecule having said homopolymeric sequence,
wherein said homopolymeric sequence is a poly (A) tail of a eukaryotic mRNA;
and wherein the LNA oligonucleotide capture probe comprises a compound of the formula:

5'-Y^{q}-( X^{p}-Yⁿ)ₘ-X^{P}-Z-3'

wherein X is an LNA monomer, Y is a DNA monomer; Z represents an optional DNA monomer ; p is an integer from about 1 to about 15; n is an integer from about 1 to about 15 or n represents 0; q is an integer from about I to about 10 or q = 0; and m is an integer from about 5 to about 20.

As referred to herein, a repetitive element is a nucleotide sequence (or other similar term) of an oligonucleotide that will start and end with a nucleotide having the same nucleobase substitution (e.g. G) and within those start and end nucleotides most

Said homopolymeric sequence may comprise a minor proportion of other nucleobases or analogues thereof, e.g. the sequence 5'aaaaagaaaaaaa- 3', without substantially affecting the overall homopolymeric nature of the nucleotide sequence.

It also should be appreciated that while in a repetitive sequence substantially all the nucleotide units have the same nucleobase substitution, the nucleotides can otherwise differ within the repetitive sequence. For instance, a sequence can have one or more LNA nucleotide units with the balance of units of the repetitive stretch or sequence being non-LNA DNA or RNA. Suitably, a repetitive base sequence of an oligonucleotide contains one or more LNA units, more preferably 1, 2, 3 or 4 LNA units. The number of preferred LNA units in a repetitive stretch also may vary with the total number of nucleotides in the repetitive stretch; preferably at least about 10, 20,30,40, 50, 60, 70 or 80 percent of the total units of a repetitive stretch will be LNA units, with the balance being non-LNA nucleotides, particularly DNA or RNA units.

As referred to herein, an LNA polynucleotide or oligonucleotide or other similar terms refer to a nucleic acid oligomer that comprises at least one LNA unit. Preferred LNA units are discussed below, including with respect to Formula I below.

Preferred methods of the invention include isolating a nucleic acid molecule having repeating base sequence of at least 20, or 25 or more, of the same nucleotide base in sequence without substantial interruption. Again, in such a target sequence, without substantial interruption indicates that at least about 60, 70 or 80 percent of the total nucleotides of the sequence have the same nucleobase substitution, preferably 90 percent, 95 percent or all the nucleotides of the sequence will have the same nucleobase substitution. In the repetitive sequence of the target oligonucleotide however, typically the entire nucleotides will be the same, not just the nucleobase substitution.

A sample may be provided containing nucleic acid compounds and that sample is captured with an LNA polynucleotide, which is suitably substantially complementary to the target nucleic acid compounds. The sample may be treated with a lysing buffer comprising a chaotropic agent to lyse cellular material in the sample prior to contacting the sample with the LNA polynucleotide capture probe.

Suitably, the LNA/DNA oligonucleotide capture probe is covalently attached to a solid support and after the LNA/DNA oligonucleotide capture probe and complementary repetitive nucleic acid sequences have hybridized to the LNA/DNA capture probe, the solid support is separated from excess material. The solid support is washed to remove excess material.

As mentioned, preferably the LNA polynucleotide capture probe is complementary to a repetitive nucleic acid sequence. Preferably, the LNA oligonucleotide capture probe comprises at least about four to five repeating consecutive nucleic acid bases, more preferably the LNA oligonucleotide capture probe comprises at least about ten repeating consecutive nucleic acid bases, most preferably the LNA/DNA oligonucleotide capture probe comprises at least about twenty to twenty-five repeating nucleotides.

In one aspect of the invention, the LNA/DNA oligonucleotide molecule is complementary to, for example, a polyadenylated nucleic acid sequence, a polythymidine nucleic acid sequence, a polyguanidine nucleic acid sequence, a polyuracil nucleic acid molecule or a polyoytidine molecule.

In another aspect of the invention the -1 residue of the LNA/DNA oligonucleotide molecule 3' and/or 5' end is an LNA molecule. The -1 residue of the LNA/DNA oligonucleotide molecule 3' and/or end can also be a DNA molecule.

Preferably, the LNA/DNA oligonucleotide molecule comprises at least about one or more alpha-L LNA monomers and/or oxy-LNA and/or xylo-LNA or combinations thereof.

In a preferred embodiment, the composition of the desired LNA oligonucleotide capture probe has a ratio of LNA:DNA monomers determined by a Tₘ in the range of between about 55°C to about 60-70 °C when the LNA/DNA oligonucleotide capture probe binds to its complementary target sequence.

In accordance with the invention, the LNA oligonucleotide molecule comprises at least about 25 percent to about 50 percent LNA monomers of the total residues of the LNA oligonucleotide molecule and can comprise at least about two or more consecutive LNA molecules.

In another aspect of the invention, the LNA oligonucleotide molecule comprises modified and non-modified nucleic acid molecules.

In another aspect of the invention, the LNA oligonucleotide molecule comprises moieties such as biotin, or anthraquinone in the 5' position.

In another preferred embodiment, the association constant (Kₐ) of the LNA oligonucleotide molecule is higher than the association constant of the complementary strands of a double stranded molecule.

In another preferred embodiment, the association constant of the LNA oligonucleotide molecule is higher than the disassociation constant (K_{d}) of the complementary strand of the target sequence in a double stranded molecule.

In one aspect of the invention, the LNA oligonucleotide capture probe is complementary to the sequence it is designed to isolate or it is substantially complementary to the desired nucleic acid sequence.

In another aspect, the LNA oligonucleotide capture probe has at least one base pair difference to the complementary sequence it is designed to detect For example, the LNA/DNA oligonucleotide capture probe can detect at least about one base pair difference between the complementary poly-repetitive base sequence and the LNA oligonucleotide capture probe and is useful, for example, for genotyping.

In a preferred embodiment, the LNA oligonucleotide capture probe binds to single-stranded DNA targets, double-stranded DNA target molecules as well as RNA, including secondary structures in RNA molecules.

Preferably, the LNA oligonucleotide capture probe hybridizes to nucleic acid molecules of mammalian cells, other eukaryotic cells, bacteria, viruses, especially for example RNA viruses, fungi, parasites, yeasts, phage.

In another preferred embodiment, the method may be employed for isolating RNA from infectious diseases organisms is provided wherein the genome of the infectious disease organism is comprised of RNA, the method comprising:
providing a sample containing genomic RNA; and,
treating the sample with a lysing buffer containing a chaotropic agent to lyse cellular material in the sample, dissolve the components and denature the genomic RNA in the sample; and,
contacting the genomic RNA released from the sample with at least one capturing LNA oligonucleotide probe, wherein, the capturing probe being substantially complementary to a consecutively repeating nucleic acid base in the genomic RNA.

Preferably, the chaotropic agent is guanidinium thiocyanate and the concentration of the guanidinium thiocyanate is between about 2M to about 5M. The genomic RNA is protected from degradation by RNAse inhibitors in the presence of the chaotropic agent. Preferably the hybridization of the LNA oligonucleotide capture probe with the target sequence protects the RNA from degradation by RNAase's.

In one aspect, its is preferred that the Tₘ of the LNA oligonucleotide capture probe when bound to its complementary genomic RNA sequence is between about 55°C to about 70°C.

The isolation of genomic RNA using the present method is important for extracting, purifying, and using the RNA in different assays well known in the art, such as for example, RT-PCR, and is useful in diagnosing or genotyping for example, retroviruses such as HIV.

In another aspect, the LNA oligonucleotide capture probe comprises a fluorophor moiety and a quencher moiety, positioned in such a way that the hybridized state of the oligonucleotide can be distinguished from the unbound state of the oligonucleotide by an increase in the fluorescent signal from the nucleotide.

In another preferred embodiment, the method of the present invention may be employed for amplifying a target nucleic acid molecule, the nucleotide sequence of which is complementary to the LNA oligonucleotide capture probe. The method comprises, providing a sample containing nucleic acid molecules, which is treated with a lysing buffer comprising a chaotropic agent to lyse cellular material in the sample, dissolve the components and denature the nucleic acids in the sample as set out in the claims. The nucleic acids released from the sample are contacted with at least one LNA oligonucleotide capture probe. After the nucleic acids have contacted the LNA oligonucleotide capture probe and complementary repetitive nucleic acid sequences have hybridized to the LNA capture probe under conditions described in detail in the examples which follow, the solid support is separated from excess material The captured nucleic acids are then subjected to polymerase chain reaction, or linear run-off amplification using primers or a primer to amplify the captured nucleic acid molecules. Various amplifying reactions are well known to one of ordinary skill in the art and include, but are not limited to PCR, RT-PCR, LCR, in vitro transcription, rolling circle PCR, OLA and the like. Multiple primers can also be used in multiplex PCR.

The method as set out in the claims may employ a kit for isolating a target nucleic acid comprising an A oligonucleotide complementary to the target nucleic acid; and a substrate for immobilizing the LNA oligonucleotide. The kit includes a solid surface for immobilizing the LNA oligonucleotide capture probes of the invention. Such surfaces include, but are not limited to, for example, streptavidin coated beads, microchip arrays such as the EURAY^{™} (Exiqon A/S), magnetic beads, plastics, coated particles, coated polymers and the like. Preferably, the solid surface is a polymer support, such as a microtiter plate, polystyrene beads, latex beads, open and closed slides, such as a microfluidic slide described in WO 03036298 A2. The nucleic acids from a sample are released as described above and after the complementary repetitive nucleic acid sequences have hybridized to the LNA capture probe, the solid support is separated from excess material. The solid support is washed to remove excess material.

The captured target nucleic acid is analyzed using methods well known to one of ordinary skill in the art such, for example, PCR, Northern blotting, microarray hybridizations, electrophoresis and the like.

### BRIEF DESCRIPTION OF THE DRAWINGS

Figure 1 is a graph showing the percent recovery of yeast in vitro-transcribed *ACT1* mRNA using LNA/DNA capture probes at varying hybridization temperatures using a buffer containing a chaotropic agent (4M GuSCN). The biotinylated oligo-T capture probes used are shown in the top panel. The percent recovery was calculated from gel electrophoresed dilution series of an RNA standard.
Figure 2 is a graph showing the percent recovery of yeast in vitro-transcribed *SSA4* mRNA using LNA/DNA capture probes at varying hybridization temperatures using a buffer containing a chaotropic agent (4M GuSCN). The biotinylated oligo-T capture probes used are shown in the top panel. The percent recovery was calculated from gel electrophoresed fragments.
Figure 3 is a graph showing the percent recovery of yeast *ACT1* mRNA using LNA/DNA capture probes at varying hybridization temperatures using a high salt buffer 0.5 M NaCl. The biotinylated oligo-T capture probes used are shown in the top panel. The percent recovery was calculated from gel electrophoresed fragments.
Figure 4 is a graph showing biotin-labeled LNA/DNA capture probes immobilized on a streptavidin-coated EURAY^{™} polymer slide and hybridized to 0.1 µM Cy5-oligo-dT₂₀.
Figure 5 is a graph showing biotin-labeled LNA/DNA capture probes immobilized on a streptavidin-coated EURAY^{™} polymer slide and hybridized to 0.1 µM Cy5-oligo-dT₂₀ in 4 M GuSCN buffer.
Figure 6 is a gel (left panel) and a graph (right panel) showing the recovery of in vitro-transcribed yeast SSA4 mRNA in different concentrations of guanidinium thiocyanate (GuSCN).

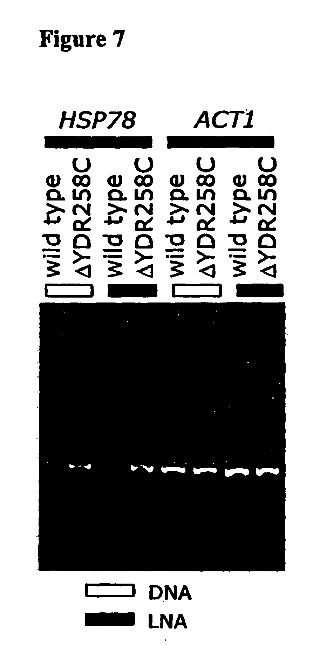

Figure 7 shows RT-PCR analysis of yeast poly(A)⁺RNA. The DNA- (open bars) or LNA oligo(T)- (solid bars) captured poly(A)⁺RNA samples were subjected to RT-PCR 100 ng of poly(A)⁺RNA from either heat shocked wild type cells or heat shocked deltaYDR258C cells were reversed transcribed into first strand cDNA and PCR amplified using specific primer sets for the yeast HSP78 and ACT1, respectively. Five microliter aliquots of the PCR reactions were applied on a native 1 % agarose gel stained with Gelstar.
Figure 8 shows Northern blot analysis of yeast poly(A)⁺RNAs isolated from different yeast strains, probed with 32P-labeled fragments for the yeast genes HSP78 and ACT1, respectively.
Figures 9A and B show capture of SSA4 spike mRNA by AQ-coupled LNA oligo-T capture probes. Solid lines represent LNA capture probes and stipple lines control DNA capture probes. The linker constructions are demonstrated by the following symbols: Diamonds depict AQ₂-HEG₃-, triangles denoe AQ₂-t15-, squares depict AQ₂-c15-, and circles AQ₂-t10-NB5-. Figure 9A demonstrates detection using an LNA probe for SSA4 spike mRNA. Figure 9B demonstrates detection using a DNA probe for SSA4 spike mRNA.
Figure 10 shows titration of polyadenylated SSA4 mRNA captured by AQ-coupled oligo-T capture probes. Solid lines LNA capture probes and stipple lines control DNA capture probes. The linker constructions are demonstrated by the following symbols: Diamonds denote AQ₂-HEG₃-, triangles denote AQ₂-t15-, squares depict AQ₂-c15-, and circles depict AQ₂-t10-NB5-.
Figure 11 shows isolation of poly(A)⁺RNA from heat shocked wild type yeast total RNA followed by specific detection of the SSA4 mRNA using a biotinylated SSA4-specific detection probe.
Figure 12 shows recovery of ACT1 in vitro spike mRNA after hybridisation in different NaCl-salt concentrations. DNA oligo-dT (open bars) and LNA oligo-T (solid bars).
Figure 13 shows quantification of isolated poly(A)⁺RNA from C. elegans worms, analysed by native agarose gel eletrophoresis as captured by either the LNA_2.T (solid bars) or DNA-dt₂₀ (open bars) capture probes. The hatched bar indicates a negative control performed without oligo-T capture probe during the isolation the poly(A)⁺RNA.
Figure 14 shows Northern blot analysis of poly(A)⁺RNA isolated from increasing amounts of *C. elegans* worm extracts probed with 32P-labeled fragments for the *C. elegans* genes RPL-21 and 26S rRNA, respectively.

### DETAILED DESCRIPTION OF THE INVENTION

This invention relates to a novel method for detecting and isolating nucleic acids released from a lysed complex biological mixture containing nucleic acids.

The methods of the present invention enable convenient assay and isolation for a nucleic acid suspected of being present in cells, parts of cells or virus, i.e. target nucleic acid(s). Such methods include lysing the cells in a hybridization medium comprising a strong chaotropic agent, hybridizing the lysate under hybridization conditions with a locked nucleic acid (LNA) having a nucleotide sequence substantially complementary to a nucleotide sequence suspected to be present in the cells, and determining the extent of hybridization.

The "target nucleic acid" means the nucleotide sequence of deoxyribonucleic acid (DNA), ribonucleic acid (RNA) (including ribosomal ribonucleic acid (rRNA), poly(A)⁺ mRNA, transfer RNA, (tRNA), small nuclear (snRNA), telomerase associated RNA, ribozymes etc.) whose presence is of interest and whose presence or absence is to be detected in the hybridization assay. Of particular interest is the detection and isolation of polyadenylated mRNA or particular mRNAs which may be of eukaryotic, prokaryotic, Archae or viral origin. Importantly, the invention may assist in the diagnosis of various infectious diseases by assaying for particular sequences known to be associated with a particular microorganism. The target nucleic acid may be provided in a complex biological mixture of nucleic acid (RNA, DNA and/or rRNA) and non-nucleic acid. The target nucleic acids of primary preference are RNA molecules and, in particular polyadenylated mRNAs or rRNAs such as the 16S or 23S rRNA. If target nucleic acids of choice are double stranded or otherwise have significant secondary and tertiary structure, they may need to be heated prior to hybridization. In this case, heating may occur prior to or after the introduction of the nucleic acids into the hybridization medium containing the capturing probe. It may also be desirable in some cases to extract the nucleic acids from the complex biological samples prior to the hybridization assay to reduce background interference by any methods known in the art

The hybridization and extraction methods of the present invention may be applied to a complex biological mixture of nucleic acid (RNA and/or DNA) and non-nucleic acid. Such a complex biological mixture includes a wide range of eukaryotic and prokaryotic cells, including protoplasts; or other biological materials which may harbour target nucleic acids. The methods are thus applicable to tissue culture animal cells, animal cells (e.g., blood, serum, plasma, reticulocytes, lymphocytes, urine, bone marrow tissue, cerebrospinal fluid or any product prepared from blood or lymph) or any type of tissue biopsy (e.g. a muscle biopsy, a liver biopsy, a kidney biopsy, a bladder biopsy, a bone biopsy, a cartilage biopsy, a skin biopsy, a pancreas biopsy, a biopsy of the intestinal tract, a thymus biopsy, a mammae biopsy, an uterus biopsy, a testicular biopsy, an eye biopsy or a brain biopsy, homogenized in lysis buffer), plant cells or other cells sensitive to osmotic shock and cells of bacteria, yeasts, viruses, mycoplasmas, protozoa, rickettsia, fungi and other small microbial cells and the like. The assay and isolation procedures of the present invention are useful, for instance, for detecting non-pathogenic or pathogenic micro-organisms of interest. By detecting specific hybridization between nucleotide probes of a known source and nucleic acids resident in the biological sample, the presence of the micro-organisms may be established.

Solutions containing high concentrations of guanidine, guaninium thiocyanate or certain other chaotropic agents and detergents are capable of effectively lysing prokaryotic and eukaryotic cells while simultaneously allowing specific hybridization of LNA probes to the released endogenous nucleic acid. The solutions need not contain any other component other than common buffers and detergents to promote lysis and solubilization of cells and nucleic acid hybridization.

The LNA oligonucleotides of the invention provide surprising advantages over previously described methods for isolating nucleic acids. For example, the oligonucleotides can hybridize to complementary sequences in the presence of high concentrations of salts or chaotropic agents, whereas DNA oligonucleotides cannot hybridize to their complementary sequences in the presence of high salts or chaotropic agents. Furthermore, the melting temperature (Tₘ) of the LNA oligonucleotides are not affected by the high salt concentrations or presence of chaotropic agents. This has the further advantage when the nucleic acids to be isolated are RNA's. As is well-known to anyone of ordinary skill in the art, many precautions are required for working with RNA's due to the presence of RNAase's which rapidly degrade RNA samples. However, the use of the LNA oligonucleotides in high salt concentrations or in the presence of chaotropic agents also inhibits the activity of RNAases thereby allowing a higher yield of isolated RNA sample for use in diagnostic tests or other appropriate methodologies.

If extraction procedures are employed prior to hybridization, organic solvents such as phenol and chloroform may be used in techniques employed to isolate nucleic acid. Traditionally, organic solvents, such as phenol or a phenol-chloroform combination are used to extract nucleic acid, using a phase separation (Ausubel et al. in Current Protocols in Molecular Biology, pub. John Wiley & Sons (1998)). These methods may be used effectively with the lysis solutions of the present invention; however, an advantage of the methods of the present invention is that tedious extraction methods are not necessary, thus improving the performance of high throughput assays. Preferably, the lysis buffer/hybridization medium will contain standard buffers and detergents to promote lysis of cells while still allowing effective hybridization of LNA probes. A buffer such as sodium citrate, Tris-HCI, PIPES or HEPES, preferably Tris-HCI at a concentration of about 0.05 to 0.1 M can be used. The hybridization medium will preferably also contain about 0.05 to 0.5% of an ionic or non-ionic detergent, such as sodium dodecylsulphata (SDS) or Sarkosyl (Sigma Chemical Co., St. Louis, Mo.) and between and 10 mM EDTA. Other additives may also be included, such as volume exclusion agents which include a variety of polar water-soluble or swellable agents, such as anionic polyacrylate or polymethacrylate, and charged saccharidic polymer, such as dextran sulphate and the like. Specificity or the stringency of hybridization may be controlled, for instance, by varying the concentration and type of chaotropic agent and the NaCl concentration, which is typically between 0 and 1 M NaCl, such as 0.1,0.2,0.3,0.4,0.5,0.6,0.7, 0.8, 0.9 or 1.0.

Chaotropic agents which disturb the secondary and tertiary structure of proteins, for example, guanidine salts such as guanidine hydrochloride (GuHCl) and thiocyanate (GuSCN), or urea, lithium chloride and other thiocyanates may be used in combination with detergents and reducing agents such as beta-mercaptoethanol or DTT to dissociate natural occurring nucleic acids and inhibit nucleases. The use of chaotropic agents in the extraction and hybridization of nucleic acids is described in EP Publication No. 0 127 327.

An LNA substantially complementary to the target nucleic acid is introduced in the hybridization process. The term "an LNA substantially complementary to the target nucleic acid" refers to a polynucleotide or oligonucleotide containing at least one LNA monomer and a variable number of naturally occurring nucleotides or their analogues, such as 7-deazaguanosine or inosine, sufficiently complementary to hybridize with the target nucleic acid such that stable and specific binding occurs between the target and the complementary nucleic acid under the hybridization conditions. Therefore, the LNA sequence need not reflect the exact sequence of the target nucleic acid. For example, a non-complementary nucleotide fragment may be attached to a complementary nucleotide fragment or alternatively, non-complementary bases or longer sequences can be interspersed into the complementary nucleic acid, provided that the complementary nucleic acid sequence has sufficient complementarity with the sequence of the target nucleic acid to hybridize therewith, forming a hybridization complex and further is capable of immobilizing the target nucleic acid to a solid support as will be described in further detail below. A capturing probe to bind the released nucleic acids can be linked to a group (e.g. biotin, fluorescein, magnetic micro-particle etc.). Alternatively, the capturing probe can be permanently bound to a solid phase or particle in advance e.g. by anthraquinone photochemistry (WO 96/31557).

The terms "complementary" or "complementarity", as used herein, refer to the natural binding of polynucleotides under permissive salt and temperature conditions by base-pairing. For example, for the sequence "A-G-T" binds to the complementary sequence "T-C-A". Complementarity between two single-stranded molecules may be "martial", in which only some of the nucleic acids bind, or it may be complete, when total complementarity exists between the single stranded molecules.

As used herein, "substantially complementary" refers to the oligonucleotides of the invention that are at least about 50% homologous to target nucleic acid sequence they are designed to detect, more preferably at least about 60%, more preferably at least about 70%, more preferably at least about 80%, more preferably at least about 90%, more preferably at least about 90%, more preferably at least about 95%, most preferably at least about 99%.

The term "homology", as used herein, refers to a degree of complementarity. There may be partial homology or complete homology (i.e., identity). A partially complementary sequence is one that at least partially inhibits an identical sequence from hybridizing to a target nucleic acid, it is referred to using the functional term "substantially homologous." The inhibition of hybridization of the completely complementary sequence to the target sequence may be examined using a hybridization assay (Southern or northern blot, solution hybridization and the like) under conditions of low stringency. A substantially homologous sequence or probe will compete for and inhibit the binding (i.e., the hybridization) of a completely homologous sequence or probe to the target sequence under conditions of low stringency. This is not to say that conditions of low stringency are such that non-specific binding is permitted; low stringency conditions require that the binding of two sequences to one another be a specific (i.e., selective) interaction. The absence of non-specific binding may be tested by the use of a second target sequence which lacks even a partial degree of complementarity (e.g., less than about 30% identity); in the absence of non-specific binding, the probe will not hybridize to the second non-complementary target sequence.

As known in the art, numerous equivalent conditions may be employed to comprise either low or high stringency conditions. Factors such as the length and nature (LNA, DNA, RNA, nucleobase base composition) of the sequence, nature of the target (DNA, RNA, base composition, presence in solution or immobilization, etc.), and the concentration of the salts and other components, such as chaotropic agents (e.g., the presence or absence of formamide, dextran sulfate and/or polyethylene glycol) are considered, and the hybridization solution may be varied to generate conditions of either low or high stringency different from, but equivalent to, the conditions discussed infra.

The term "stringent conditions", as used herein, is the "stringency" which occurs within a range from about Tₘ-5° C. (5° C. below the melting temperature (Tₘ) of the probe) to about 20° C. to 25° C. below Tₘ. As will be understood by those of skill in the art, the stringency of hybridization may be altered in order to identify or detect identical or related polynucleotide sequences.

As used in the methods disclosed herein, the LNA oligomers comprise a repeat element of the following:

5'-Y^{q}-(X^{p}-Y^{m})ₘ-X^{p}-Z-3'

wherein X is an LNA monomer, Y is a DNA monomer, Z represents an optional DNA monomer; p is an integer from about 1 to about 15; n is an integer from about 1 to about 15 or n represents 0; q is an integer from about 1 to about 10 or q = 0; and m is an integer from about 5 to about 20. By way of example:
5'-TttTttTttTttTtt-3'
   wherein T = LNA thymidine analogue, t = DNA thymidine.
5'-GggGggGggGggGgg-3'
   wherein G = LNA guanidine analogue, g = DNA guanidine.

The LNA oligomers can be comprised of a repeating sequence of thymidines with a guanine or any other nucleobase located in any position of the oligomers. As an illustrative example which is not meant to limit or construe the invention in any way the LNA oligomers can be selected from Table 3 and may optionally comprise a G, A, U or C in any position of the oligomers. For example:
5 '-GttTttTttTttTtg-3'
   wherein G = LNA guanidine analogue, T = LNA thymidine analogue, t = DNA thymidine, g = DNA guanidine.
5'-TttTttTttTttTgt-3'
   wherein T = LNA thymidine analogue, t = DNA thymidine, g = DNA guanidine.

In accordance with the invention, any combination of LNA bases and DNA bases and/or nucleobases can be used in any position as the above examples illustrate. The repeating element can be located in any position of the oligonucleotide, such as but not limited to, for example the 5' end, 3' end, and/or any position in between the 5' and 3' end of the oligonucleotide. The terms "LNA oligomers", "LNA oligonucleotide capture probe" and "mixmer" will be used interchangeably and refers to the oligonucleotides of the invention which are comprised of at least one DNA or RNA nucleic acid.

An attractive possibility of the invention is the use of different LNA-oligomers directed against different sequences in the genome which are spotted in an array format and permanently affixed to the surface (Nature Genetics, suppl. vol. 21, Jan 1999, 1-60 and WO 96/31557). Such an array can subsequently be incubated with the mixture of the lysis buffer/hybridization medium containing dissolved cells and a number of suitable detection LNA-probes. The lysis and hybridization would then be allowed to occur, and finally the array would be washed and appropriately developed. The result of such a procedure would be a semi-quantitative assessment of a large number of different target nucleic acids.

As for DNA or RNA the degree of complementarity required for formation of a stable hybridization complex (duplex) which includes LNA varies with the stringency of the hybridization medium and/or wash medium. The complementary nucleic acid may be present in a pre-prepared hybridization medium or introduced at some later point prior to hybridization.

The hybridization medium is combined with the biological sample to facilitate lysis of the cells and nucleic acid base-pairing. Preferably, the volume of biological sample to the volume of the hybridization medium will be about 1:10.

It is intended and an advantage of the hybridization methods of the present invention that they be carried out in one step on complex biological samples. However, minor mechanical or other treatments may be considered under certain circumstances. For example, it may be desirable to clarify the lysate before hybridization such as by slow speed centrifugation or filtration or to extract the nucleic acids before hybridization as described above.

The hybridization assay of the present invention can be performed by any method known to those skilled in the art or analogous to immunoassay methodology given the guidelines presented herein. Preferred methods of assay are the sandwich assays and variations thereof and the competition or displacement assay. Hybridization techniques are generally described in "Nucleic Acid Hybridization, A Practical Approach," Ed. Hames, B. D. and Higgins, S. J., IRL Press, 1985; Gall and Pardue (1969), Proc. Natl. Acad. Sci., U.S.A., 63:378-383; and John, Burnsteil and Jones (1969) Nature, 223:582-587. Further improvements in hybridization techniques will be well known to the person of skill in the art and can readily be applied.

In this invention the capturing LNA-probe is typically attached to a solid surface e.g. the surface of a microtiter tray well or a microarray support or a microbead. Therefore, a convenient and very efficient washing procedure can be performed thus opening the possibility for various enzymatically based reactions that may add to the performance of the invention. Most noteworthy is the possibility that the sensitivity of the hybridization assays may be enhanced through use of a nucleic acid amplification system which multiplies the target nucleic acid being detected. Examples of such systems include the polymerase chain reaction (PCR) system and the ligase chain reaction (LCR) system. Other methods recently described and known to the person of skill in the art are the nucleic acid sequence based amplification (NASBA^{™}, Cangene, Mississauga, Ontario) and Q Beta Replicase systems. PCR is a template dependent DNA polymerase primer extension method of replicating selected sequences of DNA. The method relies upon the use of an excess of specific primers to initiate DNA polymerase replication of specific sub-sequences of a DNA polynucleotide followed by repeated denaturation and polymerase extension steps. The PCR system is well known in the art (see US 4,683,195 and US 4,683,202). For additional information regarding PCR methods, see also PCR Protocols: A Guide to Methods and Applications, ed. Innis, Gelland, Shinsky and White, Academic Press, Inc. (1990). Reagents and hardware for conducting PCR are available commercially through Perkin-Elmer/Cetus Instruments of Norwalk, Conn.

LCR, like PCR, uses multiple cycles of alternating temperature to amplify the numbers of a targeted sequence of DNA. LCR, however, does not use individual nucleotides for template extension. Instead, LCR relies upon an excess of oligonucleotides which are complementary to both strands of the target region. Following the denaturation of a double stranded template DNA, the LCR procedure begins with the ligation of two oligonucleotide primers complementary to adjacent regions on one of the target strands. Oligonucleotides complementary to either strand can be joined. After ligation and a second denaturation step, the original template strands and the two newly joined products serve as templates for additional ligation to provide an exponential amplification of the targeted sequences. This method has been detailed in Genomics, 4:560-569 (1989). As other amplification systems are developed, they may also find use in this invention.

As an illustrative example, the methods of the invention make use of the mixmers to hybridize to nucleic acids from a sample. If the sample is RNA, the presence of the chaotropic agent, and/or the hybridization to the mixmer protects the RNA from degradation by RNAases, for example RNAaseH. The sample is then suitably used in, for example RT_PCR, using primers of interest to amplify a desired gene or desired sequences.

The Oligomer ligation assay (OLA) or "Oligonucleotide Ligation Assay" (OLA) uses two oligonucleotides which are designed to be capable of hybridizing to abutting sequences of a single strand of a target molecules. One of the oligonucleotides is biotinylated, and the other is detectably labeled. If the precise complementary sequence is found in a target molecule, the oligonucleotides will hybridize such that their termini abut, and create a ligation substrate that can be captured and detected. OLA is capable of detecting single nucleotide polymorphisms and may be advantageously combined with PCR as described by Nickerson et al. (1990) Proc. Natl. Acad. Sci. USA 87:8923. In this method, PCR is used to achieve the exponential amplification of target DNA, which is then detected using OLA.

The hybridization medium and processes of the present invention are uniquely suited to a one-step assay. The medium may be pre-prepared, either commercially or in the laboratory to contain all the necessary components for hybridization. For instance, in a sandwich assay the medium could comprise a chaotropic agent (e.g. guanidine thiocyanate), desired buffers and detergents, a capturing LNA-probe bound to a solid support such as a microbead, and a detecting nucleic acid which could also be an LNA. This medium then only needs to be combined with the sample containing the target nucleic acid at the time the assay is to be performed. Once hybridization occurs the hybridization complex attached to the solid support may be washed and the extent of hybridization determined.

Sandwich assays are commercially useful hybridization assays for detecting or isolating nucleic acid sequences. Such assays utilize a "capturing" nucleic acid covalently immobilized to a solid support and labeled "signal" nucleic acid in solution. The sample will provide the target nucleic acid. The "capturing" nucleic acid and "signal" nucleic acid probe hybridize with the target nucleic acid to form a "sandwich" hybridization complex. To be effective, the signal nucleic acid is designed so that it cannot hybridize with the capturing nucleic acid, but will hybridize with the target nucleic acid in a different position than the capturing probe.

Virtually any solid surface can be used as a support for hybridization assays, including metals and plastics. Two types of solid surfaces are generally available, namely:
a) Membranes, polystyrene beads, nylon, Teflon, polystyrene/latex beads, latex beads or any solid support possessing an activated carboxylate, sulfonate, phosphate or similar activatable group are suitable for use as solid surface substratum to which nucleic acids or oligonucleotides can be immobilized.
b) Porous membranes possessing pre-activated surfaces which may be obtained commercially (e.g., Pall Immunodyne Immunoaffinity Membrane, Pall BioSupport Division, East Hills, N.Y., or Immobilon Affinity membranes from Millipore, Bedford, Mass.) and which may be used to immobilize capturing oligonucleotides. Microbeads, including magnetic beads, of polystyrene, teflon, nylon, silica or latex may also be used.

However, use of the generally available surfaces mentioned in a) and b) often creates background problems, especially when complex mixtures of nucleic acids and various other dissolved bio-molecules are analysed by hybridization. A significant decrease in the background has been obtained when the catching-probe is covalently attached to solid surfaces by the anthraquinone (AQ) based photo-coupling method described in the art (see WO 96/31557). This method allows the covalent attachment of the catching LNA-oligo to the surface of most polymer materials - including various relatively thermostable polymers such as polycarbonate and polyethylene - as well as treated glass surfaces. Thus by use of the AQ photo-coupling method, the capturing LNA-probe can be attached to surfaces of containers that is compatible with present day PCR amplification techniques. It is preferred to covalently attach the LNA-probe and the 5'-end or the 3'-end to the anthraquinone via a linker, such as one or two hexaethylene trimer (HEG3) linker units. Preferably, said linker connecting the 5'-end of the LNA oligonucleotide probe to the anthraquinone moiety is selected from the group comprising one or more of a hexaethylene monomer, dimer , trimer, tetramer, pentamer, hexamer, or higher hexaethylene polymer, a poly-T sequence of 10-50 nucleotides in length or a poly-C sequence of 10-50 nucleotides in length or longer; or a non-base sequence of 10-50 nucleotide units in length.

Sequences suitable for capturing or signal nucleic acids for use in hybridization assays can be obtained from the entire sequence or portions thereof of an organism's genome, from messenger RNA, or from cDNA obtained by reverse transcription of messenger RNA. Methods for obtaining the nucleotide sequence from such obtained sequences are well known in the art (see Ausubel et al. in Current Protocols in Molecular Biology, pub. John Wiley & Sons (1998), and Sambrook et et al. Molecular Cloning, A Laboratory Manual, Cold Spring Harbor Laboratory Press, 1989). Furthermore, a number of both public and commercial sequence databases are accessible and can be approached to obtain the relevant sequences.

Once the appropriate sequences are determined, LNA probes are preferably chemically synthesized using commercially available methods and equipment as described in the art *(*Tetrahedron, 1998, 54, 3607-30.). For example, the solid phase phosphoramidite method can be used to produce short LNA probes. (Caruthers et al., Cold Spring Harbor Symp. Quant. Biol., 47:411-418 (1982), and Adams et al., J. Am. Chem. Soc., 105:661 (1983).

When synthesizing a probe for a specific target, the choice of nucleotide sequence will determine the specificity of the test. For example, by comparing DNA sequences from several virus isolates, one can select a sequence for virus detection that is either type specific or genus specific. Comparisons of DNA regions and sequences can be achieved using commercially available computer programs.

The determination of the extent of hybridization may be carried out by any of the methods well-known in the art. If there is no detectable hybridization, the extent of hybridization is thus 0. Typically, labelled signal nucleic acids are used to detect hybridization. Complementary nucleic acids or signal nucleic acids may be labelled by any one of several methods typically used to detect the presence of hybridized polynucleotides. The most common method of detection is the use of ligands which bind to labelled antibodies, fluorophores or chemiluminescent agents. However, probes may also be labelled with ³H, ¹²⁵I, ³⁵S ¹⁴C. ³³P or ³²P and subsequently detected by autoradiography. The choice of radioactive isotope depends on research preferences due to ease of synthesis, varying stability, and half lives of the selected isotopes. Other labels include antibodies which can serve as specific binding pair members for a labelled ligand. The choice of label depends on sensitivity required, ease of conjugation with the probe, stability requirements, and available instrumentation.

LNA-probes are typically labelled during synthesis. The flexibility of the phosphoramidite synthesis approach furthermore facilitates the easy production of LNAs carrying all commercially available linkers, fluorophores and labelling-molecules available for this standard chemistry. LNA may also be labelled by enzymatic reactions e.g. by kinasing.

Situations can be envisioned in which the detection probes are DNA or RNA. Such probes can be labelled in various ways depending on the choice of label. Radioactive probes are typically made by using commercially available nucleotides containing the desired radioactive isotope. The radioactive nucleotides can be incorporated into probes by several means such as by nick translation of double-stranded probes; by copying single-stranded M13 plasmids having specific inserts with the Klenow fragment of DNA polymerase in the presence of radioactive dNTP; by transcribing cDNA from RNA templates using reverse transcriptase in the presence of radioactive dNTP; by transcribing RNA from vectors containing SP6 promoters or T7 promoters using SP6 or T7 RNA polymerase in the presence of radioactive rNTP; by tailing the 3' ends of probes with radioactive nucleotides using terminal transferase; or by phosphorylation of the 5' ends of probes using [³²P]-ATP and polynucleotide kinase.

Non-radioactive probes are often labelled by indirect means. Generally, a ligand molecule is covalently bound to the probe. The ligand then binds to an anti-ligand molecule which is either inherently detectable or covalently bound to a signal system, such as a detectable enzyme, a fluorescent compound, or a chemiluminescent compound. Ligands and anti-ligands may be varied widely. Where a ligand has a natural anti-ligand, for example, biotin, thyroxine, and cortisol, it can be used in conjunction with the labelled, naturally occurring anti-ligands. Alternatively, any haptenic or antigenic compound can be used in combination with an antibody.

As is the case of DNA, LNA-probes can also be conjugated directly to signal generating compounds, e.g., by conjugation with an enzyme or fluorophore. Enzymes of interest as labels will primarily be hydrolases, particularly phosphatases, esterases and glycosidases, or oxidoreductases, particularly peroxidases. Fluorescent compounds include fluorescein and its derivatives, rhodamine and its derivatives, dansyl, umbelliferone, etc. Chemiluminescent compounds include luciferin, AMPPD ([3-(2'-spiroamantane)-4-methoxy-4-(3'-phosphoryloxy)-phenyl-1,2-dioxetane]) and 2,3-dihydrophthalazinediones, e.g., luminol.

The amount of labelled probe which is present in the hybridization medium or extraction solution may vary widely. Generally, substantial excesses of probe over the stoichiometric amount of the target nucleic acid will be employed to enhance the rate of binding of the probe to the target DNA. Treatment with ultrasound by immersion of the reaction vessel into commercially available sonication baths can often accelerate the hybridization rates.

After hybridization at a temperature and time period appropriate for the particular hybridization solution used, the support to which the capturing LNA-probe:target nucleic acid hybridization complex is attached is introduced into a wash solution typically containing similar reagents (e.g., sodium chloride, buffers, organic solvents and detergent), as provided in the hybridization solution. These reagents may be at similar concentrations as the hybridization medium, but often they are at lower concentrations when more stringent washing conditions are desired. The time period for which the support is maintained in the wash solutions may vary from minutes to several hours or more.

Either the hybridization or the wash medium can be stringent. After appropriate stringent washing, the correct hybridization complex may now be detected in accordance with the nature of the label.

The probe may be conjugated directly with the label. For example, where the label is radioactive, the probe with associated hybridization complex substrate is exposed to X-ray film. Where the label is fluorescent, the sample is detected by first irradiating it with light of a particular wavelength. The sample absorbs this light and then emits light of a different wavelength which is picked up by a detector (Physical Biochemistry, Freifelder, D., W. H. Freeman & Co. (1982), pp. 537-542). Where the label is an enzyme, the sample is detected by incubation on an appropriate substrate for the enzyme. The signal generated may be a coloured precipitate, a coloured or fluorescent soluble material, or photons generated by bioluminescence or chemiluminescence. The preferred label for probe assays generates a coloured precipitate to indicate a positive reading, e.g. horseradish peroxidase, alkaline phosphatase, calf intestine alkaline phosphatase, glucose oxidase and beta-galactosidase. For example, alkaline phosphatase will dephosphorylate indoxyl phosphate which will then participate in a reduction reaction to convert tetrazolium salts to highly coloured and insoluble formazans.

Detection of a hybridization complex may require the binding of a signal generating complex to a duplex of target and probe polynucleotides or nucleic acids. Typically, such binding occurs through ligand and anti-ligand interactions as between a ligand-conjugated probe and an anti-ligand conjugated with a signal. The binding of the signal generation complex is also readily amenable to accelerations by exposure to ultrasonic energy.

The label may also allow indirect detection of the hybridization complex. For example, where the label is a hapten or antigen, the sample can be detected by using antibodies. In these systems, a signal is generated by attaching fluorescent or enzyme molecules to the antibodies or in some cases, by attachment to a radioactive label. (Tijssen, P., "Practice and Theory of Enzyme Immunoassays," Laboratory Techniques in Biochemistry and Molecular Biology, Burdon, R. H., van Knippenberg, P.H., Eds., Elsevier (1985), pp. 9-20.)

In the present context, the term "label" thus means a group which is detectable either by itself or as a part of an detection series. Examples of functional parts of reporter groups are biotin, digoxigenin, fluorescent groups (groups which are able to absorb electromagnetic radiation, e.g. light or X-rays, of a certain wavelength, and which subsequently reemits the energy absorbed as radiation of longer wavelength; illustrative examples are dansyl (5-dimethylamino)-1-naphthalenesulfonyl), DOXYL (N-oxyl-4,4-dimethyloxazolidine), PROXYL (N-oxyl-2,2,5,5-tetramethylpyrrolidine), TEMPO (N-oxyl-2,2,6,6-tetramethylpiperidine), dinitrophenyl, acridines, coumarins, Cy3 and Cy5 (trademarks for Biological Detection Systems, Inc.), erytrosine, coumaric acid, umbelliferone, Texas Red, rhodamine, tetramethyl rhodamine, Rox, 7-nitrobenzo-2-oxa-1-diazole (NBD), pyrene, fluorescein, Europium, Ruthenium, Samarium, and other rare earth metals), radioisotopic labels, chemiluminescence labels (labels that are detectable via the emission of light during a chemical reaction), spin labels (a free radical (e.g. substituted organic nitroxides) or other paramagnetic probes (e.g. Cu²⁺, Mg²⁺) bound to a biological molecule being detectable by the use of electron spin resonance spectroscopy), enzymes (such as peroxidases, alkaline phosphatases, (β-galactosidases, and glycose oxidases), antigens, antibodies, haptens (groups which are able to combine with an antibody, but which cannot initiate an immune response by themselves, such as peptides and steroid hormones), carrier systems for cell membrane penetration such as: fatty acid residues, steroid moieties (cholesteryl), vitamin A, vitamin D, vitamin E, folic acid peptides for specific receptors, groups for mediating endocytose, epidermal growth factor (EGF), bradykinin, and platelet derived growth factor (PDGF). Especially interesting examples are biotin, fluorescein, Texas Red, rhodamine, dinitrophenyl, digoxigenin, Ruthenium, Europium, Cry5, Cy3, etc.

Non-isotopic nucleic acid detection and signal amplification methods to increase the sensitivity in nucleic acid hybridisation-based assays

Hybridization-based assays have proved as highly valuable tools in basic research, drug development as well as in diagnostics, and have significantly advanced the studies of gene structure and function at the level of individual genes as well as functional genomics research in a global scale. For specific detection of antigens and nucleic acids in a wide variety of hybridisation-based assays, such as: (i) in situ hybridisation, (ii) immunohistochemistry, (iii) detection and quantification of specific nucleic acid sequences by Southern blot, slot blot, dot blot, Northern blot analyses, respectively, or (iv) high-density DNA microarray techniques, both fluorescence and enzymatic procedures are commonly used. In hybridisation-based nucleic acid detection, the nonisotopic detection methods have gradually replaced radioactive reagents. The currently used nucleic acid detection methods are most often based on (i) chemiluminiscence using enzyme-conjugated (e.g. alkaline phosphatase or horse radish peroxidase) nucleic acid probes, (ii) bioluminescence using firefly or bacterial luciferase or green fluorescent protein as reporter molecule, (iii) ligands, such as digoxigenin (DIG) or fluorescein isothiocyanate (FITC) combined with enzyme-conjugated anti-ligand antibodies or (iv) biotin-labeled nucleic acid probes combined with enzyme-conjugated streptavidin or avidin.

Recently, several methods have been developed to amplify the detection signals in hybridisation assays. Bobrov et al. (Bobrov, M.N., Harris, T.D., Shaufghnessy, K.J. and Litt, G.J. 1989. Catalyzed reporter deposition, a novel method of signal amplification. Application to immunoassays. J. Immunol. Methods 125: 279-285) introduced the catalysed reporter deposition (CARD) method, which is based on the deposition of a large number of haptenized tyramide molecules by peroxidase activity. Van Gijlswik et al 1997 (van Gijlswik, R.P.M., Zijlmans, H.J.M.A.A., Wiegant, J., Bobrow, M.N., Erickson, T.J., Adler, K.E., Tanke, H.J. and Raap, A.K. 1997. Fluorochrome-labeled Tyramides: Use in Immunocytochemistry and Fluorescence In Situ Hybridization. J. Histochem. Cytochem. 45(3): 375-382) teach the use of fluorochrome-labeled tyramides in highly sensitive detection of antigens and nucleic acid sequences compared to conventional methods. As an alternative to tyramide signal amplification (TSA) based methods, Stears *et al.* (Stears, R.L., Getts, R.C. and Gullans, S.R 2000. A novel, sensitive detection system for high-density microarrays using dendrimer technology. Physiol. Genomics 3: 93-99), have developed a highly sensitive detection system based on the use of a fluorescent oligonucleotide dendrimeric signal amplification system. Essentially, the dendrimer detection system involves labelling of the detection probe population, i.e. first strand cDNA reverse transcribed from total or mRNA, using an RT primer (e.g. oligo(dT)) with a capture sequence complementary to the capture sequence on the free arm of the fluorescent dendrimer. After hybridisation of the cDNA target nucleic acid population onto a microarray, comprising an array of capture probes, and washing of the unhybridized nucleic acids, the microarray is developed using the fluorescent dendrimer detection system, based on the specific hybridisation of the two complementary capture sequences; the ones on the cDNA targets and the dendrimer free arm nucleotide sequence, respectively. By using different capture sequences on the dendrimer free arm, different, labelled dendrimers can be developed enabling multiplexing with different fluorochromes, f.ex. comparative microarrays hybridisations using two fluors. In a hybridization reaction, signal intensity is determined by the amount of label that can be localized at the reaction site. The dendrimers can be labeled with an average of at least 200 labels, and thus the result is up to a 200-fold passive enhancement of signal intensity.

In regard to the isolation of RNA, it has been described (US 5,376,529) that a chaotropic agent, such as a salt of isothiocyanate (e.g. guanidine thiocyanate) does not provide for the complete disruption of protein and nucleic acid interactions, and thus prevents optimal hybridization. A significant increase in hybridization was reported to occur when heat is applied to the hybridization solution containing the chaotropic agent and target nucleic acid. Previously, researchers have attempted to keep hybridization temperatures low to maintain stability of the reactants. See Cox et al., EP Application No. 84302865.5. However, the significantly increased thermal stability of LNA/DNA and LNA/RNA heteroduplexes makes hybridization with LNA-probes feasible at elevated temperatures. Thus the present invention provides a method for increasing the sensitivity of ribonucleic acid detection assays and for simplifying the steps of the assays. The processes for conducting nucleic acid hybridizations wherein the target nucleic acid is RNA comprise heating a nucleic acid solution or sample to an elevated temperature e.g. 65 -70°C as described in the art (US 5,376,529). The nucleic acid solution of the present invention will comprise a chaotropic agent, a target nucleic acid, and an LNA substantially complementary to the target nucleic acid of interest. The nucleic acid solution will be heated to fully disrupt the protein and nucleic acid interactions to maximize hybridization between the LNA and its target

When very high affinity LNA probes are used, hybridization may take place even at the increased temperature needed to fully disrupt DNA:DNA and DNA:RNA interactions. The solution is then cooled until the complementary nucleic acid has hybridized with the target nucleic acid to form a hybridization complex.

These methods are additionally advantageous because they allow for minimal handling of the samples and assay reagents. A ready-to-use reagent solution may be provided, for example, which would contain a chaotropic agent, other appropriate components such as buffers or detergents, a capturing LNA-probe bound to a solid support, and a signal or detection LNA (or nucleic acid), both capable of hybridizing with a target nucleic acid. Conveniently, a complex biological sample suspected of containing a target nucleic acid can be directly combined with the pre-prepared reagent for hybridization, thus allowing the hybridization to occur in one step. The combined solution is heated as described herein and then cooled until hybridization has occurred. The resulting hybridization complex is then simply washed to remove unhybridized material, and the extent of hybridization is determined.

Kits for the extraction of and hybridization of nucleic acids, e.g. mRNA, are also contemplated. Such kits would contain at least one vial containing an extraction solution or a hybridization medium which comprises a strong chaotropic agent and a capturing LNA-probe bound to a solid support. Detergents, buffer solutions and additional vials which contain components to detect target nucleic acids may also be included.

When used herein, the terms "LNA " or "capturing LNA-probe" refer to oligomers comprising at least one nucleoside analogue, preferably having a 2'-O,4'-C bridge, preferably a methyleneoxy biradical described in United States Patent 6,268,490 (Imanishi, et al.), or the corresponding methylenethio biradical or a methyleneamino biradical, or an ethyleneoxy biradical as described in EP 1 152 009 (Sankyo Company, Limited), or a compound of the general formula I 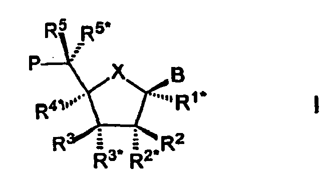 wherein X is selected from -O-, -S-, -N(R^{N*})-, -CR⁶(R^{6*})-;
B is selected from nucleobases;
P designates the radical position for an intemucleoside linkage to a succeeding monomer, or a 5'-terminal group, such internucleoside linkage or 5'-terminal group optionally including the substituent R⁵;
R³ or R^{3*} is P^{*} which designates an intemucleoside linkage to a preceding monomer, or a 3'-terminal group;
R^{4*} and R^{2*} together designate a biradical consisting of 1-4 groups/atoms selected from -C(R^{a}R^{b})-, -C(R^{a})=C(R^{a})-, -C(R^{a})=N-, -O-, ⁻Si(R^{a})₂⁻, -S-, -SO₂-, -N(R^{a})-, and >C=Z,
wherein Z is selected from -O-, -S-, and -N(R^{a})-, and R^{a} and R^{b} each is independently selected from hydrogen, optionally substituted C₁₋₁₂-alkyl, optionally substituted C₂₋₁₂-alkenyl, optionally substituted C₂₋₁₂-alkenyl, hydroxy, C₁₋₁₂-alkoxy, C₂₋₁₂-alkenyloxy, carboxy, C₁₋₁₂-alkoxycarbonyl, C₁₋₁₂-alkylcarbonyl, formyl, aryl, aryloxy-carbonyl, aryloxy, arylcarbonyl, heteroaryl, heteroaryloxy-carbonyl, heteroaryloxy, heteroarylcarbonyl, amino, mono- and di(C₁₋₆-alkyl)amino, carbamoyl, mono- and di(C C₁₋₆-alkyl)-aminocarbonyl, amino- C₁₋₆-alkyl-aminocarbonyl, mono- and di(C₁₋₆-alkyl)amino-C₁₋₆-alkyl-aminocarbonyl, C₁₋₆-alkyl-carbonylamino, carbamido, C₁₋₆-alkanoyloxy, sulphono, C₁₋₆-alkylsulphonyloxy, nitro, azido, sulphanyl; C₁₋₆-alkyl-thio, halogen, DNA intercalators, photochemically active groups, thermochemically active groups, chelating groups, reporter groups, and ligands, where aryl and heteroaryl may be optionally substituted, and where two geminal substituents R^{a} and R^{b} together may designate optionally substituted methylene (=CH₂, optionally substituted one or two times with substituents as defined as optional substituents for aryl); and
each of the substituents R^{1*}, R², R³, R^{3*}, R⁵, R^{5*}, R⁶ and R^{6*} which are present and not involved in P or P*, is independently selected from hydrogen, optionally substituted C₁₋₁₂-alkyl, optionally substituted C₂₋₁₂-alkenyl, optionally substituted C₂₋₁₂-alkynyl, hydroxy, C₁₋₁₂-alkoxy, C₂₋₁₂-alkenyloxy, carboxy, C₁₋₁₂-alkoxycarbonyl, C₁₋₁₂-alkylcarbonyl, formyl, aryl, aryloxy-carbonyl, aryloxy, arylcarbonyl, heteroaryl, heteroaryloxy-carbonyl, heteroaryloxy, heteroarylcarbonyl, amino, mono- and di(C₁₋₆-alkyl)amino, carbamoyl, mono- and di(C₁₋₆-alkyl)-amino-carbonyl, amino- C₁₋₆-alkylaminocarbonyl, mono- and di(C₁₋₆-alkyl)-amino- C₁₋₆-alkyl-aminocarbonyl, C₁₋₆-alkyl-carbonylamino, carbamido, C₁₋₆-alkanoyloxy, sulphono, C₁₋₆-alkylsulphonyloxy, nitro, azido, sulphanyl, C₁₋₆-alkylthio, halogen, DNA intercalators, photochemically active groups, thermochemically active groups, chelating groups, reporter groups, and ligands (where the latter groups may include a spacer as defined for the substituent B), where aryl and heteroaryl may be optionally substituted, and where two geminal substituents together may designate oxo, thioxo, imino, or optionally substituted methylene, or together may form a spiro biradical consisting of a 1-5 carbon atom(s) alkylene chain which is optionally interrupted and/or terminated by one or more heteroatoms/groups selected from -0-, -S-, and -(NR^{N})- where R^{N} is selected from hydrogen and C₁₋₄-alkyl, and where two adjacent (non-geminal) substituents may designate an additional bond resulting in a double bond; and R^{N*}_{.} when present and not involved in a biradical, is selected from hydrogen and C₁₋₄-alkyl; and basic salts and acid addition salts thereof.

Throughout the examples herein, the term "LNA" (Locked Nucleoside Analogues) refers to the bi-cyclic nucleoside analogues incorporated in the oligomer. and having a, 2'-0,4'-C methylene beta-D-ribofuranose configuration (US pat No. 6,268,490).
Another LNA variant is the α-L-LNA monomer, which is disclosed in the international patent application, publication No. WO 00/66604. When used herein, α-L-LNA monomers include such compounds having the following general formula II: 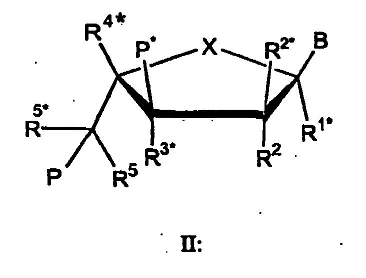 wherein X represent oxygen, sulfur, amino, carbon or substituted carbon, and preferably is oxygen; B is as-disclosed for formula I-above; R^{1*}, R², R^{3*}, R⁵ and R^{5*} are hydrogen; P designates the radical position for an internucleoside linkage to a succeeding monomer, or a 5'-terminal group, P^{*} is an internucleoside linkage to a preceding monomer, or a 3'-terminal group; and R^{2*} and R^{4*} together designate -0-CH₂-, -S-CH₂-, or -NH-CH₂- where the hetero atom is attached in the 2'-position, or a linkage of -(CH₂)ₙ- where n is 2, 3 or 4, preferably 2.

Preferred oligonucleotides in the methods and kits of the invention comprise the locked nucleoside analogues of US pat. No. 6,268,490. Alternative LNA analogues comprise α-L-RNA units such as those disclosed in U.S. application number 60/337,447 filed November 5,2001, including those α-L-RNA units of the following formula III: 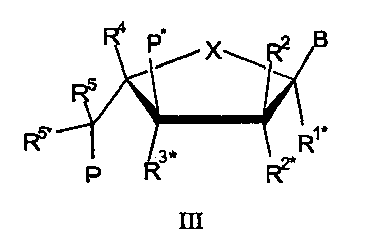 wherein
X is selected from -0-, -S-, -N(R^{N*})-, -C(R⁶R^{6*})-, -O-C(R⁷R^{7*})-, -C(R⁶R^{6*})-O-, -S-C(R⁷R^{7*})-, -C(R⁶R^{6*})-S-, -N(R^{N*})-C(R⁷R^{7*})-, -C(R⁶R^{6*})-N(R^{N*})-, and -C(R⁶R^{6*})-C(R⁷R^{7*})-;
B is selected from hydrogen, hydroxy, optionally substituted C₁₋₄-alkoxy, optionally substituted C₁₋₄-alkyl, optionally substituted C₁₋₄-acyloxy, optionally protected nucleobases, DNA intercalators, photochemically active groups, thermochemically active groups, chelating groups, reporter groups, and ligands;
P designates a radical position for an intemucleoside linkage to a succeeding monomer, or a 5'-terminal group, such internucleoside linkage or 5'-terminal group optionally including the substituent R⁵;
P* designates an internucleoside linkage to a prece-ding monomer, or a 3'-terminal group;
R² represents F, Cl, Br, I, SR", SeH, SeR", N(R^{N}*)₂, OH, a protected hydroxy group, SH, a protected mercapto group, an optionally substituted linear or branched C₁₋₁₂-alkoxy, an optionally substituted linear or branched C₁₋₁₂-alkenyloxy;
each of the substituents R¹*, R²*, R³*, R⁴, R⁵, R⁵*, R⁶, R⁶*, R⁷, and R⁷*, is independently selected from hydrogen, optionally substituted linear or branched C₁-₁₂-alkyl, optionally substituted linear or branched C₁-₁₂-alkenyl, optionally substituted linear or branched C₁₋₁₂-alkynyl, hydroxy, C₁₋₁₂-alkoxy, C₁₋₁₂-alkenyloxy, carboxy, C₁₋₁₂-alkoxycarbonyl, C₁₋₁₂-alkylcarbonyl, formyl, aryl, aryloxy-carbonyl, aryloxy, arylcarbonyl, heteroaryl, heteroaryloxy-carbonyl, heteroaryloxy, hydroxy protection group, hetero-arylcarbonyl, amino, mono- and di(C₁₋₆-alkyl)amino, carba-moyl, mono- and di(C₁₋₆-alkyl)-amino-carbonyl, amino-C₁₋₆-alkyl-amino-carbonyl, mono- and di(C₁₋₆-alkyl)amino-C₁₋₆-alkylamino-carbonyl, C₁₋₆-alkyl-carbonylamino, carbamido, C₁₋₆-alkano-yloxy, sulphono, C₁₋₆-alkylsulphonyloxy, nitro, azido, sulphanyl, C₁₋₆-alkylthio, halogen, DNA intercalators, photochemically active groups, thermochemically active groups, chelating groups, reporter groups, and ligands, where aryl and heteroaryl may be optionally substituted, and where two geminal substituents together may designate oxo, thioxo, imino, or optionally substituted methylene, or together may form a spiro biradical consisting of a 1-5 carbon atom(s) alkylene chain which is optionally interrupted and/or terminated by one or more heteroatoms/groups selected from -O-, -S-, and -(NR^{N})- where R^{N} is selected from hydrogen and C₁₋₄-alkyl, and where two adjacent (non-geminal) substituents may designate an additional bond resulting in a double bond; R", when present, represents a C₁₋₆-alkyl or phenyl group and R^{N}*, when present, is selected from hydrogen and C₁₋₄-alkyl; and basic salts and acid addition salts thereof.

In that α-L-RNA formula, the nucleobase B may be selected from a variety of substituents. In one aspect of the invention it is preferred that B designates a nucleobase selected from uracil-1-yl, thymin-1-yl, adenin-9-yl, guanin-9-yl, cytosin-1-yl, and 5-methylcytosin-1-yl. The above meanings of B represent the natural occurring nucleobases.
The oligonucleotide according to the invention preferably contains at least one α-L-RNA monomer, wherein X is selected from the group consisting of -O-, -S-, and-N(R^{N}*)-. Most preferred is a α-L-RNA monomer, wherein X represent -O-.

In that α-L-RNA formula, the substituents R¹*, R²*, R³*, R⁴, R⁵, and R⁵* may, in a preferred embodiment independently represent hydrogen, C₁₋₄-alkyl or C₁₋₄-alkoxy. In one aspect of the invention R² represents hydrogen. In another aspect, R² represents a hydroxy protection group.

In that α-L-RNA formula, the protected hydroxy group of R² may suitable be a linear or branched C₁₋₆-alkoxyl group or a silyloxy group substituted with one or more linear or branched C₁₋₆-alkyl groups. Notably, the substituent R² is *tert-*butyldimethylsilyloxy.

In that α-L-RNA formula, the substituent P, when representing a 5'-terminal group, suitably designates hydrogen, hydroxy, optionally substituted linear or branched C₁₋₆-alkyl, optionally substituted linear or branched C₁₋₆-alkoxy, optionally substituted linear or branched C₁₋₆-alkylcarbonyloxy, optionally substituted aryloxy, monophosphate, diphosphate, triphosphate, or -W-A', wherein W is selected from -O-, -S-, and N(R^{H})- where R^{H} is selected from hydrogen and C₁₋₆-alkyl, and where A' is selected from DNA intercalators, photochemically active groups, thermochemically active groups, chelating groups, reporter groups, and ligands. Preferably, when a 5'-terminal group, P represent hydroxy or dimethoxytrityloxy.

Further examples of useful synthetic nucleotide monomers for use in olinucleotides, methods and kits of the invention are *Xylo*-LNA as dislcosed in WO 00/56748.

Modified LNA monomers can also be used. For use in oligonucleotides of the invention are 2'-deoxyribonucleotides, ribonucleotides, and analogues thereof that are modified at the 2'-position in the ribose, such as 2'-O-methyl, 2'-fluoro, 2'-trifluoromethyl, 2'-O-(2-methoxyethyl), 2'-O-aminopropyl, 2'-O-dimethylamino-oxyethyl, 2'-O-fluoroethyl or 2'-O-propenyl, and analogues wherein the modification involves both the 2'and 3' position, preferably such analogues wherein the modifications links the 2'- and 3'-position in the ribose, such as those described in Nielsen et al., J. Chem. Soc., Perkin Trans. 1, 1997, 3423-33, and in WO 99/14226, and analogues wherein the modification involves both the 2'- and 4'-position, preferably such analogues wherein the modifications links the 2'- and 4'-position in the ribose, such as analogues having a -CH₂-S- or a -CH₂-NH- or a -CH₂-NMe- bridge (see Singh et al. J. Org. Chem. 1998, 6, 6078-9). Although LNA monomers having the β-D-ribo configuration are often the most applicable, other configurations also are suitable for purposes of the invention. Of particular use are α-L-ribo, the β-D-xylo and the α-L-xylo configurations (see Beier et al., Science, 1999, 283, 699 and Eschenmoser, Science, 1999, 284, 2118), in particular those having a 2'-4' -CH₂-S-, - CH₂-NH-, -CH₂-O- or -CH₂-NMe- bridge.

As discussed above, as used herein, "a poly nucleic acid molecule having a repeating base sequence" refers to a sequence comprising the same nucleobases substantially consecutively. For example the nucleobases are comprised of thymidines, adenosines, uracil, guanine, uracil, purine, xanthine, diaminopurine, 8-oxo-N⁶-methyladenine, 7-deazaxanthine, 7-deazaguanine, N⁴,N⁴-ethanocytosin, N⁶,N⁶-ethano-2,6-diamino-purine, 5-methylcytosine, 5-(C³-C⁶)-alkynylcytosine, 5-fluorouracil, 5-bromouracil, pseudoisocytosine, 2-hydroxy-5-methyl-4-triazolopyridine, isocytosine, isoguanine, inosine and the "non-naturally occurring" nucleobases described in Benner et al., U.S. Pat No. 5,432,272. The term "nucleobase" is intended to cover every and all of these examples as well as analogues and tautomers thereof. Especially interesting nucleobases are adenine, guanine, thymine, cytosine, 5-methylcytosine, and uracil, and the like.

The number of consecutive repeating bases in a consecutive sequence is suitably at least about 4 or 5, and may be up to about 15, 20, 25, 30, 35 or 40 or more repeating bases. More particularly, particularly suitable oligonucleotides may comprise substantially uninterrupted stretches (i.e. same base unit in sequence with no more than 20 percent total number of distinct bases in the sequence) of 4, 5, 6, 7, 8, 9, 10, 11, 12, 13, 14, 15, 16, 17, 18, 19, 20, 21, 22, 23, 24 or 25 or more nucleotides having the same nucleobase.

In the present context, the term "nucleobase" covers naturally occurring nucleobases as well as non-naturally occurring nucleobases. It should be clear to the person skilled in the art that various nucleobases which previously have been considered "non-naturally occurring" have subsequently been found in nature. Thus, "nucleobase" includes not only the known purine and pyrimidine heterocycles, but also heterocyclic analogues and tautomers thereof. Illustrative examples of nucleobases are adenine, guanine, thymine, cytosine, uracil, purine, xanthine, diaminopurine, 8-oxo-N⁶-methyladenine, 7-deazaxanthine, 7-deazaguanine, N⁴,N⁴-ethanocytosine, N⁶,N⁶-ethano-2,6-diaminopurine, 5-methylcytosine, 5-(C³-C⁶)-alkynylcytosine, 5-fluorouracil, 5-bromouracil, pseudoisocytosine, 2-hydroxy-5-methyl-4-triazolopyridine, isocytosine, isoguanine, inosine and the "non-naturally occurring" nucleobases described in Benner et al., U.S. Pat No. 5,432,272. The term "nucleobase" is intended to cover every and all of these examples as well as analogues and tautomers thereof. Especially interesting nucleobases are adenine, guanine, thymine, cytosine, and uracil, which are considered as the naturally occurring nucleobases in relation to therapeutic and diagnostic applications in humans.

When used herein, the term "DNA intercalator" means a group which can intercalate into a DNA or RNA helix, duplex or triplex. Examples of functional parts of DNA intercalators are acridines, anthracene, quinones such as anthraquinone, indole, quinoline, isoquinoline, dihydroquinones, anthracyclines, tetracyclines, methylene blue, anthracyclinone, psoralens, coumarins, ethidium-halides, dynemicin, metal complexes such as 1,10-phenanthroline-copper, tris(4,7-diphenyl-1,10-phenanthroline) ruthenium-cobalt-enediynes such as calcheamicin, porphyrins, distamycin, netropcin, viologen, daunomycin. Especially interesting examples are acridines, quinones such as anthraquinone, methylene blue, psoralens, coumarins, and ethidium-halides.

In the present context, the term "photochemically active groups" covers compounds which are able to undergo chemical reactions upon irradiation with light. Illustrative examples of functional groups hereof are quinones, especially 6-methyl-1,4-naphthoquinone, anthraquinone, naphthoquinone, and 1,4-dimethyl-anthraquinone, diazirines, aromatic azides, benzophenones, psoralens, diazo compounds, and diazirino compounds.

In the present context "thermochemically reactive group" is defined as a functional group which is able to undergo thermochemically induced covalent bond formation with other groups. Illustrative examples of functional parts of thermochemically reactive groups are carboxylic acids, carboxylic acid esters such as activated esters, carboxylic acid halides such as acid fluorides, acid chlorides, acid bromide, and acid iodides, carboxylic acid azides, carboxylic acid hydrazides, sulfonic acids, sulfonic acid esters, sulfonic acid halides, semicarbazides, thiosemicarbazides, aldehydes, ketones, primary alcohols, secondary alcohols, tertiary alcohols, phenols, alkyl halides, thiols, disulphides, primary amines, secondary amines, tertiary amines, hydrazines, epoxides, maleimides, and boronic acid derivatives.

In the present context, the term "chelating group" means a molecule that contains more than one binding site and frequently binds to another molecule, atom or ion through more than one binding site at the same time. Examples of functional parts of chelating groups are iminodiacetic acid, nitrilotriacetic acid, ethylenediamine tetraacetic acid (EDTA), aminophosphonic acid, etc.

In the present context "ligand" means a molecule which binds to another molecule. Ligands can comprise functional groups such as: aromatic groups (such as benzene, pyridine, naphthalene, anthracene, and phenanthrene), heteroaromatic groups (such as thiophene, furan, tetrahydrofuran, pyridine, dioxane, and pyrimidine), carboxylic acids, carboxylic acid esters, carboxylic acid halides, carboxylic acid azides, carboxylic acid hydrazides, sulfonic acids, sulfonic acid esters, sulfonic acid halides, semicarbazides, thiosemicarbazides, aldehydes, ketones, primary alcohols, secondary alcohols, tertiary alcohols, phenols, alkyl halides, thiois, disulphides, primary amines, secondary amines, tertiary amines, hydrazines, epoxides, maleimides, C₁-C₂₀ alkyl groups optionally interrupted or terminated with one or more heteroatoms such as oxygen atoms, nitrogen atoms, and/or sulphur atoms, optionally containing aromatic or mono/polyunsaturated hydrocarbons, polyoxyethylene such as polyethylene glycol, oligo/polyamides such as poly-R-alanine, polyglycine, polylysine, peptides, oligo/polysaccharides, oligo/polyphosphates, toxins, antibiotics, cell poisons, and steroids, and also "affinity ligands", i.e. functional groups or biomolecules that have a specific affinity for sites on particular proteins, antibodies, poly- and oligosaccharides, and other biomolecules.

It will be clear for the person skilled in the art that the above-mentioned specific examples of DNA intercalators, photochemically active groups, thermochemically active groups, chelating groups, reporter groups, and ligands correspond to the "active/functional" part of the groups in question. For the person skilled in the art it is furthermore clear that DNA intercalators, photochemically active groups, thermochemically active groups, chelating groups, reporter groups, and ligands are typically represented in the form M-K- where M is the "active/functional" part of the group in question and where K is a spacer through which the "active/functional" part is attached to the 5- or 6-membered ring. Thus, it should be understood that the group B, in the case where B is selected from DNA intercalators, photochemically active groups, thermochemically active groups, chelating groups, reporter groups, and ligands, has the form M-K-, where M is the "active/functional" part of the DNA intercalator, photochemically active group, thermochemically active group, chelating group, reporter group, and ligand, respectively, and where K is an optional spacer comprising 1-50 atoms, preferably 1-30 atoms, in particular 1-15 atoms, between the 5- or 6-membered ring and the "active/functional" part

In the present context, the term "spacer" means a thermochemically and photochemically non-active distance-making group which is used to join two or more different moieties of the types defined above. Spacers are selected on the basis of a variety of characteristics including their hydrophobicity, hydrophilicity, molecular flexibility and length (e.g. see Hermanson et al., "Immobilized Affinity Ligand Techniques", Academic Press, San Diego, California (1992), p. 137-ff). Generally, the length of the spacers is less than or about 400 angstroms, in some applications preferably less than 100 angstroms. The spacer, thus, comprises a chain of carbon atoms optionally interrupted or terminated with one or more heteroatoms, such as oxygen atoms, nitrogen atoms, and/or sulphur atoms. Thus, the spacer K may comprise one or more amide, ester, amino, ether, and/or thioether functionalities, and optionally aromatic or mono/polyunsaturated hydrocarbons, polyoxyethylene such as polyethylene glycol, oligo/polyamides such as poly-(3-alanine, polyglycine, polylysine, and peptides in general, oligosaccharides, oligo/polyphosphates. Moreover the spacer may consist of combined units thereof. The length of the spacer may vary, taking into consideration the desired or necessary positioning and spatial orientation of the "active/functional" part of the group in question in relation to the 5-or 6-membered ring. In particularly interesting embodiments, the spacer includes a chemically cleavable group. Examples of such chemically cleavable groups include disulphide groups cleavable under reductive conditions, peptide fragments cleavable by peptidases, etc.

In one variant, K designates a single bond so that the "active/functional" part of the group in question is attached directly to the 5- or 6-membered ring.

In a preferred embodiment, the substituent B in the general formulae I and II is preferably selected from nucleobases, in particular from adenine, guanine, thymine, cytosine and uracil.

In the oligomers (formula 1), P designates the radical position for an internucleoside linkage to a succeeding monomer, or a 5'-terminal group. The first possibility applies when the LNA in question is not the 5'-terminal "monomer", whereas the latter possibility applies when the LNA in question is the 5'-terminal "monomer". It should be understood (which will also be clear from the definition of internucleoside linkage and 5'-terminal group further below) that such an internucleoside linkage or 5'-terminal group may include the substituent R⁵ (or equally applicable: the substituent R5) thereby forming a double bond to the group P. (5'-Terminal refers to the position corresponding to the 5' carbon atom of a ribose moiety in a nucleoside.)

On the other hand, an intemucleoside linkage to a preceding monomer or a 3'-terminal group (P) may originate from the positions defined by one of the substituents R³ or R3, preferably from the positions defined by the substituents R³. (3'-Terminal refers to the position corresponding to the 3' carbon atom of a ribose moiety in a nucleoside.)

It should be understood that the orientation of the group P* either as R3 ("normal" configuration) or as R³ (xylo configuration) represents two equally interesting possibilities. It has been found that all-"normal" (R3 = P*) oligomers and oligomers with combinations of "normal" LNA monomers and nucleotides (2-deoxynucleotides and/or nucleotides) hybridize strongly (with increasing affinity) to DNA, RNA and other LNA oligomers. It is presently believed that combination of all-xylo LNA oligomers and oligomers with xylo LNA (R³ = P*) monomers and, e.g., xylo nucleotides (nucleotides and/or 2-deoxynucleotides) will give rise to comparable hybridization properties. It has been shown that an oligomer with "normal" configuration (R3 = P*) will give rise to an anti-parallel orientation of an LNA oligomer when hybridized (with increasing affinity) to either DNA, RNA or another LNA oligomer. It is thus contemplated that an oligomer with xylo configuration (R³ = P*) will give rise to a parallel orientation when hybridized to DNA, RNA or another LNA.

In view of the above, it is contemplated that the combination of "normal" LNAs and xylo-LNAs in one oligomer can give rise to interesting properties as long as these monomers of different type are located in domains, i.e. so that an uninterrupted domain of at least 5, such as at least 10, monomers (e.g. xylo-LNA, xylo-nucleotides, etc. monomers) is followed by an uninterrupted domain of at least 5, e.g. at least 10, monomers of the other type (e.g. "normal" LNA, "normal" nucleotides, etc.), etc. Such chimeric type oligomers may, e.g., be used to capture nucleic acids.

In the present context, the term "monomer" relates to naturally occurring nucleosides, non-naturally occurring nucleosides, PNAs, etc. as well as LNAs. Thus, the term "succeeding monomer" relates to the neighbouring monomer in the 5'-terminal direction and the "preceding monomer" relates to the neighbouring monomer in the 3'-terminal direction. Such succeeding and preceding monomers, seen from the position of an LNA monomer, may be naturally occurring nucleosides or non-naturally occurring nucleosides, or even further LNA monomers.

Consequently, in the present context (as can be derived from the definitions above), the term "oligomer" means an oligonucleotide modified by the incorporation of one or more LNAs).

In the present context, the orientation of the biradical (R²-R⁴) is so that the left-hand side represents the substituent with the lowest number and the right-hand side represents the substituent with the highest number, thus, when R2' and R4. together designate a biradical "-O-CH₂", it is understood that the oxygen atom represents R² , thus the oxygen atom is e.g. attached to the position of R², and the methylene group represents R".

Considering the numerous interesting possibilities for the structure of the biradical (R²'-R⁴') in LNA(s) incorporated in oligomers, it is believed that the biradical is preferably selected from -(CR'R')ᵣ Y-(CR'R')ₛ, -(CR'R')ᵣ Y-(CR'R')s-Y-,-Y-(CR'R'), Y-, -Y-(CR'R')r Y(CR'R')ₛ -(CR'R')r+s, -Y-, -Y-Y-, wherein each Y is independently selected from -O-, -S-, -Si(R)₂-, -N(R')-, >C=O, -C(=O)-N(R')-, and - N(R')-C(=O)-, wherein each R' is independently selected from hydrogen, halogen, azido, cyano, nitro, hydroxy, mercapto, amino, mono- or di(C₁₋₆-alkyl)amino, optionally substituted C₁₋₆-alkoxy, optionally substituted C₁₋₆-alkyl, DNA intercalators, photochemically active groups, thermochemically active groups, chelating groups, reporter groups, and ligands, and/or two adjacent (non-geminal) R' may together designate a double bond; and each of r and s is 0-4 with the proviso that the sum r+s is 1-4. Particularly interesting situations are those wherein the biradical is selected from -Y-, -(CR'R)ᵣ₊ₛ, -(CR'R'), Y-(CR'R')ₛ, and -Y-(CR'R)ᵣ₊ₛ Y-, wherein and each of r and s is 0-3 with the proviso that the sum r+s is 1-4.

Particularly interesting oligomers are those wherein R²' and R4' in at least one LNA in the oligomer together designate a biradical selected from -0-, -S-, -N(R')-,-(CR'R')r+s-, -(CR'R')ᵣ O-(CR'R')s-, -(CR*R*)r S-(CR'R')s-, (CR*R*), N(R)-(CR'R')s-, -O-(CR.R,)r+s-O-, -S-(CR'R')s O-, -O-(CR'R)ᵣ₊ₛ S-, -N(R')-(CR'R')ₛ₊ᵣO-, - O-(CR'R')ᵣ₊ₛ-N(R')-, -S-(CR'R')ᵣ₊ₛ S-, -N(R)-(CRR')ᵣ₊ₛ-N(R-N(R')-(CR'R')ᵣ₊ₛ S-, and - S-(CR'R)ᵣ₊ₛ N(R')-.

It is furthermore preferred that one R' is selected from hydrogen, hydroxy, optionally substituted C₁₋₆-alkoxy, optionally substituted C₁₋₆-alkyl, DNA intercalators, photochemically active groups, thermochemically active groups, chelating groups, reporter groups, and ligands, and any remaining substituents R' are hydrogen.

In one preferred variant, one group R in the biradical of at least one LNA is selected from DNA intercalators, photochemically active groups, thermochemically active groups, chelating groups, reporter groups, and ligands (where the latter groups may include a spacer as defined for the substituent B).

Preferably, each of the substituents R'*, R², R³, R^{3'}, R⁵, R^{s}', R⁶ and R⁶' of the LNA(s), which are present and not involved in P or P', is independently selected from hydrogen, optionally substituted C₁₋₆-alkyl, optionally substituted C₂₋₆-alkenyl, hydroxy, C₁₋₆-alkoxy, C₂₋₆-alkenyloxy, carbony, C₁₋₆-alkoxycarbonyl, C₁₋₆-alkylcarbonyl, formyl, amino, mono and di(C₁₋₆-alkyl)amino, carbamoyl, mono- and di(C₁₋₆-alkyl)-amino-carbonyl, C₁₋₆-alkylcarbonylamino, carbamido, azido, C₁₋₆-alkanoyloxy, sulphono, sulphanyl, C₁₋₆-alkylthio, DNA intercalators, photochemically active groups, thermochemically active groups, chelating groups, reporter groups, and ligands, and halogen, where two geminal substituents together may designate oxo, and where R", when present and not involved in a biradical, is selected from hydrogen and C₁₋₄-alkyl.

In a preferred variant of the LNAs, X is selected from -0-, -S-, and -NR"'-, in particular -O-, and each of the substituents R", R², R³, R³, R⁵, R^{5*}, R⁶ and R⁶' of the LNA(s), which are present and not involved in P or P*, designates hydrogen.

In an even more preferred variant, X is O, R2 is selected from hydrogen, hydroxy, and optionally substituted C₁₋₆-alkoxy, one of R³ and R3 is P* and the other is hydrogen, and R"', R⁵, and R5, designate hydrogen, and, more specifically, the biradical (R²-R⁴) is selected from -O-, (CH₂)O-,-O-(CH₂)₁₋₃-, -(CH₂)O-1-S-(CH₂)₁₋₃,-(CH₂)O-,-N(R")-(CH₂)₁₋₃, and -(CH₂)₂₋₄, in particular from -O-CH₂-, -S-CH₂-, and - NR "-CH₂-. Generally, with due regard to the results obtained so far, it is preferred that the biradical constituting R2 and R4, forms a two carbon atom bridge, i.e. the biradical forms a five membered ring with the furanose ring (X=O). Particularly interesting are also those oligomers where R²* and R4. of an incorporated LNA of formula I together designate a biradical selected from -O-CH₂-, -S-CH₂-, and -NR"-CH₂-; X is O, B designates a nucleobase selected from adenine, guanine, thymine, cytosine and uracil; R² is hydrogen, one of R³ or R3 designates P* and the other is hydrogen, and R'*, R³, R⁵, and R5 designate hydrogen.

In these embodiments, it is furthermore preferred that at least one LNA incorporated in an oligomer includes a nucleobase (substituent B) selected from adenine and guanine. In particular, it is preferred that an oligomer having LNA incorporated therein includes at least one nucleobase selected from thymine, uracil and cytosine and at least one nucleobase selected from adenine and guanine. For LNA monomers, it is especially preferred that the nucleobase is selected from adenine and guanine.

Within a variant of these interesting embodiments, all monomers of a oligonucleotide are LNA monomers.

As it will be evident from the general formula I (LNA(s) in an oligomer) and the definitions associated therewith, there may be one or several asymmetric carbon atoms present in the oligomers depending on the nature of the substituents and possible biradicals, cf. below.

In one variant, R³* designates P'. In another variant, R³ designates P*, and in a third variant, some R3' designates P* in some LNAs and R³ designates P* in other LNAs within an oligomer.

The oligomers typically comprise 1-100 LNAs of the general formula I and 0-100 nucleosides selected from naturally occurring nucleosides and nucleoside analogues. The sum of the number of nucleosides and the number of LNAs) is at least 2, preferably at least 3, in particular at least 5, especially at least 7, such as in the range of 2-100, preferably in the range of 2-100, such as 3-100, in particular in the range of 2-50, such as 3-50 or 5-50 or 7-50.

In the present context, the term "nucleoside" means a glycoside of a heterocyclic base. The term "nucleoside" is used broadly as to include non-naturally occurring nucleosides, naturally occurring nucleosides as well as other nucleoside analogues. Illustrative examples of nucleosides are ribonucleosides comprising a ribose moiety as well as deoxyribo-nucleosides comprising a deoxyribose moiety. With respect to the bases of such nucleosides, it should be understood that this may be any of the naturally occurring bases, e.g. adenine, guanine, cytosine, thymine, and uracil, as well as any modified variants thereof or any possible unnatural bases.

When considering the definitions and the known nucleosides (naturally occurring and non-naturally occurring) and nucleoside analogues (including known bi- and tricyclic analogues), it is clear that an oligomer may comprise one or more LNAs) (which may be identical or different both with respect to the selection of substituent and with respect to selection of biradical) and one or more nucleosides and/or nucleoside analogues. In the present context "oligonucleotide" means a successive chain of nucleosides connected via internucleoside linkages; however, it should be understood that a nucleobase in one or more nucleotide units (monomers) in an oligomer (oligonucleotide) may have been modified with a substituent B as defined above.

As mentioned above, the LNA(s) of an oligomer is/are connected with other monomers via an intemucleoside linkage. In the present context, the term "internucleoside linkage" means a linkage consisting of 2 to 4, preferably 3, groups/atoms selected from -CH₂-, -O-, -S-, -NR"-, >C=O, >C=NR", >C=S, -Si(R")₂-,-SO-,-S(O)₂-, -P(0)2⁻, -PO(BH₃)-, -P(O,S)-, -P(S)₂-, -PO(R")-, -PO(OCH₃)-, and -PO(NHR")-, where R" is selected form hydrogen and C₁₋₄-alkyl, and R" is selected from C₁₋₆-alkyl and phenyl. Illustrative examples of such internucleoside linkages are -CH_{Z}-CH_{Z}-CH_{Z}-, -CH_{Z}-CO-CH_{Z}-, -CH_{Z}-CHOH-CH_{Z}-, -O-CH_{Z}-O-, -O-CH_{Z}CH_{Z}-, -O-CH_{Z}-CH= (including R⁵ when used as a linkage to a succeeding monomer), -CH_{Z}-CH_{Z}-O-, -NR"-CH_{Z}-CH_{Z}-, -CH_{Z}-CH_{Z}-NR"-, -CH_{Z}NR"-CH_{Z}-, -O-CH_{Z}-CH_{Z}-NR"-, -NR"-CO-O-, -NR "-CO-NR"-, -NR "-CS-NR"-, -NR "-C(=NR")-NR"-, -NR "-CO-CHZ-NR"-, -O-CO-O-, -O-CO-CH_{Z}O-, -O-CH_{Z}-CO-O-, -CH_{Z}-CO-NR"-, -O-CO-NR H-, -NR H-CO-CH₂-, -O-CH_{Z}-CO-NR"-, -O-CH_{Z}-CH_{Z}-NR"-, -CH=N-O-, -CHZ-NR"-O, -CH_{Z}-O-N= (including R⁵ when used as a linkage to a succeeding monomer), -CHZ-O-NR"-, -CO-NR "-CH_{Z}, -CH_{Z}NR"-O-, - CH_{z}-NR"-CO-, -O-NR "-CH_{Z}, -O-NR H-, -O-CH_{Z}-S-, -S-CH_{Z}-O-, -CH_{Z}-CH_{Z}-S-, -O-CH₂-CH_{Z}S-, -S-CH_{Z}-CH= (including R⁵ when used as a linkage to a succeeding monomer), -S-CH₂-CH₂-, -S-CH₂-CH₂-O-, -S-CH₂-CH₂-S-, -CH₂-S-CH₂-, -CH₂-SO-CH₂-, - CH₂SO₂CH₂-, -O-SO-O-, -O-S(O)₂-O-, -O-S(O₂)-CH₂-, -O-S(O)₂-NR H-, -NRH-S(O)₂⁻CH₂⁻, -O-S(O)₂-CH2⁻, -O-P(O))₂-O-, -O-P(O,S)-O-, -O-P(S)₂-O-, -S-P(O)₂-O-, -S-P(O,S)-O-, -SP(S)₂-O-, -O-P(O)₂-S-, -O-P(O,S)-S-, -O-P(S)₂-S-, -S-P(O)2-S-, -S-P(O,S) S-, -S-P(S)2-S-, -O-PO(R")-O-, -O-PO(OCH₃)-O-, -O-PO(OCH₂CH₃)-O-, -O-PO(OCH₂CH₂S-R)-O-, -O-PO(BH3)-0-, -O-PO(NHR")-O-, -O-P(O)₂-NR H-, NR"-P(O)2-O-, -O-P(O,NR")-O-, -CH2P(O)₂-0-, -O-P(O)₂-CH₂-, and -O-Si(R")₂-O-; among which -CH₂-CO-NR"-, -CH₂-NR"-O-, -S-CH₂-O-, -O-P(O)₂-0-, -O-P(O,S)-0-, -O-P(S)₂-0-, -NR "-P(O)₂-O-, -O-P(O,NR")-O-, -O-PO(R")-O-, -O-PO(CH₃)-0-, and -0-PO(NHR")-O-, where R" is selected from hydrogen and C₁₋₄-alkyl, and R" is selected from C₁₋₆-alkyl and phenyl, are especially preferred. Further illustrative examples are given in Mesmaeker et al., Current Opinion in Structural Biology 1995, 5, 343-355. The left-hand side of the internucleoside linkage is bound to the 5-membered ring as substituent P', whereas the right-hand side is bound to the 5'-position of a preceding monomer.

It is also clear from the above that the group P may also designate a 5'-terminal group in the case where the LNA in question is the 5'-terminal monomer. Examples of such 5'-terminal groups are hydrogen, hydroxy, optionally substituted Cₗ₋ᵣ,-alkyl, optionally substituted C₁₋₆-alkoxy, optionally substituted C₁₋₆-alkylearbonyloxy, optionally substituted aryloxy, monophosphate, diphosphate, triphosphate, and -W-A', wherein W is selected from -O-, -S-, and -N(R")- where R" is selected from hydrogen and C₁₋₆₋alkyl, and where A' is selected from DNA intercalators, photochemically active groups, thermochemically active groups, chelating groups, reporter groups, and ligands (where the latter groups may include a spacer as defined for the substituent B).

In the present description and claims, the terms "monophosphate", "diphosphate", and "triphosphate" mean groups of the formula: -O-P(O)₂-O", -OP(O)₂-O-P(O)₂-O-, and -O-P(O)₂-O-P(O)₂-O-P(O)₂-O-, respectively.

In a particularly interesting embodiment, the group P designates a 5'-terminal group selected from monophosphate, diphosphate and triphosphate. Especially the triphosphate variant is interesting as a substrate for nucleic acid polymerases.

Analogously, the group P" may designate a 3'-terminal group in the case where the LNA in question is the 3'-terminal monomer. Examples of such 3'-terminal groups are hydrogen, hydroxy, optionally substituted C₁₋₆-alkoxy, optionally substituted C₁₋₆-alkylcarbonyloxy, optionally substituted aryloxy, and -W-A', wherein W is selected from -O-, -S-, and -N(R")- where R" is selected from hydrogen and C₁₋₆-alkyl, and where A' is selected from DNA intercalators, photochemically active groups, thermochemically active groups, chelating groups, reporter groups, and ligands (where the latter groups may include a spacer as defined for the substituent B).

Within this variant, as well as generally, the LNAs preferably include different nucleobases, in particular both nucleobases selected from thymine, cytosine and uracil and nucleobases selected from adenine and guanine.

The oligomers are also intended to cover chimeric oligomers. The term "chimeric oligomers" means two or more oligomers with monomers of different origin joined either directly or via a spacer. Illustrative examples of such oligomers which can be combined are peptides, PNA-oligomers, oligomers containing LNAs, and oligonucleotide oligomers. The combination of an oligomer having xylo-LNA (R³ = P*) domain(s) and "normal" LNA (R3 = P*) domain(s) might be constructed as an example of a chimeric oligomer as the various domains may have different affinity and specificity profiles.

Generally, the oligomers have surprisingly good hybridization properties with respect to affinity and specificity. Thus, the oligomers comprise at least one nucleoside analogue which imparts to the oligomer a Tₘ with a complementary DNA oligonucleotide which is at least 2.5°C higher, preferably at least 3.5°C higher, in particular at least 4.0°C higher, especially at least 5.0°C higher, than that of the corresponding unmodified reference oligonucleotide which does not comprise any nucleoside analogue. In particular, the Tₘ of the oligomer is at least 2.5 x N°C higher, preferably at least 3.5 x N°C higher, in particular at least 4.0 x N°C higher, especially at least 5.0 x N °C higher, where N is the number of nucleoside analogues.

In the case of hybridization with a complementary RNA oligonucleotide, at least one nucleoside analogue imparts to the oligomer a Tₘ with the complementary DNA oligonucleotide which is at least 4.0°C higher, preferably at least 5.0°C higher, in particular at least 6.0°C higher, especially at least 7.0°C higher, than that of the corresponding unmodified reference oligonucleotide which does not comprise any nucleoside analogue. In particular, the Tₘ of the oligomer is at least 4.0 x N°C higher, preferably at least 5.0 x N°C higher, in particular at least 6.0 x N°C higher, especially at least 7.0 x N°C higher, where N is the number of nucleoside analogues.

The term "corresponding unmodified reference oligonucleotide" is intended to mean an oligonucleotide solely consisting of naturally occurring nucleotides which represents the same nucleobases in the same absolute order (and the same orientation).

The Tₘ is measured under one of the following conditions:
a) 10mM Na₂HP0₄, pH 7.0, 100mM NaCl, 0.1 mM EDTA;
b) 10mM Na₂HP0₄ pH 7.0, 0.1 mM EDTA; or
c) 3M tetramethylammoniumchloride (TMAC), 10mM Na₂HP0₄, pH 7.0, 0.1 mM EDTA;
   preferably under conditions a), at equimolar amounts (typically 1.0 µM) of the oligomer and the complementary DNA oligonucleotide.

The oligomer is preferably as defined above, where the at least one nucleoside analogue has the formula I where B is a nucleobase. Especially interesting are the cases where at least one nucleoside analogue includes a nucleobase selected from adenine and guanine. Furthermore, with respect to specificity and affinity, the oligomer, when hybridized with a partially complementary DNA oligonucleotide, or a partially complementary RNA oligonucleotide, having one or more mismatches with said oligomer, should exhibit a reduction in Tₘ, as a result of said mismatches, which is equal to or greater than the reduction which would be observed with the corresponding unmodified reference oligonucleotide which does not comprise any nucleoside analogues. Also, the oligomer should have substantially the same sensitivity of Tm to the ionic strength of the hybridization buffer as that of the corresponding unmodified reference oligonucleotide.

Oligomers defined herein are typically at least 1 % modified, such as at least 2% modified, e.g. 3% modified, 4% modified, 5% modified, 6% modified, 7% modified, 8% modified, or 9% modified, at least 10% modified, such as at least 11 % modified, e.g.,12% modified, 13% modified, 14% modified, or 15% modified, at least 20% modified, such as at least 30% modified, at least 50% modified, e.g. 70% modified, and in some interesting applications 100% modified.

The oligomers preferably have substantially higher 3'-exonucleolytic stability than the corresponding unmodified reference oligonucleotide.

It should be understood that oligomers (wherein LNAs are incorporated) and LNAs as such include possible salts thereof, of which pharmaceutically acceptable salts are especially relevant. Salts include acid addition salts and basic salts. Examples of acid addition salts are hydrochloride salts, sodium salts, calcium salts, potassium salts, etc. Example of basic salts are salts where the (remaining) counter ion is selected from alkali metals, such as sodium and potassium, alkaline earth metals, such as calcium, and ammonium ions (⁺N(R%R^{b}, where each of R⁹ and R" independently designates optionally substitute(C₁₋₆-alkyl optionally substituted C₂₋₆-alkenyl, optionally substituted aryl, or optionally substituted heteroaryl). Pharmaceutically acceptable salts are, e.g., those described in Remington's Pharmaceutical Sciences, 17. Ed. Alfonso R. Gennaro (Ed.), Mack Publishing Company, Easton, PA, U.S.A., 1985 and more recent editions and in Encyclopedia of Pharmaceutical Technology. Thus, the term "an acid addition salt or a basic salt thereof used herein is intended to comprise such salts. Furthermore, the oligomers and LNAs as well as any intermediates or starting materials therefor may also be present in hydrate form.

The following non-limiting examples are illustrative of the invention.

### Examples

The invention will now be further illustrated with reference to the following examples. It will be appreciated that what follows is by way of example only and that modifications to detail may be made while still falling within the scope of the invention.

### Example 1: Recovery assay of in vitro mRNA captured by oligo-T capture probes.

LNA oligo-T capture probes were used to investigate the efficiency of poly(A)⁺RNA selection. Biotinylated oligo-T capture probes attached to streptavidin-coated magnetic particles captured a defined amount of *in vitro* synthesized polyadenylated mRNAs from the yeast *Saccharomyces cerevisiae* under various hybridization conditions. After several stringency washes the selected mRNA was eluted from the beads. The recovery per cents were calculated from gel electrophoresed fragments.

### Experimental

Pre-blocking of Streptavidin-coated magnetic particles. 60 µL of Streptavidin-coated magnetic particles (Roche Cat no. 1 641 778 or 1 641 786) were pipetted into an Eppendorf tube for each sample. The magnetic separator was used to remove the supernatant 100 µL 1µg/mL yeast RNA (Ambion cat. no. 7120G) diluted in TE (10 mM Tris-HCI, 1 mM EDTA, pH 7.5) was added to pre-block the magnetic particles for 5 min at room temperature. The particles were washed in 100 µL TE.

The prepared by mixing 1µg *in vitro* mRNA (Yeast *SSA4* or *ACT1*) into an Eppendorf tube and 200 µL GuSCN buffer (4M GuSCN (Sigma), 25 mM Na-citrate (JT Baker), pH 7.0, 0.5 % sodium N-lauroyl sarcosinate (Sigma)) or 200 µL high NaCl-salt buffer 1 (20 mM Tris-HCl (pH 7, Ambion), 0.5 M NaCl, 1 mM EDTA (pH 8.0, Ambion) 0.1%(w/v) lauryl sarcosinate (Sigma)) was added and the samples were vortexed briefly. The biotinylated oligo-T capture probes (Table 1 and 2) were added to the sample preparation and transferred to the washed particles. The hybridization of the *in vitro* mRNA to the oligo-T capture probes and the streptavidin biotin complex to form was allowed 5 min at ambient temperatures (room temperature, 37°C, 55°C, 60°C or 65°C) shaking the particles at 700 rpm in an Eppendorf Thermomixer (Radiometer Denmark). The particles were collected using the PickPen from Bionobile (Bionobile, Finland) and a short washing step in 100µL GuSCN buffer was performed. Quickly recollected the particles were released into 100 µL washing buffer 1 (20 mM Tris-HCl (pH 7, Ambion), 0.5 M NaCl, 1 mM EDTA (pH 8.0, Ambion) 0.1%(w/v) lauryl sarcosinate (Sigma)), (Sambrook 2. ed.). This step was repeated. The particles were washed in 100 µL washing buffer 2 (20 mM Tris-HCl (pH 7, Ambion), 0.25 M NaCl (Ambion), 1 mM EDTA (pH 8.0, Ambion) 0.1 %(w/v) lauryl sarcosinate (Sigma)) and in 100 µL washing buffer 3 (20 mM Tris-HCl (pH 7, Ambion), 0.1 M NaCl (Ambion), 1 mM EDTA (pH 8.0 Ambion) 0.1%(w/v) lauryl sarcosinate (Sigma)). Finally the particles were transferred to an Eppendorf tube containing 50 µL DEPC-H₂O (Ambion Cat. no. 9924). The sample was incubated for 5 min at 95°C and quenched on ice for 5 min to release the *in vitro* mRNA from the particles. The particles were recollected two times and the supernatant was span briefly (13.2 rpm 60 sec) and transferred to a clean siliconezed eppendorf tube (Ambion). The mRNA was ethanol precipitated by adding 1/10 volume 3 M NaOAc (Ambion), 150 µg/mL Glycogen Carrier (Ambion) and 2.5 volume. 96% Ethanol to the tube and freezed at -20°C over night. After spinning at least 30 min 16400xg at 4°C the supernatant was removed and the pellet washed with ice-cold 70% EtOH and air-dried. The pellet was dissolved in 10 µL DEPC treated H₂O.

For analysis 3 µL of the sample and a standard dilution curve of either *SSA4* or *ACT1* were applied on a native 1 % agarose gel in 1x TAE buffer containing 1:10000 Gelstar. The gel was electrophoresesed for 20-30 min, 7 V/cm and the quantified on the Typhoon 9200 Imager (Amersham Pharmacia Biotech).

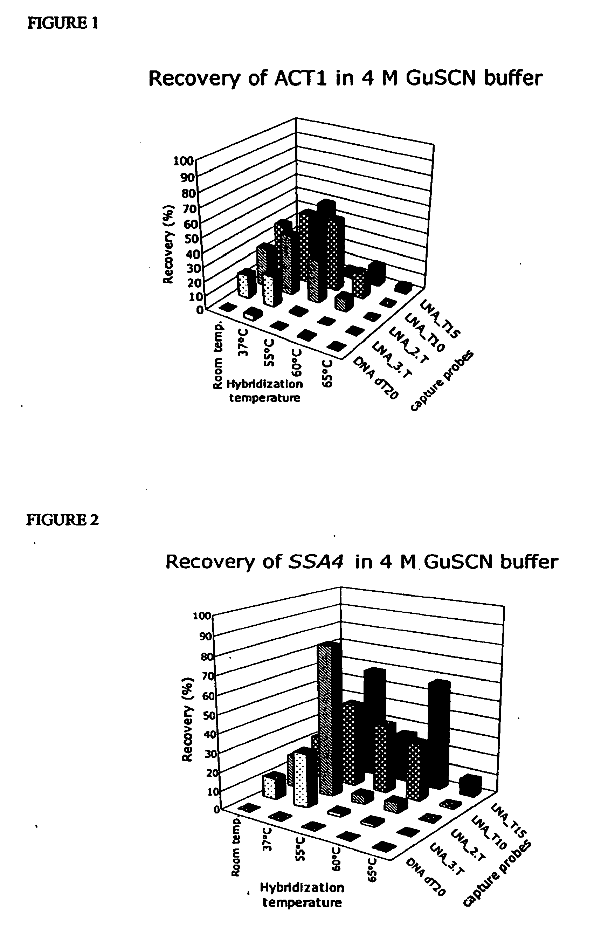

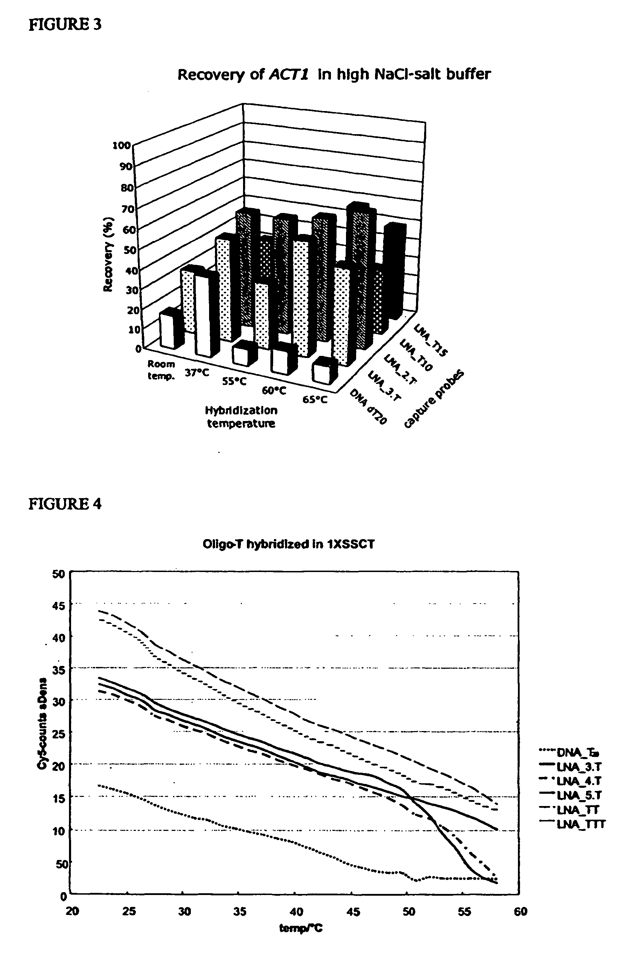

The evaluation of the oligo-T capture probes spiked with various amounts of LNA shows that LNA oligonucleotides bind to complementary DNA or RNA with affinities significantly higher than the corresponding DNA oligonucleotide. The DNA_dT₂₀ capture probe recover ca. 40% *in vitro* mRNA in high NaCl-salt buffer (washing buffer#1) at 37°C. Room temperature or elevated temperatures lower the amount of recovered mRNA. Using the oligo-T capture probes result in higher yield in the NaCl-salt buffer (Figure 3) and up to 20-fold increased mRNA yields compared to the DNA control in a guanidinium containing buffer (Figures 1 and 2). The GuSCN buffer inhibits the RNAse activity. The LNA-enhanced poly(A)⁺ RNA selection works efficiently even at elevated temperatures which can be an advantage for unfolding secondary structures in the RNA.

**Table 1**

| Comp. No. | Oligo Name: | Sequence 5'-: |
|---|---|---|
| 1 | DNA_dT₂₀ | 5'-biotin-tttttttttttttttttttt |
| 2 | LNA_2.T | 5'-biotin-TtTtTtTtTtTtTtTtTtTt |
| 3 | LNA_3.T | 5'-biotin-TttTttTttTttTttTttTt |
| 4 | LNA_T₁₀ | 5'-biotin-TTTTTTTTT |
| 5 | LNA_T₁₅ | 5'-biotin-TTTTTTTTTTTT |

| | | |
|---|---|---|
| Note: LNA nucleotides are indicated with uppercase letters, DNA nucleotides are indicated by lowercase letters. C^{rnet} indicates 5-methyl cytosine LNA; 5'-biotin indicates 5' biotin-(CH₂)₄-CONH-CH₂)₆-. | | |

### Example 2: Use of LNA oligo-T probes to improve purification of mRNA

LNA oligonucleotide as oligo-T capture probes to improve the purification of polyadenylated RNA. Melting experiments were performed in solution and "on-chip" (the oligo-T capture probes bound to a solid surface). The biotinylated oligo-T capture probes were attached to streptavidin-coated magnetic particles and used for purification of poly(A)⁺RNA.

Melting experiments in solution. The melting of the duplexes either LNA/DNA or DNA/DNA (control) were studied measuring absorbance (λ=260) as a function of temperature from 10°C to 90°C with an increase of 1°C/min in a Perkin-Elmer λ-40 spectrophotometer equipped with a Peltier element controlling the temperature. Hybridization mixtures of 500 µL were prepared in 10 mM sodium phosphate buffer pH 7.0 100 mM NaCl, 0.1 mM EDTA containing equimolar (1 µM) amounts of the different LNA or DNA oligonucleotides and the complementary DNA oligo-dA₂₁ or RNA oligo-rA₂₀. All melting curves were monophasic and sigmoid and the melting temperature (Tₘ) was obtained as the maximum of the first derivative *(d(A260)*/*dT)* of the melting curve (A260 vs. temperature). All LNA oligonucleotides obtained higher Tₘ values compared to the control DNA (see table 2). The higher number of LNA nucleotides in the oligonucleotide the higher ΔTₘ.

**Table 2**

| Comp . No: | Oligo Name: | Sequence 5'-: | Tₘ/°C (DNA) | ΔTₘ°C (DNA) | Tₘ/°C (RNA) | ΔTₘ/°C (RNA) |
|---|---|---|---|---|---|---|
| 1 | DNA_T₂₀ | 5'-biotin-tttttttttttttttttttttttttttt | 43.7 | - | 40.3 | - |
| 3 | LNA_3.T | 5'-biotin-TttTttTttTttTttTttTt | 58.4 | 14.7 | 60.8 | 20.5 |
| 6 | LNA_4.T | 5'-biotin-ttTtttTtttTtttTtttTt | 51.0 | 7.3 | 56.9 | 16.6 |
| 7 | LNA_5.T | 5'-biotin-tttTttttTttttTttttTt | 47.8 | 4.1 | 52.0 | 11.7 |
| 4 | LNA_T₁₀ | 5'-biotin-TTTTTTTTTT | 83.6 | 39.3 | 76.3 | 36.0 |
| 5 | LNA_T₁₅ | 5'-biotin- TTTTTTTTTTTTTTT | >95 | >51.3 | 94.6 | 54.3 |
| 8 | LNA_T₂₀ | 5'-biotin-TTTTTTTTTTTTTTTTTTTT | >95 | >51.3 | >95 | >54.7 |
| 9 | LNA_ TT | 5'-biotin-ttTTtttTTtttTTtttTTt | 59.9 | 16.2 | 63.2 | 22.9 |
| 10 | LNA_TT T | 5'-biotin-ttTTTttttTTTttUTTTt | 66.3 | 22.6 | 65.2 | 24.9 |

| | | | | | | |
|---|---|---|---|---|---|---|
| Note: LNA nucleotides are indicated with uppercase letters, DNA nucleotides are indicated by lowercase letters. C^{met} indicates 5-methyl cytosine LNA; 5'-biotin indicates 5' biotin-(CH₂)₄-CONH-(CH₂)₆-. | | | | | | |

### Example 3: Melting experiments "on-chip"

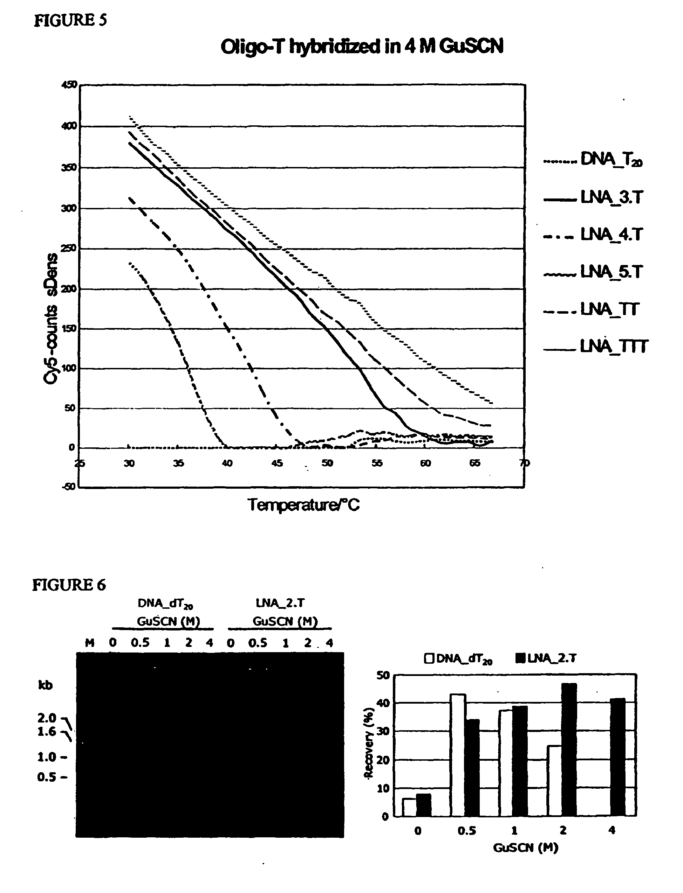

Capture probe melting profiles have been performed with a microscope equipped with a peltier-controlled heating stage it has been shown possible to investigate fluorescent signals from micro arrays during specific changes in temperature. Melting properties of different surface attached probes and their targets can this way be revealed ( figure 5).

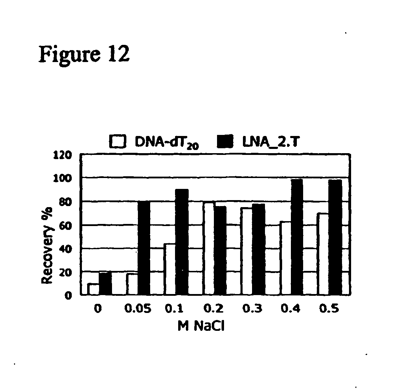

Euray^{™} polymer slides were coated with 20 µg/mL streptavidin, Prozyme, (cat. no. PZSA20) in phosphate saline buffer (PBS, pH 7, 0.15 M Na⁺) for 22 hours at 4°C in a humidity chamber. The slides were washed three times in PBS and briefly in demineralized water and dried for 5 min. The slides were spotted using 10 µM of LNA or DNA oligonucleotides (table 1, table 2). The array setup: biotinylated oligonucleotides were spotted in duplicate and three times 300 pL per spot with a distance of 300 µm between spots. he slides were incubated O/N at 4°C in a humidity chamber to allow binding of biotin to the streptavidin. The slides were hybridized with 0.1 µM Cy5-oligo-dT₂₀ in either 1 x SSCT (150 mM NaCl, 15 mM Na-citrate, pH 7.0, 0.1 % Tween 20) or GuSCN buffer (4 M GuSCN, 100 mM sodium phosphate buffer pH 7.0, 0.2 mM EDTA) for 2 hours at room temperature. The slides were washed in the same buffer used for hybridization. The slides were mounted with degassed hybridization buffer using a glass coverslip and nail polish for sealing and data was collected. Results show that signals from the LNA oligonucleotides are higher than the signal from the control DNA oligonucleotide when the hybridization is performed in 1 x SSCT buffer (Figure 12). However, when the hybridization is performed in the GuSCN no signal is obtained from the DNA control (figure 5). Surprisingly LNA oligonucleotides perform as well in the SSCT buffer as in the GuSCN (Figures 1, 2, 3).

### Example 4: The effect of Guanidinium Thiocyanate (GuSCN) concentration on poly(A)+RNA selection

The present method describes the hybridization efficiency of poly(A)⁺RNA selection in various concentration of guanidinium thiocynate (GuSCN) (see figure 6). Biotinylated oligo-T capture probes attached to streptavidin-coated magnetic particles captured a defined amount of *in vitro* synthesized polyadenylated mRNAs from the yeast *Saccharomyces cerevisiae* under various hybridization conditions. After several stringency washes the selected mRNA was eluted from the beads. The recovery per cents were calculated from gel electrophoresed fragments.

### Experimental

Pre-blocking of Streptavidin-coated magnetic particles. 60 µL of Streptavidin-coated magnetic particles (Roche Cat no. 1 641 778 or 1 641 786) were pipetted into an Eppendorf tube for each sample. The magnetic separator was used to remove the supernatant 100 µL 1 µg/mL yeast RNA (Ambion cat. no. 7120G) diluted in TE (10 mM Tris-HCl, 1 mM EDTA, pH 7.5) was added to pre-block the magnetic particles for 5 min at room temperature. The particles were washed in 100 µL TE.

The prepared by mixing 4.2 µg *in vitro m*RNA (Yeast *ACT1*) into an Eppendorf tube and 200 µL GuSCN containing buffer (0, 0.5, 1, 2 or 4 M GuSCN (Sigma) in 25 mM Na-citrate (JT Baker), pH 7.0, 0.5 % sodium N-lauroyl sarcosinate (Sigma)) or 200 µL high NaCl-salt buffer 1 (20 mM Tris-HCl (pH 7, Ambion), 0.5 M NaCl, 1 mM EDTA (pH 8.0, Ambion) 0.1%(w/v) lauryl sarcosinate (Sigma)) was added and the samples were vortexed briefly. The biotinylated oligo-T capture probes (Table 3) were added to the sample preparation and transferred to the washed particles. The hybridization of the *in vitro* mRNA to the oligo-T capture probes and the streptavidin biotin complex to form was allowed 5 min at 37°C shaking the particles at 700 rpm in an Eppendorf Thermomixer (Radiometer Denmark). The particles were collected using the PickPen from Bionobile and a short washing step in 100µL GuSCN buffers was performed. Quickly recollected the particles were released into 100 µL washing buffer 1 (20 mM Tris-HCl (pH 7, Ambion), 0.5 M NaCl, 1 mM EDTA (pH 8.0, Ambion) 0.1%(w/v) lauryl sarcosinate (Sigma)), (Sambrook 2. ed.). This step was repeated. The particles were washed in 100 µL washing buffer 2 (20 mM Tris-HCl (pH 7, Ambion), 0.25 M NaCl (Ambion), 1 mM EDTA (pH 8.0, Ambion) 0.1 %(w/v) lauryl sarcosinate (Sigma)) and in 100 µL washing buffer 3 (20 mM Tris-HCl (pH 7, Ambion), 0.1 M NaCl (Ambion), 1 mM EDTA (pH 8.0 Ambion) 0.1 %(w/v) lauryl sarcosinate (Sigma)). Finally the particles were transferred to an Eppendorf tube containing 50 µL DEPC-H₂O (Ambion Cat. no. 9924). The sample was incubated for 5 min at 95°C and quenched on ice for 5 min to release the *in vitro* mRNA from the particles. The particles were recollected two times and the supernatant was span briefly (13.2 rpm 60 sec) and transferred to a clean siliconezed eppendorf tube (Ambion). The mRNA was ethanol precipitated by adding 1/10 volume 3 M NaOAc (Ambion), 150 µg/mL Glycogen Carrier (Ambion) and 2.5 volume. 96% Ethanol to the tube and freezed at -20°C over night. After spinning at least 30 min 16400xg at 4°C the supernatant was removed and the pellet washed with ice-cold 70% EtCH and air-dried. The pellet was dissolved in 10 µL DEPC treated H₂O.

For analysis 3 µL of the sample and a standard dilution curve of either *SSA4* were applied on a native 1-% agarose gel in 1x TAE buffer containing 1:10000 Gelstar. The gel was elechophoresesed for 20-30 min, 7 V/cm and the quantified on the Typhoon 9200 Imager (Amersham Pharmacia Biotech).

The recovery of *in vitro* mRNA in 0 0.5,1,2, and 4 M GuSCN containing buffer shows that the DNA_dT₂₀ capture probe has it optimum at 0.5 M GuSCN buffer (figure 6). The LNA_2.T capture probe has it optimum at 2 M GuSCN but maintain the same recovery efficiency at 4 M GuSCN compared to the DNA-dT₂₀ capture probe.

**Table 3**

| Comp. No. | Oligo Name: | Sequence 5'-: |
|---|---|---|
| 1 | DNA_dT₂₀ | 5'-biotin-tttttttttttttttttttttttttttt |
| 2 | LNA_2.T | 5'-biotin-TtTtTtTtTtTtTtTtTtTt |

### Example 5: Synthesis of Compound 3 (LNA_3.T):

DNA and LNA phosphoramidites were dissolved in anhydrous acetonitrile to a final concentration of 0.1M and placed on an Expedite DNA synthesiser. The Biotin amidite was likewise dissolved in anhydrous acetonitril according to manufacturers protocol and placed on the DNA synthesiser. The synthesis was performed by standard phosphoramidite chemistry. Thus, the first monomer, bound to the solid support, was detritylated and coupling to the second monomer was subsequent coupled using tetrazole as activator. After capping of any unreacted hydroxyl groups, the phosphit-triester was oxidised using iodine, base and water. The cycle was repeated with the different DNA and LNA monomers to synthesise the sequence, whereupon Biotin was added using the same cycle. The oligonucleotide was synthesised as its DMT-ON derivative. The oligonucleotide was subsequently deprotected with concentrated aqueous ammonia at 60°C for 4 hours, whereupon the oligonucleotide was evaporated to dryness. The oligonucleotide was dissolved in water and purified by RP-HPLC using 0.05M TEAA (triethylammonium acetate) buffer (pH 7,4) and acetonitrile. The oligonucleotide was collected, evaporated to dryness and detritylated using 80% aqueous acetic acid for 1 hour. The oligonucleotide was evaporated to dryness and dissolved in water before a second RP-HPLC purification was performed using the same solventsystem. The oligonucleotide was collected, evaporated to dryness and dissolved in water (0,5 ml). The concentration of the oligonucleotide was determined to be 150µM by measurement of the absorbance at 260 nm. The oligonucleotide was verified by MALDI-MS (calc. mass: 6623, found mass: 6626). Water, used for dissolution of the oligonucleotide was autoclaved before use.

### Example 6. The use of immobilized, anthraquinone-coupled LNA oligo(T) capture probes in poly(A)+RNA selection.

Anthraquinone coupled LNA oligo-T capture probes were photo-immobilized in PCR tubes using an anthraquinone (AQ) moiety as described by Koch *et al.*(Koch T, Jacobsen N, Fenstoldt J, Boas U, Fenger M, Jakobsen MH Photochemical Immobilization of Anthraquinone Conjugated Oligonucleotides and PCR Amplicons on Solid Surface. Bioconjugate Chemistry 2000;11:474-83). The LNA oligo-T capture probes consisted of an AQ moiety, either one or two hexaethylene trimer (HEG3) linker units and a 20-mer oligo-dT spiked at every third position with LNA T. For proof-of-principle, the recovery of *in vitro*-synthesized yeast *ACT1* or *SSA4* mRNA were detected. The recovered mRNA was visualized on a native agarose gel and quantified using a standard titration curve based on *SSA4.* The recovered mRNA could be used as template in a RT-PCR reaction.

### Example 7. Efficient LNA oligo(T)-based capture of poly(A)⁺RNA from low NaCl-salt binding buffer

The present method describes the use of LNA oligo-T capture probes to investigate the efficiency of polyadenylated messenger RNA (poly(A)⁺RNA) selection under high stringency hybridization conditions using a low concentration NaCl-salt binding buffer. The method enables efficient isolation of poly(A)⁺RNA directly from total RNA in a binding buffer containing a fifth of the NaCl-concentration that is required by other conventional methods. Subsequently the washing steps are also performed under high-ionic strength conditions favouring the destabilization of weak, non-specific interactions, preventing co-isolation of unwanted molecules. The low salt binding conditions may eliminate some of the poly(A)⁺RNA secondary structures, while the low-ionic strength washes greatly reduces ribosomal RNA and protein contamination.

### Experimental Procedures

### 1. Poly(A)⁺RNA isolation

Pre-blocking of Streptavidin-coated magnetic particles; 60 µL of Streptavidin-coated magnetic particles (Roche Cat no. 1 641 778 or 1 641 786) were pipetted into an Eppendorf tube for each sample. The magnetic separator was used to remove the supernatant. 100 µL 1 µg/mL yeast RNA (Ambion, USA cat. no. 7120G) diluted in TE (10 mM Tris-HCl (Ambion, USA), 1 mM EDTA (Ambion, USA), pH 7.5) was added to pre-block the magnetic particles for 5 min at room temperature. The particles were washed in 100 pL TE.

In an Eppendorf tube 100 µg yeast total RNA (from *Saccharomyces cerevisiae*) was prepared in a final volume of 50 µL DEPC-treated H₂O. 50 µL 2x binding buffer (20 mM Tris-HCl (pH 7.0, Ambion, USA), 0.2 M NaCl (Ambion, USA), 1 mM EDTA (pH 8.0, Ambion, USA) 0.1 % (^{w}/ᵥ) lauryl sarcosinate (Sigma, USA) was added and vortexed briefly. The biotinylated LNA oligo(T) capture probe (5'-biotin-C6-TtTtTtTtTtTtTtTtTtTt-3'; T = LNA thymine and t = DNA thymine) was added to the sample preparation together with the pre-blocked streptavidin-coated magnetic particles and allowed hybridization for 10 minutes at 37°C shaking (400 rpm in an Eppendorf Thermomixer (Radiometer, Denmark)). The particles were collected using a magnetic particle separator (Roche, USA) and the supernatant removed. The particles were washed three times in 100 µL wash buffer (20 mM Tris-HCl (pH 7, Ambion, USA), 0.05 M NaCl (Ambion, USA), 1 mM EDTA (pH 8.0, Ambion, USA) 0.1%(w/v) lauryl sarcosinate (Sigma, USA)). Finally, the poly(A)⁺RNA was eluted form the particles by adding 50 µL DEPC-H₂O (Ambion Cat. no. 9924), heated for 10 minutes at 65°C and quenched on ice for10 minutes.

### 2. Reverse transcription - PCR amplification

After the RNA isolation by the LNA oligo(T) capture probes 100 ng polyadenylated RNA was primed with 5 µg oligo-dT₁₂₋₁₈ primer (Amersham Biosciences) and heated 10 min at 70°C and quench on ice. The mixture was transferred to 20 µL cDNA synthesis reaction containing 50 mmol/L Tris-HCl (pH 8.3 at room temperature), 75 mmol/L KCl, 3 mmol/L MgCl₂, 10 mmol/L DTT (Invitrogen, USA), 1 mmol/L of each dATP, dCTP, dGTP, and dTTP (Amersham Biosciences, USA), 20U Superasin (Ambion, USA) and incubate 5 min at 37°C. 200U SuperScript^{™} II RT (Invitrogen, USA) was added and incubated 30 min at 37°C and 30 min at 42°C. Additional 200U of SuperScript^{™} II RT was added and the incubation time at 42°C was prolonged for one hour. Finally, the reaction was heated 5 min at 70°C and primers removed on a Sephacryl S-400 HR spin column (Pharmacia, USA) according to the manufacturer's recommendations.

The relevant cDNA fragment was amplified from first strand cDNA using specific primer sets for *S. cerevisiae ACT1* and *HSP78* genes, respectively. PCR reactions (50 µL) were prepared by mixing 15 mmol/L Tris-HCl, pH 8.0, 50 mmol/L KCl (GeneAmp Gold buffer, PE Biosystems); 2.5 mmol/L MgCl₂; 200 µmol/L of each dATP, dCTP, dGTP and dTTP (Amersham Pharmacia Biotech, USA); 0.4 mmoL/ forward primer (DNA technology, Denmark); 0.4 mmol/L reverse primer (DNA Technology, Denmark); 1.25 U (0.25 µL of a 5U/µl) AmpliTaq Gold polymerase (PE Biosystems, USA) and cDNA as template. After an initial 5 min denaturation step at 95°C, 25 cycles of PCR were carried out (60 s at 95°C, 60 s at 60°C and 60 s at 72°C), followed by extension at 72°C for 10 min. The amplicons were analysed by native agarose gel electrophoresis. The specific primer sets were: *ACT1:*
5'-ACGTGAATTCTTTCCATCCAAGCCGTTTTG3'
   and 
5- -GATCCCCGGGAATTGCCATGTTAGAAACACTTGTGGTGAA-
CGA-3',
   *HSP78:* 5'-ACGTGAGCTCTTTTGACATGTCAGAATTTCAAG-3'
   and

5 '-GATCCCCGGGAATTGCCATGTTACTTTTCAGCTTCCTCTTC-
AAC-3'.

For Northern blot analysis the PCR amplicons were agarose gel-purified by the QIAEX-II agarose gel extraction kit (Qiagen, USA) according to the protocol provided by the supplier.

### 3. Northern blot Analysis

The isolated *S*. *cerevisiae* poly(A)⁺RNAs (500 ng, wild type, wild type heat shocked, or ΔYDR258C mutant heat shocked) were subjected to electrophoresis in 1.5% agarose - 2.2 M formaldehyde gel (1) and blotted onto Hybond-N nylon membrane (Amersham Biosciences) with 10×SSC (1.5 M NaCl, 0.015 M sodium citrate, pH 7.0) as transfer buffer (2). The 748 bp and 756 bp PCR amplicon of the *ACT1* and *HSP78* cDNA, respectively, was ³²P-labelled (> 5×10⁸ cpm/µg) by random-priming (Megaprime^{™} DNA labelling system, Amersham Biosciences) according to the manufacture's recommendations. Redivue α-³²P-dCTP (3000 Ci/mmol) was purchased from Amersham Biosciences. The radioactive labelled probes were hybridised with the filter at 42°C for 18 hours in ULTRAhyb^{™} (Ambion). The filter was washed twice in 2xSSC, 0.1% SDS at 42°C for 5 min, then twice in 0.1×SSC, 0.1% SDS at 42°C for 15 min. After autoradiography on a Storage Phosphor screen (Amersham Biosciences) and image analysis quantification by a Typhoon 9200 scanner.

### 4. Results and discussion

Total RNA preparations were extracted from S. *cerevisiae* wild type, wild type heat shocked cells, and deltaYDR258C mutant heat shocked cultures, respectively using the FastRNA Red kit from Bio101, USA, according to the manufacturer's instructions. The total RNA preparations were subjected to the LNA oligo(T) capture protocol as described above, and the quality of the isolated mRNA preparations was subsequently assessed by RT-PCR and Northern blot analysis.

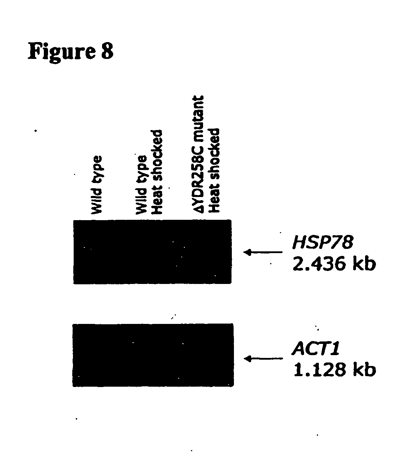

Figure 8 shows the results of RT-PCR, in which 100 ng LNA oligo(T)-captured poly(A)⁺RNA isolated from wild type heat shocked or deltaYDR258C mutant heat shocked (the HSP78 gene is deleted in the deltaYDR258C mutant) total RNA was reverse transcribed into first strand cDNA and subsequently PCR amplified using specific primer sets for the yeast *HSP78* and *ACT1* genes. No PCR fragments for the *HSP78* were detected when cDNA from the deltaYDR258C mutant was used as template for RT-PCR, in accordance with the *HSP78* deletion, whereas a *HSP78* specific cDNA fragment is readily detected from the wild-type yeast poly(A)⁺RNA by RT-PCR. By comparison, an ACT1-specific PCR fragment was detected in mRNA preparations from both yeast strains. The Northern blot analysis (Figure B.) was performed according to Sambrook and Russell (Sambrook, J. and Russell, D.W. (2001) Molecular Cloning : a Laboratory Manual. Cold Spring Harbor Laboratory Press, 2001, Cold Spring Harbor, N.Y.). 500 ng of LNA oligo(T)-captured poly(A)⁺RNA was applied on the Northern gel from each mRNA sample preparation. The Northern blot was probed with two ³²P-labelled probes for *ACT1* and *HSP78,* respectively. The image analysis of the hybridised Northern blot demonstrates that the *HSP78* gene is up-regulated by 14-fold upon a heat shock treatment of the wild type yeast strain. In contrast, the *HSP78* mRNA is not detected in the mRNA sample isolated from the heat shocked deltaYDR258C mutant strain, in accordance with the deleted *HSP78* gene. The Northern blot analysis clearly demonstrates that the poly(A)⁺RNA sample preparations are highly intact as visualized by the sharp bands on the hybridised Northern blot for both mRNAs without any smearing.

### Example 8. Isothermal RNA amplification using T7 anchored LNA-(T)₂₀vn primer.

A novel, LNA-substituted T7 RNA polymerase site-containing primer was used to synthesize cRNA (complementary RNA) from *in vitro* synthesised yeast HSP78 polyadenylated spike RNA. The present example demonstrates the utility of using an LNA-substituted, T7 anchored LNA-T primer in synthesis of RNA *in vitro.* The advantages of using an LNA-T anchor primer as opposed to a conventional DNA oligo(dT) T7 anchor primer are as follows:
(i) more efficient capture of mRNA, including mRNA capture from limited cell populations, e.g. from laser capture microdissected cells, cell lysates and total RNA preparations followed by cDNA synthesis and isotherm RNA (cRNA) amplification for subsequent analysis;
(ii) *in vitro* RNA synthesis at higher temperatures due to increased duplex stability of the LNA-T anchor primer using a thermostable RNA polymerase, resulting in full-length cRNA
(iii) the possibility to combine efficient mRNA sample preparation using LNA-T anchor primer. Either from guanidinium thiocyanate-lyse cell extracts directly, or from total RNA preparations under low salt binding conditions (high stringency hybridization conditions), followed directly by cDNA synthesis and cRNA amplification with the same primer.

### 1. In vitro synthesis ofyeast HSP78 RNA

### 1. 1.Isolation of yeast genomic DNA

Genomic DNA was prepared from a wild type standard laboratory strain of *Saccharomyces cerevisiae* using the Nucleon MiY DNA extraction kit (Amersham Biosciences, USA) according to the supplier's instructions.

### 1.2.PCR amplification

Amplification of the yeast HSP78 gene fragment was done by standard PCR using yeast genomic DNA as template. In the first step of amplification, a forward primer containing a restriction enzyme site and a reverse primer containing a universal linker sequence were used. In this step 20 bp was added to the 3'-end of the amplicon, next to the stop codon. In the second step of amplification, the reverse primer was exchanged with a nested primer containing a poly-T₂₀ tail and a restriction enzyme site. The HSP78 amplicon contains 736 bp of the *HSP78* ORF plus 20 bp universal linker sequence and a poly-A₂₀ tail.
The PCR primers used were;
YDR258C-For-SacI: acgtgagctcttttgacatgtcagaatttcaag
YDR258C-Rev-Uni: gatccccgggaattgccatgttacttttcagcttcctcttcaac
Uni-polyT-BamHI: acgtggatcctttttttttttttttttttgatccccgggaattgccatg,

### 1.3. Plasmid DNA constructs

The PCR amplicon was cut with the restriction enzymes, *Eco*RI + *Bam*HI. The DNA fragment was ligated into the pTRIamp18 vector (Ambion) using the Quick Ligation Kit (New England Biolabs) according to the manufacturer's instructions and transformed into *E. coli* DH-5α by standard methods.

### 1.4. DNA sequencing

To verify the identity of the HSP78 clone, isolated plasmid DNA was sequenced using M13 forward and M13 reverse primers and analysed on an ABI 377.

### 2. Synthesis of cRNA using HSP78 spike RNA as template

One µg *in vitro* HSP78 spike mRNA was used as template and the MessageAmp^{™} aRNA kit (Ambion, USA) was used for cRNA synthesis according to the manufacturer's instructions, except that 50 µM final concentration of unique T7 oligo(dTt₁₀vn) primer was used instead of the primer from the kit. The sequence of the unique primer is 5'-ggccagtgaattgtaatacgactcactatagggaggcggTtTtTtTtTtTtTtTtTtTtvn-3'. Before ncRNA purification (according to manufacturer's instructions), the double-stranded cDNA template was removed from the reaction mixture by DNase I treatment for 30 min. at 37°C. The yield of the resulting cRNA (Table I) was measured using a Nanodrop spectrophotometer (Nanodrop, USA) and the quality of the in vitro synthesized spike RNA was assessed by gel electrophoresis on a 1 % agarose gel.

**Table 4.**

| **The yield of HSP78 cRNA using a T7 anchored LNA-(T)₂₀vn primer.** | | |
|---|---|---|
| | Input HSP78 template RNA | Yield of HSP78 cRNA |
| RNA | 1.00 µg | 18.80 µg |

### Example 9. Covalent immobilization of anthraquinone-coupled LNA-T oligonucleotides on a solid support by irradiation for mRNA sample preparation in guanidinium thiocyanate lysis buffer

### Titration of the optimal anthraquinone (AQ)-conjugaledoligo-TcaPture probe concentration

### Immobilization of anthraquinone-coupled oligo-T capture probes

LNA and control DNA oligonucleotide (Table A below) were synthesized containing an anthraquinone (AQ2) and different linkers. Each oligonucleotide was diluted in 0.2 M NaCl to a final concentration of 0, 3.125, 6.25, 12.5, 25, 50 or 100 µM, respectively, and 100 µL per well were dispensed into microtiter wells (C96, polysorp, Nunc, Denmark). The oligonucleotide solutions were irradiated for 15 minutes under soft UV light. After irradiation the microplate was washed with four times of 300 µL DEPC-treated water (Ambion, USA).

### In vitro synthesis of polyadenylated SSA4 spike RNA

Genomic DNA was prepared from a wild type standard laboratory strain of S. *cerevisiae* using the Nucleon MiY DNA extraction kit (Amersham Biosciences, USA) according to the supplier's instructions. Amplification of partial yeast genes was performed by standard PCR using yeast genomic DNA as template. In the first step of amplification, a forward primer containing a restriction enzyme site and a reverse primer containing a universal linker sequence were used. In this step 20 bp was added to the 3'-end of the amplicons, next to the stop codon. In the second step of amplification, the reverse primer was exchanged with a nested primer containing a poly-dT₂₀ tail and a restriction enzyme site. The SSA4 PCR amplicon contains 729 bp of the *SSA4* ORF plus a 20 bp universal linker sequence and a poly-dA₂₀ tail.

The PCR primers used were;
YER103W-Rev-Uni: 
5'-GATCCCCGGGAATTGCCATGCTAATCAACCTCTTCAACCGTT-
GG-3',
 YER103W-For-SacI:
5'-ACGTGAGCTCATTGAAACTGCAGGTGGTATTATGA-3',
Uni-polyT-BamHI:

5'-A CGTGGATCCTTTTTTTTTTTTTTTTTTTTGATCCCCGGGAATT-
GCCATG-3'.

The PCR amplicon was cut with restriction enzymes, *Sac*I *+ Bam*HI, and the purified SSA4 fragment was ligated into the pTRIamp18 vector (Ambion, USA) using the Quick Ligation Kit (New England Biolabs, USA) according to the manufacturer's instructions and transformed into *E. coli* DH-5α by standard methods. DNA sequencing (ABI 377) was used to verify the plasmid construct by the use of M13 forward and M13 reverse primers.

### Capture and detection of the SSA4 spike mRNA by immobilized oligo-T capture probes and a biotinylated LNA detection probe

Fifty nanograms (ng) of the *in vitro* polyadenylated *SSA4* mRNA was diluted in the guaninidinium thiocyanate (GuSCN) buffer (4 mol/L GuSCN (Sigma), 25 mmol/L sodium citrate (JT Baker), pH 7.0, 0.5 g/100 mL sodium N-lauroyl sarcosinate (Sigma, USA)). The mixture was heated to 65°C 10 minutes and quenched on ice. The SSA4 mRNA solution was dispersed into the wells by adding 50 ng SSA4 spike mRNA in 100 µL per well and incubated for 15 minutes at room temperature. The microtiter wells were washed three times in wash buffer (0.05 mol/L NaCl 20 mmol/L Tris-HCl, pH 7.6, 1 mmol/L EDTA, pH 8, 0.1 g/100 mL sodium N-lauroyl sarcosinate). The detection was carried out by either a biotinylated DNA (biotin-C₆-aatcttcccttatcgttagtaattgtaatcttgtt; DNA in lower cases)
or LNA detection probe
(biotin-C₆-AatmCttmCccTtaTcgTtaGtaAttGtaAtcTtgTt;
DNA in lower case and LNA in upper case).

The detection probe was diluted to 0.1 µM in wash buffer and 100 µL was dispersed per well and allowed to hybridize for 15 minutes at room temperature. The detection probe solution was removed from the wells, and 100 µL per well of 1 µg/mL horse radish peroxidase-conjugated streptavidin (Pierce, USA) diluted in wash buffer was added to the wells and incubated at room temperature for 15 minutes. The wells were washed three times in wash buffer and assayed for peroxidase activity by adding 100 µL of TMB substrate solution (3,3',5,5'-tetramethylbenzidine, Pierce, USA), the reaction was stopped after 60 minutes by adding 100 µL 0.5 M H₂SO₄ and the absorbance at 450 nm was read in a microtiter-plate reader (Wallac Victor²).

### Results and discussion

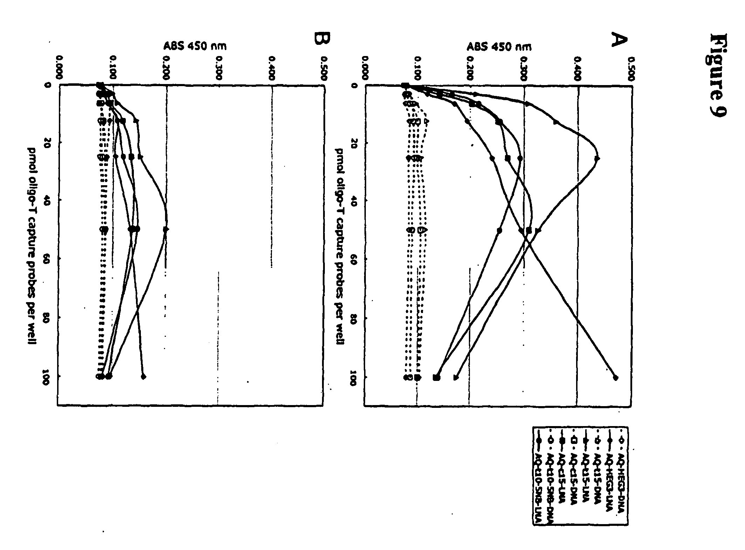

Figure 9A demonstrates the detection of the *SSA4* spike mRNA when the polyA::oligoT capture is performed in 4M GuSCN buffer and high stringency washes employing the different LNA oligo-T capture probes combined with a SSA4-specific LNA detection probe. In contrast, the control DNA oligo-(dT) capture probes do not show any detection signals under these assay conditions. When the DNA detection probe (Figure 9B) is used instead of LNA probe to detect the captured SSA4 spike RNA, low SSA4 signals were detected from the LNA-T capture probes only, while no signals were obtained from the DNA (dT) control probes. It should be noted that the DNA detection probe hybridises only weakly to its target under the high stringency hybridisation conditions used here. In conclusion, only LNA oligo-T capture probes are able to capture polyadenylated RNA in 4M guanidinium thiocyanate hybridisation buffer. Furthermore, when high stringency hybridization conditions (0.05 M NaCl) are used for detection of the SSA4 spike mRNA, only the LNA detection probe is able to hybridise and detect the mRNA target. The optimal capture probe concentration differs with regard to the different linkers used in the various anthraquinone.-coupled LNA-T capture oligonucleotides. Under the experimental conditions presented here the optimal concentrations were: 25 pmol per microplate well for AQ₂-t15- and AQ₂-t10-NB5-, respectively; 50 pmol per well for AQ₂-c15-, and at least 100 pmol per well for AQ₂-HEG₃- linker construct

**Table 5.**

| **Anthraquinone-coupled LNA-T and DNA (dT) capture probes.** | | |
|---|---|---|
| Comp. No. | Oligo Name: | Sequence 5'-: |
| 11 | AQ-HEG₃-t20 | AQ₂-HEG₃-tttttttttttttttttttt |
| 12 | AQ-HEG₃-2.T | AQ₂-HEG₃-TtTtTtTtTtTtTtTtTtTt |
| 13 | AQ-t15-t20 | AQ2-t15-tttttttttttttttttttt |
| 14 | AQ-t15-2.T | AQ₂-t15-TtTtTtTtTtTtTtTtTtTt |
| 15 | AQ-c15-t20 | AQ₂-c15- |
| 16 | AQ-c15-2.T | AQ₂-c15-TtTtTtTtTtTtTtTtTtTt |
| 17 | AQ-t10-NB5-t20 | AQ₂-t10-NB5-tttttttttttttttttttt |
| 18 | AQ-t10-NB5- | AQ₂-t10-NB5-TtTtTtTtTtTtTtTtTtTt |

| | | |
|---|---|---|
| AQ: anthraquinone; HEG: hexa-ethylene glycol; t15: 15-mer deoxy-thymine; c15: 15-mer deoxy-cytosine; t10-NB5: 10-mer deoxy-thymine 5-mer non-base; t: DNA thymine and T: LNA thymine. | | |

### Example 10. Titration of the spike mRNA target

### Immobilization of anthraquinone-coupled oligo-T capture probes

The AQ-coupled oligo-T capture probes (Table A) were immobilized onto microplate wells as in the previously example. However, the optimal concentrations of each AQ-linker-LNA-T probe construct were applied (AQ-HEG3-: 100 pmol per well, AQ-c15-: 50 pmol per well, and AQ-t15- and AQ-t10-NB5-: 25 pmol per well).

### Capture and detection of in vitro SSA4 mRNA by immobilized oligo-T capture probes and biotinylated LNA detection probe

100 nanograms of the *in vitro* polyadenylated *SSA4* spike mRNA was diluted in GuSCN buffer (4 mol/L GuSCN (Sigma, USA), 25 mmol/L sodium citrate (JT Baker), pH 7.0, 0.5 g/100 mL sodium N-lauroyl sarcosinate (Sigma, USA)). The solution was heated to 65°C 10 minutes and quenched on ice. The polyadenylated *SSA4* mRNA solution was dispersed into the wells by adding 100 ng in 100 µL per well followed by a two-fold dilution series. The final concentrations were 0.87, 3.1, 6.25,12.5, 25, 50, or 100 ng SSA4 mRNA in 100 µL per well and the samples were incubated for 45 minutes at room temperature. The microtiter wells were washed three times in wash buffer (0.05 mol/L NaCl 20 mmol/L Tris-HCl, pH 7.6,1 mmol/L EDTA, pH 8,0.1g/100 mL sodium N-lauroyl sarcosinate). The LNA detection probe (biotin-C₆-AatmCttmCccTtaTcgTtaGtaAttGtaAtcTtgTt;
DNA in lower cases and LNA in upper cases) was diluted to 0.1 µM in 1×SSCT buffer (15 mM sodium citrate, 0.15 M NaCl, pH 7.0, (Eppendorf) 0.1 mL/100 mL Tween 20) and 100 µL was dispersed per well and allowed hybridisation for 30 minutes at room temperature. The wells were washed three times in 1×SSCT buffer and 100 µL per well 1 µg/mL horse radish peroxidase-conjugated streptavidin (Pierce) diluted in 1×SSCT buffer was added to the wells and incubated 15 minutes. The wells were washed three times in 1×SSCT buffer and assayed for peroxidase activity by adding 100 µL of TMB substrate solution (3,3',5,5'-tetramethylbenzidine, Pierce) the reaction was stopped after 3 minutes 30 seconds by adding 100 µL 0.5 M H₂SO₄ and the absorbance at 450 nm was read in a microtiter-plate reader (Wallac Victor²).

### Results and discussion

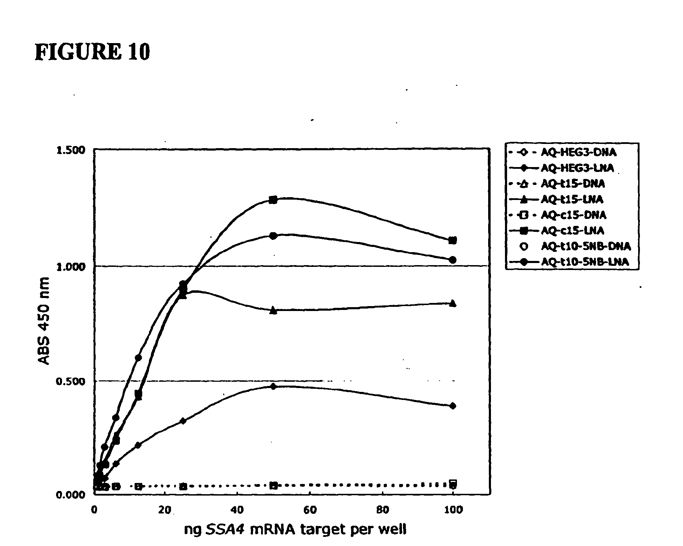

Figure 10 demonstrates efficient capture and detection of the polyadenylated SSA4 spike mRNA when different AQ-coupled LNA-T capture probes are used to capture the spike mRNA in 4M GuSCN buffer, combined with detection using a biotinylated LNA detection probe. Even mRNA amounts of less than one nanogram were readily detected with the assay. In contrast, the SSA4 spike mRNA could not be detected, when the DNA oligo(dT) control capture probes were used in the assay.

### Example 11. Isolation of poly(A)⁺RNA from yeast total RNA by immobilized LNA oligo-T capture probes followed by detection of SSA4 mRNA using a biotinylated LNA detection probe

Isolation of poly(A)⁺RNA from yeast *S. cerevisiae* total RNA followed by detection of SSA4 mRNA using a LNA detection probe was carried out, essentially as described in example 10, except that 20 µg of total RNA extracted from wild type heat shocked yeast cells were applied to each of the microplate wells, containing AQ-coupled oligo-T capture probe constructs, in 100 µL GuSCN buffer (4 mol/L GuSCN (Sigma), 25 mmol/L sodium citrate (JT Baker), pH 7.0, 0.5 g/100 mL sodium N-lauroyl sarcosinate (Sigma)) per well. Before adding the total RNA the samples to the microtiter wells, they were heated to 65°C and quenched on ice. The binding, washing and detection procedures were as described in the example 10.

### Results and discussion

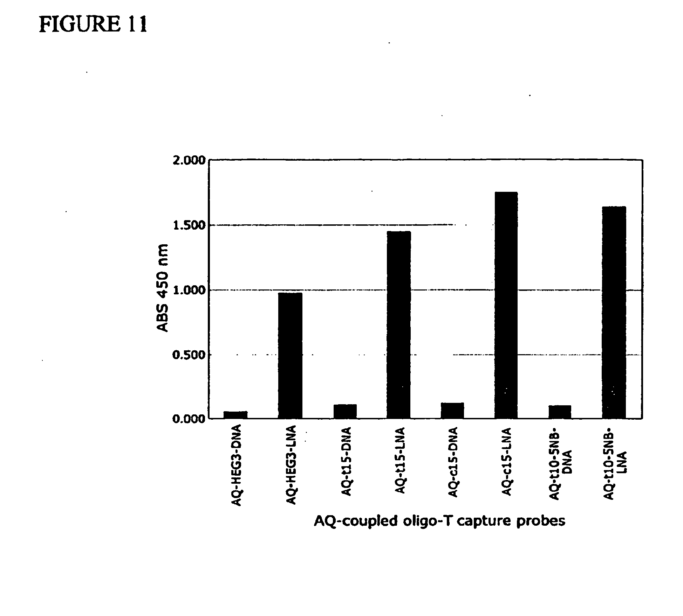

Figure 11 demonstrates that the different AQ-coupled LNA oligo-T capture probes, coupled covalently onto microplate wells are able to capture and detect the SSA4 mRNA when hybridised in 4 M GuSCN followed by detection with the biotinylated SSA4-specific LNA detection probe. By contrast, the control DNA oligo(dT) capture probes did not show a detection signal for SSA4 mRNA.

### Example 12. Isolation of poly(A)⁺RNA using LNA-T capture under high stringency hybridisation conditions using a low salt concentration

### NaCl step gradient using in vitro synthesized ACT1 spike mRNA

In an Eppendorf tube 0.5 pg of *in vitro* synthesized, polyadenylated ACT1 mRNA was combined in a final volume of 50 µL DEPC-treated H₂O. 50 µL 2x binding buffer (20 mM Tris-HCl (pH 7.0, Ambion, USA), X M NaCl (Ambion, USA) where X is 0.05, 0.1, 0.2, 0.3, 0.4, or 0.5M NaCl, respectively; and 1 mM EDTA (pH 8.0, Ambion, USA) 0.1 % (^{w}/ᵥ) lauryl sarcosinate (Sigma, USA) was added and mixed briefly. The biotinylated LNA oligo(T) capture probe (5'-biotin-C6-TtTtTtTtTtTtTtTtTtTt-3'; T = LNA thymine and t = DNA thymine) was added to the sample preparation together with the pre-blocked streptavidin-coated magnetic particles (preparation described in previous examples) and allowed to hybridize for 10 minutes at 37°C shaking (400 rpm in an Eppendorf Thermomixer (Radiometer, Denmark)). The particles were collected using a magnetic particle separator (Roche, USA) and the supernatant removed. The particles were washed three times in 100 µL wash buffer (20 mM Tris-HCl (pH 7, Ambion, USA), 0.05 M NaCl (Ambion, USA), 1 mM EDTA (pH 8.0, Ambion, USA) 0.1%(w/v) lauryl sarcosinate (Sigma, USA)). Finally, the poly(A)⁺RNA was eluted form the particles by adding 50 µL DEPC-H₂O (Ambion Cat. no. 9924, USA), heated for 10 minutes at 65°C and quenched on ice for 10 minutes.

For analysis, 10 µL of the samples and a standard dilution curve of *ACT1* mRNA were applied on a native 1 % agarose gel in 1x TAE buffer containing 1:10000 Gelstar. The gel was electrophoresesed for 20-30 min, 7 V/cm and then quantified on the Typhoon 9200 Imager (Amersham Pharmacia Biotech, USA).

### Results and discussion

The quantification of the captured ACT1 spike mRNA (Figure 12.) shows that the LNA oligo-T capture probe is able to capture the *in vitro* ACT1 spike mRNA under low salt conditions. At 0.05 M NaCl concentration, the LNA oligo-T capture probe shows a recovery of 80% compared to the control DNA oligo-T capture probe with a recovery less than 20%. The LNA oligo-T capture probe, when hybridized in 0.1 M NaCl shows a two-fold increase in yield compared to the control DNA oligo(dT).

### Example 13. Isolation of poly(A)+RNA from C. elegans worm extracts lysed in 4 M GuSCN buffer and mRNA validation using Northern blot analysis

### Poly(A)⁺RNA isolation

The *C*. *elegans* N2 strain was grown in S-media, with *E*. *coli* NA22 food, at 23°C. The entire culture was sucrose density cleaned by standard methods before taking samples. *C*. *elegans* mixed stage worms were harvested and re-suspended in either four volumes of RNA*later*^{™} (Ambion, USA) or 4M GuSCN lysis buffer and immediately frozen in liquid nitrogen and stored at - 80°C. *C*. *elegans* mixed stage worms stored in RNA*Later*^{™} or the GuSCN lysis buffer were thawed, and the wet weight was calculated by removing the supernatant (2 min 4000g) and weighing. The pellet was subsequently re-suspended in the same volume. Aliquots of *C*. *elegans* mixed staged worms were spun for 2 min at 4000g and 200 µL GuSCN lysis buffer was added and the samples were vortexed briefly. Quartz sand was added and mixed for 2 min on ice using a pestle for pulverising the sample. A short spin (60 s at 16100g) was performed and the supernatant was carefully removed to a clean tube. The lysate was heated for 30 min at 65°C on an Eppendorf Thermomixer (shaking 700 rpm, Radiometer, Denmark). The tube was spun briefly (60 s at 16100g) and the supernatant transferred to a clean RNase-free tube.

In Eppendorf tubes corresponding to 0, 2.8, 5.5, 11, 22, or 44 mg wet weight *C. elegans* worms, respectively, was mixed in a final volume of 200 µL GuSCN containing buffer (4 M GuSCN (Sigma, USA) in 25 mM Na-citrate (JT Baker), pH 7.0, 0.5 % sodium N-lauroyl sarcosinate (Sigma, USA)) as described in previous examples. To each of the samples 200 pmol biotinylated LNA-T or DNA oligo(T) capture probe (5'-biotin-C₆-TtTtTtTtTtTtTtTtTtTt-3' or 5'-biotin-C₆-tttttttttttttttttttt-3'; T = LNA thymine and t = DNA thymine) was added together with the pre-blocked streptavidin-coated magnetic particles (described previously) and allowed to hybridize for 10 minutes at 37°C shaking (400 rpm in an Eppendorf Thermomixer (Radiometer, Denmark)). The particles were collected using a magnetic particle separator (Roche, USA) and the supernatant was removed. The particles were washed three times in 100 µL wash buffer (20 mM Tris-HCl (pH 7, Ambion, USA), 0.05 M NaCl (Ambion, USA), 1 mM EDTA (pH 8.0, Ambion, USA) 0.1%(w/v) lauryl sarcosinate (Sigma, USA)). Finally, the poly(A)⁺RNA was eluted from the particles by adding 50 µL DEPC-H₂O (Ambion Cat. no. 9924), heated for 10 minutes at 65°C and quenched on ice for 10 minutes.

### Reverse transcription - PCR amplification

After the mRNA isolation by the LNA oligo(T) capture, 100 ng of polyadenylated RNA was primed with 5 µg oligo-dT₁₂₋₁₈ primer (Amersham Biosciences, USA) and heated 10 min at 70°C and quenched on ice. The mixture was transferred to 20 µL first strand cDNA synthesis reaction mixture containing 50 mmol/L Tris-HCl (pH 8.3 at room temperature), 75 mmol/L KCl, 3 mmol/L MgCl₂, 10 mmol/L DTT (Invitrogen, USA), 1 mmol/L of each dATP, dCTP, dGTP, and dTTP (Amersham Biosciences, USA), 20U Superasin (Ambion, USA) and incubated for 5 min at 37°C. 200U SuperScript^{™} II RT (Invitrogen, USA) was added and the reaction mixture was incubated for 30 min at 37°C and 30 min at 42°C. Additional 200U of Superscript^{™} II RT were added and the incubation time at 42°C was prolonged for one hour. Finally, the reaction was heated 5 min at 70°C and primers removed on a Sephacryl S-400 HR spin column (Pharmacia, USA) according to the manufacturer's recommendations.

The relevant cDNA fragment was amplified from first strand cDNA using a specific primer set for C. *elegans* 26S gene, PCR reactions (50 µL) were prepared by mixing 15 mmol/L Tris-HCl, pH 8.0, 50 mmol/L KCl (GeneAmp Gold buffer, PE Biosystems); 2.5 mmol/L MgCl₂; 200 µmol/L of each dATP, dCTP, dGTP and dTTP (Amersham Pharmacia Biotech, USA); 0.4 mmoL/ forward primer (DNA technology, Denmark); 0.4 mmol/L reverse primer (DNA Technology, Denmark); 1.25 U (0.25 µL of a 5U/µl) AmpliTaq Gold polymerase (PE Biosystems, USA) and cDNA as template. After an initial 5 min denaturation step at 95°C, 25 cycles of PCR were carried out (60 s at 95°C, 60 s at 60°C and 60 s at 72°C), followed by extension at 72°C for 10 min. The PCR products were analysed by native agarose gel electrophoresis. The specific primer set was: *C*. *elegans* 26S rRNA sense 5'-GCCAGAGGAAACTCTGGTGGAAGTCC-3' and C. elegans 26S rRNA revcom 5'-AGCCTCCCTTGGTGTTTTAAGGGCCG-3'. For Northern blot analysis the PCR amplicons were agarose gel-purified by the QIAEX-II agarose gel extraction kit (Qiagen, USA) according to the protocol provided by the supplier.

### Northern blot analysis

Equal volumes of the isolated C. *elegans* poly(A)⁺RNAs were subjected to electrophoresis in 1.5% agarose - 2.2 M formaldehyde gel and blotted onto Hybond-N nylon membrane (Amersham Biosciences) with 10×SSC (1.5 M NaCl, 0.015 M sodium citrate, pH 7.0) as transfer buffer (described previously). A 483 bp PCR amplicon of the *C*. *elegans RPL-21* cDNA (a kind gift from M. Zagrobelny, University of Copenhagen, Denmark) was ³²P-labelled (> 5×10⁸ cpm/µg) by random-priming (Megaprime^{™} DNA labelling system, Amersham Biosciences) according to the manufacturer's recommendations. Redivue α-³²P-dCTP (3000 Ci/mmol) was purchased from Amersham Biosciences. The radioactively labelled probe was hybridised with the filter at 42°C for 18 hours in ULTRAhyb^{™} (Ambion, USA) according to the manufacturer's instructions. The filter was washed twice in 2xSSC, 0.1% SDS at 42°C for 5 min, then twice in 0.1×SSC, 0.1% SDS at 42°C for 15 min. After autoradiography on a Storage Phosphor screen (Amersham Biosciences) and image analysis quantification by a Typhoon 9200 scanner, the probe was removed from the filter according to the manufacturers instructions. Then the filter was re-hybridised with a 989 bp PCR amplicon of the 26S rRNA cDNA. The ³²P-labelling of the probe, hybridisation to the filter and the washing steps were identical to those with the RPL-21 probe.

### Results and discussion

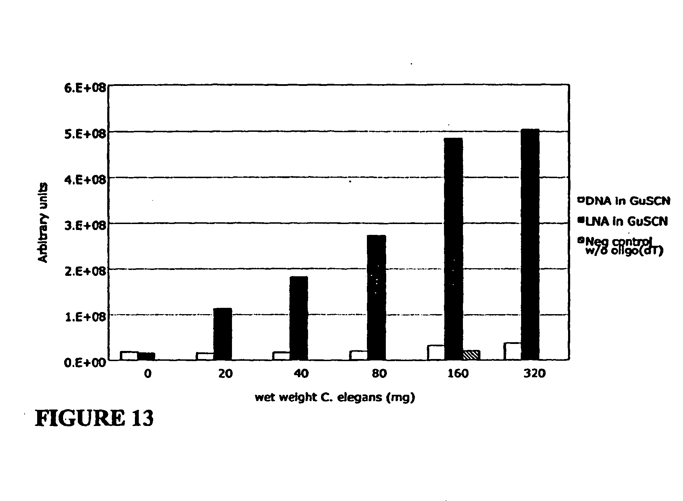

Direct isolation of poly(A)⁺RNA from *C*. *elegans* worm extracts lysed in 4 M GuSCN buffer resulted in efficient mRNA capture when the LNA_2.T capture probe method was employed (Figure 13), while only very low yields were obtained with DNA (dT) capture probes. The mRNA yield increase was linear upon using increasing amounts of input *C*. *elegans* worm extract.

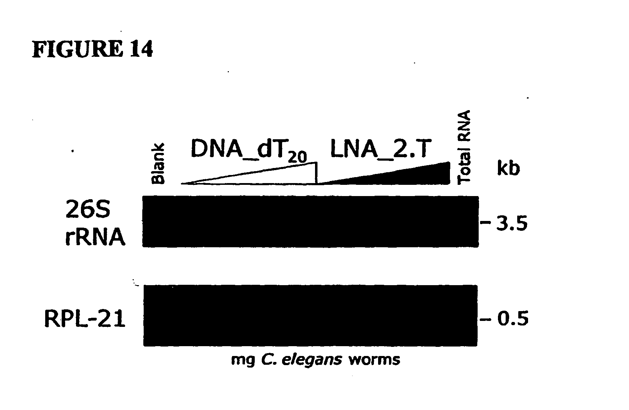

The data obtained by the Northern blot analysis followed by the image analysis quantification (Figure 14) demonstrate a 50-fold increase in the isolation of *RPL-21* mRNA when using LNA_2.T compared to the reference DNA-dT₂₀ using the same amount of starting material. In addition, the *C*. *elegans* poly(A)⁺RNA samples are highly intact, as revealed by the Northern blot analysis. Since the rRNA ratio in the LNA_2.T and DNA-dT₂₀ purified mRNA samples is significantly lower than the RPL-21 ratio, it can be concluded that the LNA-captured mRNA contains significantly less contaminating rRNA compared to the DNA (dT) control. Combined, these results demonstrate that the LNA oligo(T) capture method results in the isolation of highly intact poly(A)⁺RNA in the presence of 4 M GuSCN, in which an extremely potent inhibition of nucleases, including endogeneous RNases and proteases is obtained.

**Table 6**

| Wet weight C. elegans worms in mg | The ratio of 26S LNA/DNA | The ratio of RPL-21 LNA/DNA |
|---|---|---|
| 2.8 | 10.6 | 34.8 |
| 5.5 | 16.8 | 45.4 |
| 11 | 10.2 | 55.0 |
| 22 | 4.2 | 36.3 |
| 44 | 3.5 | 29.5 |

The invention has been described in detail including preferred embodiments thereof. However, it is understood that those skilled in the art, upon consideration of this disclosure, may make modifications and improvements.

## Claims

1. A method for detecting and/or isolating via hybridisation a nucleic acid molecule having a homopolymeric sequence comprising:
a) treating a sample containing nucleic acid compounds with a lysing buffer comprising a chaotropic agent; and
b) treating said sample with a Locked Nucleic Acid (LNA) oligonucleotide capture probe comprising at least twenty repeating consecutive nucleotides to thereby detect and/or isolate via hybridisation a nucleic acid molecule having said homopolymeric sequence, wherein said homopolymeric sequence is a poly (A) tail of a eukaryotic mRNA;
and wherein the LNA oligonucleotide capture probe comprises a compound of the formula:
5'-)Y^{q}-(X^{p}-Yⁿ)ₘ-X^{p}-Z-3'
wherein X is an LNA monomer, Y is a DNA monomer; Z represents an optional DNA monomer; p is an integer from about 1 to about 15; n is an integer from about 1 to about 15 or n represents 0; q is an integer from about 1 to about 10 or q = 0; and m is an integer from about 5 to about 20, wherein the LNA oligonucleotide capture probe hybridizes to its complementary sequence.

2. The method of claim 1, wherein the LNA oligonucleotide capture probe is covalently attached to a solid support.

3. The method of claim 1 or claim 2, wherein the LNA oligonucleotide capture probe is synthesized with an anthraquinone moiety and a linker at the 5'-end or the 3'-end of said probe.

4. The method of claim 3, wherein said linker is selected from the group comprising one or more of a hexaethylene glycol monomer, dimer , trimer, tetramer, pentamer, hexamer, or higher hexaethylene glycol polymer; a poly-T sequence of 10-50 nucleotides in length or a poly-C sequence of 10-50 nucleotides in length or longer; or a non-base sequence of 10-50 nucleotide units in length or longer.

5. The method of claim 2, wherein said solid support is a polymer support selected from the group consisting of a microtiter plate, polystyrene beads, latex beads, a polymer microscope slide or a polymer-coated microscope slide or a microfluidic slide.

6. The method of any one of the preceding claims, wherein the LNA oligonucleotide capture probe is complementary to a homopolymeric nucleotide comprising at least one nucleobase that is different than the bases comprising the homopolymeric nucleic acid sequence.

7. The method of any one of the preceding claims, wherein the LNA oligonucleotide comprises at least thirty, at least forty, or at least fifty repeating consecutive nucleotides.

8. The method of claim 4, wherein a covalent coupling onto a solid polymer support of said LNA oligonucleotide probe is carried out via excitation of the anthraquinone moiety using UV light.

9. The method of claim 1, wherein the LNA oligonucleotide molecule is selected from the following table:
| Comp. No. | Oligo Name: | Sequence 5'-: |
|---|---|---|
| 2 | LNA_2.T | 5'-biotin-TtTtTtTtTtTtTtTtTtTt |
| 3 | LNA_3.T | 5'-biotin-TttTttTttTttTttTttTt |
| 6 | LNA_4.T | 5'-biotin-ttTtttTtttTtttTtttTt |
| 7 | LNA_5.T | 5'-biotin-tttTttttTttttTttttTt |
| 8 | LNA_T₂₀ | 5'-biotin- |
| | | TTTTTTTTTTTTTTTTTTTT |
| 9 | LNA_TT | 5'-biotin-ttTTtttTTtttTTtttTTt |
| 10 | LNA_TTT | 5'-biotin-ttTTTttttTTTttttTTTt |
| 11 | AQ-HEG₃-2.T | AQ-HEG₃-TtTtTtTtTtTtTtTtTtTt |
| 12 | AQ-t15-2.T | AQ-t15-TtTtTtTtTtTtTtTtTtTt |
| 13 | AQ-c15-2.T | AQ-c15-TtTtTtTtTtTtTtTtTtTt |
| 14 | AQ-t10-NB5-2.T | AQ-t10-NB5-TtTtTtTtTtTtTtTtTtTt |
wherein AQ refers to anthraquinone, HEG refers to hexa-ethylene glycol, t15 refers to 15-mer deoxy-thymine, c15 refers to 15-mer deoxy-cytosine, t10-NB5 refers to 10-mer deoxy-thymine 5-mer non-base, and t refers to DNA thymine and T: LNA thymine.

10. The method of claim 9, wherein the LNA oligonucleotide molecule is selected from the group of oligonucleotides corresponding to Compounds 2, 3 or 6 to 10 having an anthraquinone in the 5' position instead of biotin.

11. The method of claim 9, wherein the LNA oligonucleotide molecule is selected from the group consisting of a oligonucleotides corresponding to Compounds 2, 3 or 6 to 10 having an anthraquinone in the 5' position and a linker which is selected from the group comprising one or more of a hexaethylene glycol monomer, dimer , trimer, tetramer, pentamer, hexamer, or higher hexaethylene glycol polymer; a poly-T sequence of 10-50 nucleotides in length or a poly-C sequence of 10-50 nucleotides in length or longer.

12. The method of claim 9, wherein the LNA oligonucleotide molecule is selected from the group a oligonucleotides corresponding to Compounds 2, 3 or 6 to 10 without the biotin substitution in the 5' position.

13. The method of anyone of claims 1 to 8, wherein the LNA oligonucleotide comprises at least one nucleotide having a nucleobase that is different from the nucleobases of the remaining oligonucleotide sequence.

14. The method of any one of claims 1 to 9, wherein the -1 residue of the LNA oligonucleotide molecule 3' and/or 5' end is an LNA residue.

15. The method of any one of claims 1 to 9, wherein the LNA oligonucleotide comprises at least about one or more alpha-L LNA monomers.

16. The method of any one of claims 1 to 9, wherein the LNA oligonucleotide comprises at least about one or more xylo-LNA monomers.

17. The method of any one of claims 1 to 16, wherein the LNA oligonucleotide molecule comprises at least about 20 to 50 percent LNA residues based on total residues of the LNA oligonucleotide.

18. The method of any one of claims 1 to 17, wherein the LNA oligonucleotide comprises at least about two or more consecutive LNA molecules.

19. The method of any one of claims 1 to 18, wherein the LNA oligonucleotide comprises modified and non-modified nucleotide molecules.

20. The method of any one of claims 1 to 19, wherein the LNA oligonucleotide capture probe is complementary to the sequence it is designed to detect and/or isolate.

21. The method of anyone of claims 1 to 19, wherein the LNA oligonucleotide has at least one base pair difference to the complementary sequence it is designed to detect and/or isolate.

22. The method of claim 21, wherein the LNA oligonucleotide can detect at least about one base pair difference between the complementary poly-repetitive base sequence and the LNA/DNA oligonucleotide.

23. The method of any one of claims 1 to 22, wherein the LNA oligonucleotide comprises a fluorophore moiety and a quencher moiety, positioned in such a way that the hybridized state of the oligonucleotide can be distinguished from the unbound state of the oligonucleotide by an increase in the fluorescent signal from the nucleotide.

24. The method of any one of claims 1 to 17, wherein the Tₘ of the LNA oligonucleotide is between about 50°C to about 70°C when the LNA oligonucleotide hybridizes to its complementary sequence.

25. The method of any one of the preceding claims, wherein the chaotropic agent is guanidinium thiocyanate.

26. The method of claim 25, wherein the concentration of the guanidinium thiocyanate is at least 2M, at least 3M or at least 4M.

27. The method of claim 24, wherein the LNA oligonucleotide hybridizes to the repetitive element at a temperature in the range of 20 - 65 °C.

28. The method of any one of the preceding claims, wherein the LNA oligonucleotide capture probe is adapted for use as a TaqMan probe or Molecular Beacon.

29. The method of any one of claims 1 to 28, wherein the eukaryotic mRNA is isolated using the covalently attached LNA oligonucleotide, and detected with nucleic acid probes, such as DNA, RNA or LNA detection probes, using:
(i) chemiluminiscence using enzyme-conjugated nucleic acid probes; or
(ii) bioluminescence using firefly or bacterial luciferase or green fluorescent protein as reporter molecule; or
(iii) ligands incorporated into the nucleic acid probes, such as digoxigenin (DIG) or fluorescein isothiocyanate (FITC) combined with enzyme-conjugated anti-ligand antibodies; or
(iv) biotin-labeled nucleic acid probes combined with enzyme-conjugated streptavidin or avidin.

30. The method of claim 29, wherein the eukaryotic mRNA is detected using an LNA detection oligonucleotide combined with a tyramide signal amplification system.

31. The method of claim 29, wherein the eukaryotic mRNA is detected using an LNA detection oligonucleotide, containing a complementary overhang to a free arm in a dendrimer or a branched oligonucleotide conjugated with several digoxigenin, fluorescein isothiocyanate or biotin molecules or fluorochrome molecules, combined with alkaline phosphatase-conjugated or horse radish peroxidase-conjugated anti-digoxigenin, anti-fluorescein isothiocyanate antibodies or streptavidin or detection of fluorescence from the excited fluorochromes.

32. The method of any one of claims 1 to 31, further comprising contacting the sample with a polymerase and at least one nucleotide.

33. The method of claim 32, further comprising performing said contacting under conditions suitable for generating a plurality of copies of said nucleic acid molecule.

34. The method of claim 32 comprising adding a DNA polymerase, RNaseH and E. coli DNA ligase after conversion of the eukaryotic polyadenylated mRNA to first strand complementary DNA under conditions suitable for generating double stranded complementary DNA.

35. The method of claim 34, wherein the LNA oligonucleotide complementary to the poly(A) tail sequence in eukaryotic mRNA contains an anchor sequence for a RNA polymerase, such as T7 RNA polymerase.

36. The method of claim 35 further comprising adding an RNA polymerase, such as T7 RNA polymerase, under conditions suitable for generating a plurality of RNA copies of said nucleic acid molecule.

37. The method of any one of the preceding claims, wherein the eukaryotic mRNA is genomic RNA from a retrovirus.

38. The method of claim 37, wherein the retrovirus is HIV.

39. The method of any one of the preceding claims, wherein the detection and/or isolation of a nucleic acid is carried out under high stringency hybridisation conditions using low salt concentration.

40. The method of claim 39, wherein said chaotropic agent is GuSCN in a concentration of at least 4 M.

41. The method of claim 30 or claim 40, wherein the binding buffer contains NaCl or LiCl.

42. The method of claim 41, wherein the NaCl or the LiCl concentration is less than 100 mM, less than 50mM or less than 25mM.

43. The method of any one of claims 38 to 42, wherein the detection or hybridisation is carried out at least 25 °C, at least 37°C or at least 50°C.

## Patentansprüche

1. Verfahren zur Detektion und/oder Isolation eines Nucleinsäuremoleküls mit einer homopolymeren Sequenz mittels Hybridisierung, umfassend:
a) die Behandlung einer Probe, die Nucleinsäureverbindungen umfasst, mit einem Lysepuffer, der ein chaotropes Mittel umfasst; und
b) die Behandlung der Probe mit einer "Locked Nucleic Acid"- (LNA-) Oligonucleotid-Fangsonde, die zumindest 20 wiederkehrende aufeinander folgende Nucleotide umfasst, um **dadurch** ein Nucleinsäuremolekül mit der homopolymeren Sequenz mittels Hybridisierung zu detektieren und/oder zu isolieren, wobei die homopolymere Sequenz ein Poly(A)-Schwanz einer eukaryotischen mRNA ist;
wobei die LNA-Oligonucleotid-Fangsonde eine Verbindung folgender Formel umfasst:
5'-Y^{q}-(X^{p}-Yⁿ)ₘ-X^{p}-Z-3'
worin X ein LNA-Monomer ist, Y ein DNA-Monomer ist; Z für ein fakultatives DNA-Monomer steht; p eine ganze Zahl von etwa 1 bis etwa 15 ist; n eine ganze Zahl von etwa 1 bis etwa 15 ist oder n für 0 steht; q eine ganze Zahl von etwa 1 bis etwa 10 ist oder q = 0 ist; und m eine ganze Zahl von etwa 5 bis etwa 20 ist,
wobei die LNA-Oligonucleotid-Fangsonde an ihre komplementäre Sequenz hybridisiert.

2. Verfahren nach Anspruch 1, wobei die LNA-Oligonucleotid-Fangsonde kovalent an einen festen Träger gebunden ist.

3. Verfahren nach Anspruch 1 oder 2, wobei die LNA-Oligonucleotid-Fangsonde mit einer Anthrachinongruppierung und einem Linker am 5'-Ende oder 3'-Ende der Sonde synthetisiert wird.

4. Verfahren nach Anspruch 3, worin der Linker aus der ein oder mehrere von einem Hexaethylenglykolmonomer, -dimer, -trimer, -tetramer, -pentamer, -hexamer oder einem höheren Hexaethylenglykolpolymer; einer Poly-T-Sequenz mit einer Länge von 10 bis 50 Nucleotiden oder einer Poly-C-Sequenz mit einer Länge von 10 bis 50 Nucleotiden oder länger; oder einer Nicht-Basensequenz mit einer Länge von 10 bis 50 Nucleotideinheiten oder länger umfassenden Gruppe ausgewählt ist.

5. Verfahren nach Anspruch 2, worin der feste Träger ein Polymerträger ist, der aus der aus Folgendem bestehenden Gruppe ausgewählt ist: einer Mikrotiterplatte, Polystyrolperlen, Latexperlen, einem Polymer-Objektträger oder einem polymerbeschichteten Objektträger oder einem Mikrofluidobjektträger.

6. Verfahren nach einem der vorangegangenen Ansprüche, worin die LNA-Oligonucleotid-Fangsonde in Bezug auf ein homopolymeres Nucleotid komplementär ist, das zumindest eine Nucleobase umfasst, die sich von den Basen unterscheidet, die die homopolymere Nucleinsäuresequenz bilden.

7. Verfahren nach einem der vorangegangenen Ansprüche, worin das LNA-Oligonucleotid zumindest 30, zumindest 40 oder zumindest 50 wiederkehrende aufeinander folgende Nucleotide umfasst.

8. Verfahren nach Anspruch 4, worin die kovalente Bindung der LNA-Oligonucleotidsonde an einen festen Polymerträger über die Anregung der Anthrachinongruppierung mittels UV-Licht erfolgt.

9. Verfahren nach Anspruch 1, worin das LNA-Oligonucleotidmolekül aus folgender Tabelle ausgewählt ist:
| Verb. Nr. | Oligo Name: | Sequenz 5'-: |
|---|---|---|
| 2 | LNA_2.T | 5'-Biotin-TtTtTtTtTtTtTtTtTtTt |
| 3 | LNA_3.T | 5'-Biotin-TttTttTttTttTttTttTt |
| 6 | LNA_4.T | 5'-Biotin-ttTtttTtttTtttTtttTt |
| 7 | LNA_5.T | 5'-Biotin-tttTttttTttttTttttTt |
| 8 | LNA_T₂₀ | 5'-Biotin- TTTTTTTTTTTTTTTTTTTT |
| 9 | LNA_TT | 5'-Biotin-ttTTtttTTtttTTtttTTt |
| 10 | LNA_TTT | 5'-Biotin-ttTTTttttTTTttttTTTt |
| 11 | AQ-HEG₃-2.T | AQ-HEG₃-TtTtTtTtTtTtTtTtTtTt |
| 12 | AQ-t15-2.T | AQ-t15-TtTtTtTtTtTtTtTtTtTt |
| 13 | AQ-c15-2.T | AQ-c15-TtTtTtTtTtTtTtTtTtTt |
| 14 | AQ-t10-NB5-2.T | AQ-t10-NB5.TtTtTtTtTtTtTtTtTtTt |
worin sich AQ auf Anthrachinon bezieht, HEG sich auf Hexaethylenglykol bezieht, t15 sich auf 15-mer-Desoxythymin bezieht, c15 sich auf 15-mer-Desoxycytosin bezieht, t10-NB5 sich auf 10-mer-Desoxythymin-5-mer-Nicht-Base und t sich auf DNA-Thymin und T auf LNA-Thymin bezieht.

10. Verfahren nach Anspruch 9, worin das LNA-Oligonucleotidmolekül aus der Gruppe der den Verbindungen 2, 3 oder 6 bis 10 entsprechenden Oligonucleotiden mit einem Anthrachinon an der 5'-Position anstelle von Biotin ausgewählt ist.

11. Verfahren nach Anspruch 9, worin das LNA-Oligonucleotidmolekül aus der aus Folgendem bestehenden Gruppe ausgewählt ist: Oligonucleotiden, die den Verbindungen 2, 3 oder 6 bis 10 mit einem Anthrachinon an der 5'-Position und einem Linker entsprechen, der aus der aus Folgendem bestehenden Gruppe ausgewählt ist: einem oder mehreren von einem Hexaethylenglykolmonomer, -dimer, -trimer, -tetramer, -pentamer, -hexamer oder einem höheren Hexaethylenglykolpolymer; einer Poly-T-Sequenz mit einer Länge von 10 bis 50 Nucleotiden oder einer Poly-C-Sequenz mit einer Länge von 10 bis 50 Nucleotiden oder länger.

12. Verfahren nach Anspruch 9, worin das LNA-Oligonucleotidmolekül aus der Gruppe von Oligonucleotiden ausgewählt ist, die den Verbindungen 2, 3 oder 6 bis 10 ohne Biotin-Substitution an der 5'-Position entsprechen.

13. Verfahren nach einem der Ansprüche 1 bis 8, worin das LNA-Oligonucleotid zumindest ein Nucleotid mit einer Nucleobase umfasst, die sich von den Nucleobasen der restlichen Oligonucleotidsequenz unterscheidet.

14. Verfahren nach einem der Ansprüche 1 bis 9, worin der -1-Rest am 3'-und/oder 5'-Ende des LNA-Oligonucleotidmoleküls ein LNA-Rest ist.

15. Verfahren nach einem der Ansprüche 1 bis 9, worin das LNA-Oligonucleotid zumindest etwa ein oder mehrere α-L-LNA-Monomere umfasst.

16. Verfahren nach einem der Ansprüche 1 bis 9, worin das LNA-Oligonucleotid zumindest etwa ein oder mehrere xylo-LNA-Monomere umfasst.

17. Verfahren nach einem der Ansprüche 1 bis 16, worin das LNA-Oligonucleotidmolekül, bezogen auf alle Reste des LNA-Oligonucleotids, zumindest etwa 20 bis 50 % LNA-Reste umfasst.

18. Verfahren nach einem der Ansprüche 1 bis 17, worin das LNA-Oligonucleotid zumindest etwa zwei oder mehrere aufeinander folgende LNA-Moleküle umfasst.

19. Verfahren nach einem der Ansprüche 1 bis 18, worin das LNA-Oligonucleotid modifizierte und nicht-modifizierte Nucleotidmoleküle umfasst.

20. Verfahren nach einem der Ansprüche 1 bis 19, worin die LNA-Oligonucleotid-Fangsonde in Bezug auf die Sequenz komplementär ist, zu deren Detektion und/oder Isolation sie entwickelt wurde.

21. Verfahren nach einem der Ansprüche 1 bis 19, worin das LNA-Oligonucleotid zumindest einen Basenpaarunterschied in Bezug auf die komplementäre Sequenz aufweist, zu deren Detektion und/oder Isolation sie entwickelt wurde.

22. Verfahren nach Anspruch 21, worin das LNA-Oligonucleotid zumindest etwa einen Basenpaarunterschied zwischen der komplementären polyrepetitiven Basensequenz und dem LNA/DNA-Oligonucleotid detektieren kann.

23. Verfahren nach einem der Ansprüche 1 bis 22, worin das LNA-Oligonucleotid eine Fluorophorgruppierung und eine Quenchergruppierung umfasst, die so angeordnet sind, dass der hybridisierte Zustand des Oligonucleotids von dem ungebundenen Zustand des Oligonucleotids durch einen Anstieg des von dem Nucleotid ausgehenden Fluoreszenzsignals unterschieden werden kann.

24. Verfahren nach einem der Ansprüche 1 bis 17, worin der Tₘ des LNA-Oligonucleotids zwischen etwa 50°C und etwa 70°C liegt, wenn das LNA-Oligonucleotid an seine komplementäre Sequenz hybridisiert.

25. Verfahren nach einem der vorangegangenen Ansprüche, worin das chaotrope Mittel Guanidiniumthiocyanat ist.

26. Verfahren nach Anspruch 25, worin die Konzentration von Guanidiniumthiocyanat zumindest 2 M, zumindest 3 M oder zumindest 4 M beträgt.

27. Verfahren nach Anspruch 24, worin das LNA-Oligonucleotid bei einer Temperatur im Bereich von 20 bis 65°C an das repetitive Element hybridisiert.

28. Verfahren nach einem der vorangegangenen Ansprüche, worin die LNA-Oligonucleotid-Fangsonde zur Verwendung als TaqMan-Sonde oder Hybridisierungssonde geeignet ist.

29. Verfahren nach einem der Ansprüche 1 bis 28, worin die eukaryotische mRNA unter Verwendung des kovalent gebundenen LNA-Oligonucleotids isoliert und mit Nucleinsäuresonden, wie z.B. DNA-, RNA- oder LNA-Detektionssonden, detektiert wird, wobei Folgendes eingesetzt wird:
(i) Chemilumineszenz unter Einsatz von enzymkonjugierten Nucleinsäuresonden; oder
(ii) Biolumineszenz unter Einsatz von Glühwürmchenluciferase oder bakterieller Luciferase oder grünfluoreszierendem Protein als Reportermolekül; oder
(iii) in die Nucleinsäuresonden integrierte Liganden, wie z.B. Digoxigenin (DIG) oder Fluoresceinisothiocyanat (FITC) in Kombination mit enzymkonjugierten Anti-Liganden-Antikörpern; oder
(iv) Biotin-markierte Nucleinsäuresonden in Kombination mit enzymkonjugiertem Streptavidin oder Avidin.

30. Verfahren nach Anspruch 29, worin die eukaryotische mRNA unter Einsatz eines LNA-Detektionsoligonucleotids in Kombination mit einem Tyramidsignalamplifikationssystem detektiert wird.

31. Verfahren nach Anspruch 29, worin die eukaryotische mRNA unter Einsatz eines LNA-Detektionsoligonucleotids, das einen komplementären Überhang zu einem freien Arm in einem Dendrimer oder einem verzweigten Oligonucleotid, das mit mehreren Digoxigenin-, Fluoresceinisothiocyanat- oder Biotinmolekülen oder Fluorochrommoleküle konjugiert ist, enthält, in Kombination mit an alkalische Phosphatase oder Meerrettichperoxidase konjugiertem Anti-Digoxigenin, Anti-Fluoresceinisothiocyanat-Antikörpern oder Streptavidin oder durch die Detektion der von den angeregten Fluorochromen ausgehenden Fluoreszenz detektiert wird.

32. Verfahren nach einem der Ansprüche 1 bis 31, weiters umfassend das Kontaktieren der Probe mit einer Polymerase und zumindest einem Nucleotid.

33. Verfahren nach Anspruch 32, weiters umfassend das Durchführen des Kontaktierens unter Bedingungen, die zur Erzeugung einer Vielzahl von Kopien des Nucleinsäuremoleküls geeignet sind.

34. Verfahren nach Anspruch 32, umfassend das Hinzufügen einer DNA-Polymerase, RNaseH und E.-coli-DNA-Ligase nach der Überführung der eukaryotischen polyadenylierten mRNA in komplementäre Erststrang-DNA unter Bedingungen, die zur Erzeugung von doppelsträngiger komplementärer DNA geeignet sind.

35. Verfahren nach Anspruch 34, worin das LNA-Oligonucleotid, das in Bezug auf die Poly(A)-Schwanzsequenz in eukaryotischer mRNA komplementär ist, eine Ankersequenz für eine RNA-Polymerase, wie z.B. T7-RNA-Polymerase, enthält.

36. Verfahren nach Anspruch 35, weiters umfassend das Hinzufügen einer RNA-Polymerase, wie z.B. T7-RNA-Polymerase, unter Bedingungen, die zur Erzeugung einer Vielzahl von RNA-Kopien des Nucleinsäuremoleküls geeignet sind.

37. Verfahren nach einem der vorangegangenen Ansprüche, worin die eukaryotische mRNA genomische RNA von einem Retrovirus ist.

38. Verfahren nach Anspruch 37, worin das Retrovirus HIV ist.

39. Verfahren nach einem der vorangegangenen Ansprüche, worin die Detektion und/oder Isolation einer Nucleinsäure unter hochstringenten Hybridisierungsbedingungen unter Verwendung einer niedrigen Salzkonzentration erfolgt.

40. Verfahren nach Anspruch 39, worin das chaotrope Mittel GuSCN in einer Konzentration von zumindest 4 M ist.

41. Verfahren nach Anspruch 30 oder 40, worin der Bindepuffer NaCl oder LiCl enthält.

42. Verfahren nach Anspruch 41, worin die NaCl- oder LiCl-Konzentration weniger als 100 mM, weniger als 50 mM oder weniger als 25 mM beträgt.

43. Verfahren nach einem der Ansprüche 38 bis 42, worin die Detektion oder Hybridisierung bei zumindest 25 °C, bei zumindest 37°C oder bei zumindest 50°C durchgeführt wird.

## Revendications

1. Procédé de détection et/ou d'isolation par hybridation d'une molécule d'acide nucléique ayant une séquence homopolymère comprenant:
a) le traitement d'un échantillon contenant des composés d'acide nucléique avec un tampon de lyse comprenant un agent chaotropique; et
b) le traitement dudit échantillon avec une sonde de capture d'oligonucléotide d'acide nucléique verrouillé (LNA) comprenant au moins vingt nucléotides consécutifs répétés pour détecter et/ou isoler ainsi par hybridation une molécule d'acide nucléique ayant ladite séquence homopolymère, où ladite séquence homopolymère est une queue poly(A) d'un ARNm eucaryote; et où la sonde de capture d'oligonucléotide de LNA comprend un composé de formule:
5'-Y^{q}- (X^{p}-Yⁿ)ₘ-X^{p}-Z-3'
dans laquelle X est un monomère de LNA, Y est un monomère d'ADN; Z représente un monomère d'ADN facultatif; p est un entier valant environ 1 à environ 15; n est un entier valant environ 1 à environ 15 ou n représente 0; q est un entier valant d'environ 1 à environ 10 ou q = 0; et m est un entier valant d'environ 5 à environ 20, où la sonde de capture d'oligonucléotide de LNA s'hybride sur sa séquence complémentaire.

2. Procédé selon la revendication 1, dans lequel la sonde de capture d'oligonucléotide de LNA est attachée par covalence à un support solide.

3. Procédé selon la revendication 1 ou la revendication 2, dans lequel la sonde de capture d'oligonucléotide LNA est synthétisée avec une fraction anthraquinone et un lieur à l'extrémité 5' ou l'extrémité 3' de ladite sonde.

4. Procédé selon la revendication 3, dans lequel ledit lieur est choisi dans le groupe comprenant un ou plusieurs éléments parmi un monomère, dimère, trimère, tétramère, pentamère, hexamère d'hexaéthylène glycol, ou un polymère d'hexaéthylène glycol supérieur; une séquence poly-T de 10 à 50 nucléotides de longueur ou une séquence poly-C de 10 à 50 nucléotides de longueur ou plus; ou une séquence de non-base de 10 à 50 motifs nucléotide de longueur ou plus.

5. Procédé selon la revendication 2, dans lequel ledit support solide est un support polymère choisi dans le groupe constitué d'une plaque à microtitration, de billes de poly(styrène), de billes de latex, d'une lame pour microscope de polymère ou d'une lame pour microscope revêtue de polymère ou d'une lame microfluidique.

6. Procédé selon l'une quelconque des revendications précédentes, dans lequel la sonde de capture d'oligonucléotide de LNA est complémentaire d'un nucléotide homopolymère comprenant au moins une nucléobase qui est différente des bases comprenant la séquence d'acides nucléiques homopolymère.

7. Procédé selon l'une quelconque des revendications précédentes, dans lequel l'oligonucléotide de LNA comprend au moins trente, au moins quarante ou au moins cinquante nucléotides consécutifs répétés.

8. Procédé selon la revendication 4, dans lequel un couplage covalent sur un support polymère solide de ladite sonde d'oligonucléotide de LNA est réalisé par excitation de la fraction anthraquinone en utilisant une lumière UV.

9. Procédé selon la revendication 1, dans lequel la molécule d'oligonucléotide de LNA est choisie dans le tableau suivant:
| N° de composé | Nom de l'oligonucléotide | Séquence 5' |
|---|---|---|
| 2 | LNA 2.T | 5'-biotin-TtTtTtTtTtTtTtTtTtTt |
| 3 | LNA 3.T | 5'-biotin-TttTttTttTttTttTttTt |
| 6 | LNA 4.T | 5'-biotin-ttTtttTtttTtttTtttTt |
| 7 | LNA 5.T | 5'-biotin-tttTttttTttttTttttTt |
| 8 | LNA_T₂₀ | 5'-biotin-TTTTTTTTTTTTTTTTTTTT |
| 9 | LNA TT | 5'-biotin-ttTTtttTTtttTTtttTTt |
| 10 | LNA TTT | 5'-biotin-ttTTTttttTTTttttTTTt |
| 11 | AQ-HEG₃-2.T | AQ-HEG₃-TtTtTtTtTtTtTtTtTtTt |
| 12 | AQ-t15-2.T | AQ-t15-TtTtTtTtTtTtTtTtTtTt |
| 13 | AQ-c15-2.T | AQ-c15-TtTtTtTtTtTtTtTtTtTt |
| 14 | AQ-t10-NB5-2.T | AQ-t10-NB5-TtTtTtTtTtTtTtTtTtTt |
où AQ fait référence à l'anthraquinone, HEG fait référence à l'hexaéthylène glycol, t15 fait référence à la 15-mèredésoxy-thymine, c15 fait référence à la 15-mèredésoxy-cytosine, t10-NB5 fait référence à la 10-mèredésoxy-thymine 5-mère non-base, et t fait référence à la thymine d'ADN et T à la thymine de LNA.

10. Procédé selon la revendication 9, dans lequel la molécule d'oligonucléotide de LNA est choisie dans le groupe d'oligonucléotides correspondant aux composés 2, 3 ou 6 à 10 ayant une anthraquinone en position 5' à la place d'une biotine.

11. Procédé selon la revendication 9, dans lequel la molécule d'oligonucléotide de LNA est choisie dans le groupe constitué d'oligonucléotides correspondant aux composés 2, 3 ou 6 à 10 ayant une anthraquinone en position 5' et un lieur qui est choisi dans le groupe comprenant un ou plusieurs éléments parmi un monomère, dimère, trimère, tétramère, pentamère, hexamère d'hexaéthylène glycol, ou un polymère d'hexaéthylène glycol supérieur; une séquence poly-T de 10 à 50 nucléotides de longueur ou une séquence poly-C de 10 à 50 nucléotides de longueur ou plus.

12. Procédé selon la revendication 9, dans lequel la molécule d'oligonucléotide de LNA est choisie dans le groupe constitué d'oligonucléotides correspondant aux composés 2, 3 ou 6 à 10 sans la substitution de biotine en position 5'.

13. Procédé selon l'une quelconque des revendications 1 à 8, dans lequel l'oligonucléotide de LNA comprend au moins un nucléotide ayant une nucléobase qui est différente des nucléobases de la séquence d'oligonucléotides restante.

14. Procédé selon l'une quelconque des revendications 1 à 9, dans lequel le résidu -1 de l'extrémité 3' et/ou 5' de la molécule d'oligonucléotide de LNA est un résidu de LNA.

15. Procédé selon l'une quelconque des revendications 1 à 9, dans lequel l'oligonucléotide de LNA comprend au moins environ un ou plusieurs monomères d'alpha-L LNA.

16. Procédé selon l'une quelconque des revendications 1 à 9, dans lequel l'oligonucléotide de LNA comprend au moins environ un ou plusieurs monomères de xylo-LNA.

17. Procédé selon l'une quelconque des revendications 1 à 16, dans lequel la molécule d'oligonucléotide de LNA comprend au moins environ 20 à 50 pour cent de résidus de LNA sur la base des résidus totaux de l'oligonucléotide de LNA.

18. Procédé selon l'une quelconque des revendications 1 à 17, dans lequel l'oligonucléotide de LNA comprend au moins environ deux molécules de LNA consécutives ou plus.

19. Procédé selon l'une quelconque des revendications 1 à 18, dans lequel l'oligonucléotide de LNA comprend des molécules de nucléotide modifiées et non modifiées.

20. Procédé selon l'une quelconque des revendications 1 à 19, dans lequel la sonde de capture d'oligonucléotide de LNA est complémentaire de la séquence qu'elle doit détecter et/ou isoler.

21. Procédé selon l'une quelconque des revendications 1 à 19, dans lequel l'oligonucléotide de LNA a au moins une différence de paire de base par rapport à la séquence complémentaire qu'il doit détecter et/ou isoler.

22. Procédé selon la revendication 21, dans lequel l'oligonucléotide de LNA peut détecter au moins environ une différence de paire de base entre la séquence de base poly-répétitive complémentaire et l'oligonucléotide de LNA/ADN.

23. Procédé selon l'une quelconque des revendications 1 à 22, dans lequel l'oligonucléotide de LNA comprend une fraction de fluorophore et une fraction de désactiveur, positionnées de telle sorte que l'état hybridé de l'oligonucléotide peut être distingué de l'état non lié de l'oligonucléotide par une augmentation du signal fluorescent du nucléotide.

24. Procédé selon l'une quelconque des revendications 1 à 17, dans lequel la Tₘ de l'oligonucléotide de LNA est comprise entre environ 50°C et environ 70°C lorsque l'oligonucléotide de LNA s'hybride sur sa séquence complémentaire.

25. Procédé selon l'une quelconque des revendications précédentes, dans lequel l'agent chaotropique est le thiocyanate de guanidinium.

26. Procédé selon la revendication 25, dans lequel la concentration du thiocyanate de guanidinium est d'au moins 2 M, d'au moins 3 M ou d'au moins 4 M.

27. Procédé selon la revendication 24, dans lequel l'oligonucléotide de LNA s'hybride sur l'élément répétitif à une température dans la plage de 20 à 65°C.

28. Procédé selon l'une quelconque des revendications précédentes, dans lequel la sonde de capture d'oligonucléotide de LNA est adaptée pour une utilisation comme sonde TaqMan ou phare moléculaire.

29. Procédé selon l'une quelconque des revendications 1 à 28, dans lequel l'ARNm eucaryote est isolé en utilisant l'oligonucléotide de LNA attaché par covalence, et détecté avec des sondes d'acide nucléique, telles que des sondes de détection d'ADN, d'ARN ou de LNA, en utilisant:
(i) la chimioluminescence à l'aide de sondes d'acide nucléique à enzyme conjuguée; ou
(ii) la bioluminescence à l'aide de luciférase de luciole ou bactérienne ou d'une protéine à fluorescence verte comme molécule rapporteuse; ou
(iii) des ligands incorporés dans les sondes d'acide nucléique, tels que la digoxigénine (DIG) ou l'isothiocyanate de fluorescéine (FITC) combinés à des anticorps anti-ligand à enzyme conjuguée; ou
(iv) des sondes d'acide nucléique marquées à la biotine combinée à de la streptavidine ou de l'avidine à enzyme conjuguée.

30. Procédé selon la revendication 29, dans lequel l'ARNm eucaryote est détecté en utilisant un oligonucléotide de détection de LNA combiné à un système d'amplification de signal de tyramide.

31. Procédé selon la revendication 29, dans lequel l'ARNm eucaryote est détecté en utilisant un oligonucléotide de détection de LNA, contenant une extrémité protubérante complémentaire sur un bras libre dans un dendrimère ou un oligonucléotide ramifié conjugué à plusieurs molécules de digoxigénine, d'isothiocyanate de fluorescéine ou de biotine ou de fluorochrome, combinés à des anticorps anti-isothiocyanate de fluorescéine, anti-digoxigénine à peroxydase conjuguée de raifort ou à alcaline phosphatase conjuguée ou de la streptavidine ou la détection de fluorescence des fluorochromes excités.

32. Procédé selon l'une quelconque des revendications 1 à 31, comprenant en outre le contact de l'échantillon avec une polymérase et au moins un nucléotide.

33. Procédé selon la revendication 32, comprenant en outre la réalisation dudit contact dans des conditions appropriées pour générer une pluralité de copies de ladite molécule d'acide nucléique.

34. Procédé selon la revendication 32 comprenant l'ajout d'une ADN polymérase, d'une RNase H et d'une ADN ligase E. coli après la conversion de l'ARNm polyadénylé eucaryote en ADN complémentaire monobrin dans des conditions appropriées pour générer de l'ADN complémentaire double brin.

35. Procédé selon la revendication 34, dans lequel l'oligonucléotide de LNA complémentaire de la séquence de queue poly(A) dans l'ARNm eucaryote contient une séquence d'ancrage pour une ARN polymérase, telle que la T7 ARN polymérase.

36. Procédé selon la revendication 35, comprenant en outre l'ajout d'une ARN polymérase, telle que la T7 ARN polymérase, dans des conditions appropriées pour générer une pluralité de copies d'ARN de ladite molécule d'acide nucléique.

37. Procédé selon l'une quelconque des revendications précédentes, dans lequel l'ARNm eucaryote est l'ARN génomique d'un rétrovirus.

38. Procédé selon la revendication 37, dans lequel le rétrovirus est le VIH.

39. Procédé selon l'une quelconque des revendications précédentes, dans lequel la détection et/ou l'isolation d'un acide nucléique sont réalisées dans des conditions d'hybridation très strictes en utilisant une faible concentration de sel.

40. Procédé selon la revendication 39, dans lequel ledit agent chaotropique est GuSCN en une concentration d'au moins 4 M.

41. Procédé selon la revendication 30 ou la revendication 40, dans lequel le tampon de liaison contient du NaCl ou du LiCl.

42. Procédé selon la revendication 41, dans lequel la concentration de NaCl ou de LiCl est inférieure à 100 mM, inférieure à 50 mM ou inférieure à 25 mM.

43. Procédé selon l'une quelconque des revendications 38 à 42, dans lequel la détection ou l'hybridation est réalisée à au moins 25°C, au moins 37°C ou au moins 50°C.
